# EUROPÄISCHE PATENTANMELDUNG

(11) **EP 4 361 242 A1**
(43) Veröffentlichungstag der Anmeldung: **01.05.2024**
(21) Anmeldenummer: 22203949.7
(22) Anmeldetag: 26.10.2022
(51) Int. Cl.: C12M 1/12, C12M 1/00

(54) **INKUBATOR FÜR ZELLKULTUREN**

(71) Anmelder: Eppendorf SE, 22339 Hamburg (DE)
(72) Erfinder: Wolf, Wente, 22299 Hamburg (DE)
(74) Vertreter: Wallinger Ricker Schlotter Tostmann

(57) **Zusammenfassung**

Die Erfindung bezieht sich auf einen Inkubator mit Sensoreinrichtung zur Erfassung einer VOC-Kontamination aus Reinigungsmittelrückständen in der Gasatmosphäre des Innenraums der Inkubatorkammer. Die Erfindung bezieht sich auch auf ein entsprechendes Verfahren.

## Beschreibung

Die Erfindung betrifft einen Inkubator für das Wachstum von biologischen Zellen. Die Erfindung betrifft zudem ein System und ein Verfahren zur Messung einer Inkubatoratmosphäre.

Mit solchen Inkubatoren werden in biologischen und medizinischen Laboratorien Zellen in Zellkultur unter kontrollierten Umgebungsbedingungen gehalten, und so das Wachstum lebender Zellen in vitro ermöglicht. Dazu werden die Temperatur und die Gaszusammensetzung bzw. die Luftfeuchtigkeit der Atmosphäre im Inneren einer von der Umgebung isolierten Inkubatorkammer durch die apparativen Einrichtungen des Inkubators auf den gewünschten Werten gehalten. Eukaryotische Zellen benötigen CO₂-lnkubatoren. Die Atmosphäre wird durch Luft mit einem bestimmten CO₂- und O₂-Gehalt und einer bestimmten Luftfeuchtigkeit gebildet, eine geeignete Temperatur ist oftmals 37 °C.

Inkubatoren müssen regelmäßig gereinigt sowie sterilisiert und bei Kontamination dekontaminiert werden. Diese Reinigung, Sterilisation oder Dekontamination kann durch Hochtemperatursterilisation, der Erhitzung auf bis zu 200 °C oder 180°C, Dekontamination durch Erhitzung auf bis zu 160°C, durch Anwendung mechanischer Reinigungsverfahren und/oder durch Anwendung physikalischer und/oder chemischer Reinigungsverfahren erfolgen.

Die üblicherweise von einem Laboranten durchgeführte mechanische Reinigung umfasst das manuelle Entfernen von Verunreinigungen durch Kratzen und/oder Schaben mit geeignetem Werkzeug, sowie das Wischen und/oder Reiben mit Tüchern/Schwämmen etc. Weitere physikalische Vorgänge, die bei der Reinigung von Inkubatoren relevant sind, sind das Lösen bzw. Auflösen von Verunreinigungen in geeigneten Lösemitteln wie z.B. destilliertem Wasser. Wasser ohne weitere Reinigungszusätze wird im Rahmen der vorliegenden Erfindung aber nicht als Reinigungsmittel bezeichnet, da Wasser keine VOCs enthält.

Um Sterilität gewährleisten zu können, kommen diverse Desinfektions- und Reinigungsmittel zum Einsatz, welche unterschiedliche Wirkungen auf biologische Kontaminationen wie: Viren, Mykoplasmen, Bakterien, Pilze, Hefen und eukaryotische Zellen haben.

Die nachfolgend aufgeführten Desinfektions- oder Reinigungsmittel sind die, die am häufigsten Anwendung in der Desinfektion und Reinigung von Inkubatoren finden.

Die am häufigsten zur Sterilisation von Inkubatoren verwendete Desinfektionsmittel, sind solche mit Alkoholen als Wirkstoff, insbesondere Ethanol, 1-Isopropanol und 2-Isopropanol. Ethanol wird in Laboratorien gegenüber anderen Reinigungsmitteln besonders häufig eingesetzt. Alle genannten Alkohole sind als Desinfektionsmittel für die Inkubatorreinigung sehr weit verbreitet, da sie sowohl gegen unbehüllte und behüllte Viren, Bakterien und andere Mikroorganismen wirksam sind. Alkohole sind volatil und verdunsten rückstandsfrei.

Weiterhin kommen Desinfektionsmittel auf Basis quartärer Ammoniumverbindungen zum Einsatz, wie z. B.: Benzalkoniumchlorid, Distearyldimethylammonium-chlorid, Didecyldimethylammonium-chlorid, etc., welche als kationische Tenside auf molekularer Ebene einen hydrophilen und einen hydrophoben Teil aufweisen, wodurch insbesondere Bakterien besonders effektiv bekämpft werden können, wobei andere Mikroorganismen ebenfalls abgetötet werden können. Da Desinfektionsmittel auf Basis quartärer Ammoniumverbindungen Salze in Lösung sind, trocknen diese nicht rückstandsfrei.

Desinfektionsmittel auf Basis von Phenolen und Phenolderivaten sind ebenfalls weit verbreitet und werden unter anderem aufgrund ihrer guten Eigenschaften gegen Pilze angewandt. Phenole sind volatil und sorgen für einen markanten Geruch.

Desinfektionsmittel auf Hypochlorit-Basis, z. B.: Natriumhypochlorit, sind gegen die Meisten Mikroorganismen effektiv, mit Ausnahme von Pilzen und weisen eine hohe Toxizität auf. In sauren Milieus wird giftiges Chlor freigesetzt, was die Anwendung für den Anwender erschwert, da dieses hochgiftig ist.

Peroxid-Verbindungen, insbesondere Wasserstoffperoxid, ist gegen die meisten Bakterien, Pilze und Viren effektiv, wobei die Effektivität gegenüber Sporen niedriger ausfällt. Bei der Anwendung von insbesondere Wasserstoffperoxid, entstehen bei der Zerfallsreaktion die Produkte Wasser und Sauerstoff, was bedeutet, dass nach der Desinfektion mit insbesondere Wasserstoffperoxid die Oberflächen des Inkubators feucht sein können, was eventuell Probleme bereitet.

Alle obig genannten Desinfektions- oder Reinigungsmittel (nachfolgend nur noch als "Reinigungsmittel" bezeichnet) sind biotoxisch, nicht nur gegenüber Kontaminationen sondern auch gegenüber denen im Inkubator heranwachsenden Zellkulturen, weswegen auf keinen Fall relevante Konzentrationen von Desinfektions- oder Reinigungsmittel oder deren Rückstände während des Inkubationszyklus im Inkubator vorhanden sein dürfen, da sonst die Gefahr besteht, dass Wachstumsraten von Zellkulturen verringert werden könnten, unnatürliches Wachstum entstehen könnte oder Zellkulturen absterben könnten.

Die Beeinträchtigung von Zellkulturen durch Reinigungsmittelrückstände ist problematisch für alle Anwendungen in der biologischen Forschung, der Impfstoffproduktion, der personalisierten Medizin und der regenerativen Medizinapplikationen. Negative Folgen der Beeinträchtigung sind Verlust von Zeit und Geld, sowie inakkurate bzw. fehlerhafte experimentelle Ergebnisse. Es gibt auch Fälle, beispielsweise in der Forensik oder der Reproduktionsmedizin, in denen der Wert einer einzelnen Probe, insbesondere in einem Zellkulturgefäß befindlichen Zelle/n, viel höher einzuschätzen ist als, beispielsweise, der Wert des gesamten Inkubators, so dass ein reinigungsmittelbedingter Verlust der Probe unbedingt zu vermeiden ist.

Der vorliegenden Erfindung liegt die Aufgabe zugrunde, einen Inkubator für Zellkulturen bereitzustellen, bei dem das Risiko einer negativen Beeinträchtigung der Zellkulturen durch die Reinigung der Inkubatorkammer mit den biotoxischen Reinigungsmitteln reduzierbar bzw. reduziert ist, und ein entsprechendes Verfahren bereitzustellen.

Die Erfindung löst diese Aufgabe durch den Inkubator gemäß Anspruch 1 und das Verfahren gemäß Anspruch 10. Bevorzugte Ausgestaltungen der Erfindung sind insbesondere Gegenstände der abhängigen Ansprüche.

Der erfindungsgemäße Inkubator dient der Inkubation lebender Zellkulturen und weist auf:
- eine Inkubatorkammer zur Aufnahme von Objekten, insbesondere Zellkulturbehältern, in einem verschließbaren Innenraum der Inkubatorkammer, der eine kontrollierbare Gasatmosphäre aufweist, wobei eine Oberfläche im Innenraum der Inkubatorkammer mit einem Reinigungsmittel reinigbar ist, das flüchtige organische Verbindungen (VOCs) aufweist;
- eine Sensoreinrichtung zur Detektion einer Anreicherung von in Reinigungsmittelrückständen enthaltenen flüchtigen organischen Verbindungen (VOCs), wobei die Sensoreinrichtung mindestens einen VOC-Sensor zur Detektion der VOCs aufweist und der mindestens eine VOC-Sensor mindestens einen Messbereich aufweist, der in Strömungsverbindung mit dem die VOCs aufweisenden Atmosphärengas des Innenraums stehend angeordnet ist.

Der erfindungsgemäße Inkubator begünstigt durch seine Sensoreinrichtung insbesondere die Inkubation von Zellkulturen in nachweislich VOC-freier Gasatmosphäre. Vermeidbar wird dadurch der unbeabsichtigte Betrieb des Inkubators bei relevanten -also biotoxisch wirkenden- Konzentrationen von Reinigungsmittelrückständen in der Gasatmosphäre, so dass eine negative Beeinträchtigung der Vitalität und des Wachstum der in dem Inkubator befindlichen Zellkulturen verhinderbar ist.

Der Inkubator ist somit unter optimalen Bedingungen betreibbar.

Die Erfindung beruht auf dem Umstand, dass Reinigungsmittel bei deren Anwendung gasförmige Rückstände produzieren, die auf die Anwesenheit der entsprechenden Desinfektions- oder Reinigungsmittel im Inkubator Rückschlüsse zulassen.

Es ist bekannt, dass durch Kontaminationsvorgänge, insbesondere durch das Wachstum von Bakterien und anderen Mikroorganismen, bestimmte Kontaminationsstoffe in die Umgebung abgegeben werden. Die flüchtigen organischen gasförmigen Stoffwechselprodukte von Mikroorganismen werden als MVOC (engl.: Microbial Volatile Organic Compounds) bezeichnet und können als Indikatoren für mikrobielles Wachstum genutzt werden. Die Konzentrationen dieser MVOC in einer Inkubatorkammer sind um Größenordnungen geringer als die Konzentrationen von VOCs, die als Rückstände von Reinigungsmitteln in der Inkubatorkammer verbleiben und detektiert werden. Die Anforderungen an eine Sensoreinrichtung und deren Ansteuerung, die der Detektion von diesen VOCs dienen unterscheiden sich deshalb von den Anforderungen an eine Sensoreinrichtung und deren Ansteuerung, die der Detektion von diesen MVOCs dienen.

Im Rahmen einer der vorliegenden Erfindung zugrunde liegenden Forschungsarbeit wurde eine neue Anordnung entwickelt, bei der die Gasatmosphäre des Innenraums der Inkubatorkammer, in der eine Vielzahl von verschlossenen Zellkulturbehältern angeordnet sein können, mittels einer inkubatoreigenen VOC-Sensoreinrichtung getestet wird, und zwar vorzugsweise als regelmäßig aktive Überwachungsfunktion während der Betriebsdauer des Inkubators. Es können die Benutzer auf diese Weise gewarnt werden und eine Ventilierung bzw. eine Entfernung der Reinigungsmittelrückstände des Innenraums der Inkubatorkammer kann veranlasst werden.

Die Sensoreinrichtung dient der Detektion von Kontamination der Gasatmosphäre des Innenraums. Die Sensoreinrichtung weist vorzugsweise mindestens einen, vorzugsweise genau einen, **VOC-Sensor** zur Detektion von mindestens einer flüchtigen organischen Verbindung (VOC) auf, die in den Reinigungsmittelrückständen enthalten ist. Dieser VOC-Sensor ist vorzugsweise so gestaltet oder gewählt, dass er für mindestens eine VOC, die durch Reinigungsmittelrückstände freigesetzt wird, empfindlich ist und messen kann, ob sich die Konzentration der VOC in der Gasatmosphäre des Innenraums verändert hat, insbesondere zugenommen hat

Die Detektion eines Reinigungsmittelrückstands ist insbesondere auch verwertbar als Nachweis, dass eine Reinigung der Inkubatorkammer durchgeführt wurde, insbesondere eine Reinigung nach den Richtlinien der Qualitätssicherung gemäß der guten Herstellerpraxis (GMP) durchgeführt wurde. Reinigungsmittelrückstände sind üblicherweise nur feststellbar, nachdem eine Reinigung durchgeführt wurde. Die Detektion eines Reinigungsmittelrückstands ist somit auch ein sensorischer Beweis, dass eine Reinigung durchgeführt wurde. Um eine hohe Qualität beispielsweise von zellbasierten Wirkstoffen, die aus in Inkubatoren kultivierten Zellen gewonnen werden, zu gewährleisten ist ein Inkubator regelmäßig zu reinigen, wobei eine solche Reinigung üblicherweise von Nutzern durchgeführt wird. Die Produktion nach GMP Richtlinien erfordert auch die Dokumentation einer solchen Reinigung. Die vorliegende Erfindung ist vorteilhaft für ein GMP-gerechtes Qualitätsmanagementsystem, da mittels der erfindungsgemäßen Vorrichtung und dem erfindungsgemäßen Verfahren erfasst wird, ob tatsächlich eine Reinigung durchgeführt wurde und darüber auch ein Datensatz erstellt wird. Die vorliegende Erfindung ermöglicht somit eine objektive Prüfung, ob eine Reinigung des Inkubators erfolgt ist.

Der Begriff "VOC" bezeichnet nicht MVOC oder das CO₂-Gas (Kohlenstoffdioxidgas), das insbesondere ein Bestandteil der Gasatmosphäre der Inkubatorkammer ist.

Der Inkubator weist vorzugsweise eine elektronische Steuereinrichtung, eine Inkubatortüre zum Verschliessen der Inkubatorkammer und einen elektronischen Türsensor auf, mit dem das Öffnen und/oder Schließen der Inkubatortüre erfassbar ist, wobei die elektronische Steuereinrichtung dazu programmiert ist,
a) das Öffnen und/oder Schließen der Inkubatortüre mittels des Türsensors als Türstatusparameter zu erfassen,
b) in Abhängigkeit von dem Türstatusparameter, insbesondere nach dem Schließen der Inkubatortüre, die Sensoreinrichtung automatisch zu aktivieren,
c) mittels der Sensoreinrichtung mindestens einen Messwert zu erfassen, der die Konzentration der in den Reinigungsmittelrückständen enthaltenen VOCs charakterisiert.

Der Messwert kann insbesondere eine ja/nein-Antwort auf die Frage liefern, ob sich (oder nicht) ein Reinigungsmittelrückstand in der Inkubatorkammer befindet. Auf diese Weise lässt sich messtechnisch relativ einfach eine Warnungsfunktion bei dem Inkubator implementieren, die den Benutzer auf das Vorhandensein von Reinigungsmittelrückstand hinweist. Gleichzeitig dient die Detektion eines Reinigungsmittelrückstands als Nachweis, dass eine Reinigung durchgeführt wurde. Der mindestens eine Messwert kann auch verschiedene Werte von Konzentrationen von Reinigungsmittelrückstand enthalten, insbesondere unterscheidbare Werte. Auf diese Weise kann durch computerimplementierte Auswertung, insbesondere mittels einer elektronischen, programmierbaren Steuereinrichtung, insbesondere die zeitliche Entwicklung der Konzentration von Reinigungsmittelrückstand in der Inkubatorkammer verfolgt werden.

Der Inkubaor kann ein Gehäuse aufweisen, vorzugsweise ein äußeres Gehäuse, dessen Gehäusewände mit der Umgebung in Kontakt stehen. Ein Kammergehäuse der Inkubatorkammer liegt dann vorzugsweise innerhalb des Außengehäuses. In diesem Fall kann ein Inkubator mindestens ein als inneres Gehäuse dienendes Kammergehäuse aufweisen, das durch mindestens eine Kammertüre verschließbar ist. Zusätzlich oder alternativ zur Kammertüre kann eine äußere Gehäusetüre vorgesehen sein, die in der Verschlussposition an die Umgebung grenzt, wobei insbesondere nur die äußere Gehäusetüre in der Verschlussposition an die Umgebung grenzt und die Kammertüre in der Verschlussposition der äußeren Gehäusetüre in einem Hohlraum zwischen Kammergehäuse, äußerem Gehäuse und äußerer Gehäusetüre liegt. Die äußere Gehäusetüre wird hier auch als Inkubatortüre bezeichnet und verschließt insbesondere die Inkubatortüre, insbesondere unter Verwendung einer Abdichtung zwischen äußerer Gehäusetüre und Gehäuse.

Vorzugsweise erfolgt die Aktivierung in Schritt b), wenn eine zeitabhängige Auswertung des Türparameters ergibt, dass die Inkubatortüre für mindestens die Dauer einer vorbestimmten, insbesondere in einer Datenspeichereinrichtung des Inkubators gespeicherten, Zeitspanne geöffnet war, und dass die Aktivierung in Schritt b) insbesondere nicht erfolgt, wenn die zeitabhängige Auswertung des Türparameters ergibt, dass die Inkubatortüre für weniger als die Dauer der vorbestimmten Zeitspanne geöffnet war. Da bei den üblichen Arbeiten am Inkubator, die Tür nur kurz - dies verhindert den Verlust der Atmosphäre (Gas und Luftfeuchtigkeit) - geöffnet wird um Zellkulturgefäße hereinzustellen oder herauszunehmen, liegen bevorzugte vorbestimmte Zeitspannen im Bereich von 2 min bis 20 min . Die Zeitspanne kann auch gewählt sein aus den bevorzugten Bereichsspannen 1 min bis 45 min, 2 min bis 45 min, 5 min bis 45 min, 10 min bis 45 min. Die oberen Grenzen der Bereichsangaben können auch variiert werden, ebenso die unteren Grenzen. Die Zeitspanne ist typisch für die Dauer einer Reinigung.

Der Inkubator weist vorzugsweise eine elektronische Steuereinrichtung und eine Benutzerschnittstelleneinrichtung auf, über die ein Benutzer Daten an die Datenverarbeitungseinrichtung bzw. die Steuereinrichtung eingeben kann, und über deren Bildschirm Informationen an den Benutzer ausgegeben werden können, wobei die Steuereinrichtung dazu programmiert ist,
i) zur Ausgabe einer Anleitung zum Reinigen der Inkubatorkammer mindestens eine Reinigungsanweisung als visuelle Information für den Benutzer am Bildschirm auszugeben,
ii) eine Eingabe des Benutzers an der Benutzerschnittstelleneinrichtung zu erfassen, mit der der Benutzer die Durchführung der Reinigung gemäß der Reinigungsanweisung bestätigt,
iii) in Abhängigkeit von der Eingabe des Benutzers die Sensoreinrichtung automatisch zu aktivieren,
iv) mittels der Sensoreinrichtung mindestens einen Messwert zu erfassen, der die Konzentration der in den Reinigungsmittelrückständen enthaltenen VOCs charakterisiert.

Die in Schritt i) genannte Anleitung ist insbesondere eine mehrschrittige, computergestützte Instruktion des Benutzers, die insbesondere mittels eines Assistenten realisierbar ist. Der Begriff Assistent bezeichnet hier eine am Bildschirm anzeigbare Oberfläche, mittels der ein Anwender durch mehrere Dialoge für eine ergonomische Datenein-/ausgabe geführt wird. Es wird eine Hilfestellung gegeben, welche Schritte zur Durchführung eines vorbestimmten Reinigungsprozesses vom Benutzer manuell durchgeführt werden müssen. Ein abschließender Schritt der mehrschrittigen Instruktion kann die Anzeige eines Bestätigungselement auf der anzeigbaren Benutzeroberfläche sein. Ein Beispiel für ein Betätigungselement ist eine Checkbox. Durch Markierung der Checkbox durch den Benutzer bestätigt dieser z.B. die Durchführung der Reinigung. Dies führt vorzugsweise auch zur Aktivierung der Sensoreinrichtung und erzielt damit den Vorteil einer objektiven Überprüfung, ob eine Reinigung durchgeführt wurde. Dies ist vorteilhaft zur Dokumentation von GMP konformen Prozessen, da durch die Sensoreinrichtung in Zusammenspiel mit der Steuereinrichtung exportierbare Datensätze generiert werden.

Der Inkubator weist vorzugsweise eine elektronische Steuereinrichtung und eine Benutzerschnittstelleneinrichtung auf, mittels der eine Benutzereingabe in Form von Daten erfassbar ist, und Informationen an den Benutzer ausgebbar sind, wobei die Steuereinrichtung dazu programmiert ist,
i) mittels der Benutzerschnittstelleneinrichtung eine Eingabe des Benutzers zu erfassen, mit der dieser das Aktivieren der Sensoreinrichtung anfordert,
ii) in Abhängigkeit von der Eingabe des Benutzers die Sensoreinrichtung automatisch zu aktivieren,
iii) mittels der Sensoreinrichtung mindestens einen Messwert zu erfassen, der die Konzentration der in den Reinigungsmittelrückständen enthaltenen VOCs charakterisiert.

Auf diese Weise kann ein Benutzer auf Wunsch eine VOC-Messung mittels der Sensoreinrichtung auslösen, und, falls diese Funktion implementiert ist, auch die Ausgabe einer Information über den Status des Innenraums hinsichtlich der Konzentration der aus den Reinigungsmittelrückständen resultierenden VOCs auslösen.

Der Inkubator weist vorzugsweise einen Temperatursensor und insbesondere eine Temperaturregelung auf, der/die insbesondere zum Erfassen einer Temperatur einer Wasserwanne des Inkubators eingerichtet ist oder der/die zur Feststellung einer Temperatur der Inkubatorkammer eingerichtet ist oder zur Feststellung der Wandtemperatur der Kammer unterhalb der Wasserwanne, und weist vorzugsweise eine elektronische Steuereinrichtung auf, die dazu programmiert ist,
i) in Abhängigkeit von der mittels des Temperatursensors erfassten Temperatur, insbesondere bei Überschreiten oder Unterschreiten eines vordefinierten Schwellenwertes, die Sensoreinrichtung automatisch zu aktivieren,
ii) mittels der Sensoreinrichtung mindestens einen Messwert zu erfassen, der die Konzentration der in den Reinigungsmittelrückständen enthaltenen VOCs charakterisiert.

Wurde beispielsweise ermittelt, dass die Temperatur im Kammerinneren für eine bestimmte Mindestzeit auf einen niedrigeren Wert (z. B. Raumtemperatur) als den gesetzten Sollwert (z.B. 37 °C im Kammerinneren) abgesunken war, kann dies so interpretierbar sein, dass die Inkubatortüre eine lange Zeit offenstand, um eine manuelle Reinigung mittels Reinigungsmittel durchzuführen. Nach diesem Vorgang ist die Messung von Reinigungsmittelrückständen/VOCs sinnvoll. Wurde beispielsweise ermittelt, dass die Temperatur an der Kammerwand unterhalb der Wasserwanne für eine bestimmte Mindestzeit auf einen niedrigeren Wert (z. B. Raumtemperatur) als den gesetzten Sollwert (z.B. 37 °C im Kammerinneren) abgesunken war, kann dies so interpretierbar sein, dass die Wasserwanne entfernt wurde um diese selber einer manuellen Reinigung mittels Reinigungsmittel zu unterziehen. Nach diesem Vorgang ist die Messung von Reinigungsmittelrückständen/VOCs sinnvoll. Zu einem Temperaturabfall an der Kammerwand unterhalb der Wasserwanne kommt es insbesondere dann, wenn eine Nutzer frisches Wasser in die Wasserwanne einfüllt. Dies erfolgt üblicherweise nach der Reinigung der Wasserwanne und/oder der Reinigung des gesamten Inkubators. Da Kontaminationen ihren Ursprung in der Wasserwanne haben können, ist es auch üblich, die Wasserwanne des Inkubators häufiger zu reinigen als den Rest des Inkubators. Für diese Zwischenreinigung, d.h. eine alleinige Reinigung der Wasserwanne des Inkubators, wird die Wasserwanne aus dem Inkubator entnommen, das Wasser darin ausgeleert und anschließend wird die Wanne mechanisch ausgewischt, wobei die üblichen Reinigungsmittel eingesetzt werden. Anschließend wird die Wanne wieder in die Inkubatorkammer gestellt und mit frischem Wasser befüllt. Das erfindungsgemäße Verfahren wird auch bei dieser Zwischenreinigung angewendet und erlaubt in diesem Fall die objektive Dokumentation der Reinigung, insbesondere der Reinigung der Wasserwanne.

Der Inkubator weist vorzugsweise eine Heizeinrichtung zur Durchführung einer automatischen Sterilisation der Inkubatorkammer während mindestens einer Hochtemperaturphase auf, insbesondere bei Temperaturen zwischen 100 °C und 200°C (inklusive dieser Werte), und weist vorzugsweise eine elektronische Steuereinrichtung auf, die dazu programmiert ist,
i) die Sensoreinrichtung vor, während oder nach der Durchführung der automatischen Sterilisation der Inkubatorkammer automatisch zu aktivieren,
ii) mittels der Sensoreinrichtung mindestens einen Messwert zu erfassen, der die Konzentration der in den Reinigungsmittelrückständen enthaltenen VOCs charakterisiert.

Im Zusammenhang mit dem Vorgang einer Hochtemperaturphase ist die Messung von Reinigungsmittelrückständen/VOCs sinnvoll, da dann auch die Wahrscheinlichkeit hoch ist, dass eine Reinigungsmittelrückstände erzeugende Reinigung der Inkubatorkammer erfolgt ist, erfolgt bzw. erfolgen wird.

Der Inkubator weist vorzugsweise eine Benutzerschnittstelleneinrichtung auf, mittels der eine Benutzereingabe in Form von Daten erfassbar ist, und Informationen an den Benutzer ausgebbar sind, eine elektronische Steuereinrichtung aufweist, die dazu programmiert ist,
i) die Sensoreinrichtung automatisch zu aktivieren,
ii) mittels der Sensoreinrichtung mindestens einen Messwert zu erfassen, der die Konzentration der in den Reinigungsmittelrückständen enthaltenen VOCs charakterisiert,
iii) den mindestens einen Messwert mit einem vorbestimmten Schwellenwert zu vergleichen, der typisch ist für eine gemäß einer reinigungsmittelbasierten, in vorbestimmter Weise ausgeführten Reinigung der Inkubatorkammer,
iv) in Abhängigkeit vom Ergebnis dieses Vergleichs dem Benutzer mittels der Benutzerschnittstelleneinrichtung eine Information auszugeben, dass eine in vorbestimmter Weise ausgeführte Reinigung der Inkubatorkammer erfasst wurde oder nicht erfasst wurde.

Beim Inkubator kann auf diese Weise erfasst werden, ob vom Benutzer eine Reinigung (ordnungsgemäß) durchgeführt wurde, oder ob diese eventuell ausgelassen wurde oder nicht ordnungsgemäß durchgeführt wurde. Es kann auf diese Weise ein digitales Reinigungslogbuch erstellt und in einer Datenspeichereinrichtung gespeichert werden, welches über die Reinigungshistorie Auskunft gibt. Dies ist von Vorteil für die Qualitätssicherung, insbesondere um nachzuweisen, dass die gute Herstellerpraxis (GMP) eingehalten wurde. Die Produktion nach GMP Richtlinien erfordert die Reinhaltung des Inkubators sowie auch die Dokumentation einer solchen Reinigung. Die vorliegende Erfindung ist vorteilhaft für ein GMP-gerechtes Qualitätsmanagementsystem, da mittels der erfindungsgemäßen Vorrichtung und dem damit durchgeführten Verfahren erfasst wird, ob tatsächlich eine Reinigung durchgeführt wurde und darüber auch ein Datensatz erstellt wird. Die vorliegende Erfindung ermöglicht somit eine objektive Prüfung, ob eine Reinigung des Inkubators erfolgt ist.

Die Erfindung betrifft auch ein Verfahren zur Detektion einer Anreicherung von in Reinigungsmittelrückständen enthaltenen flüchtigen organischen Verbindungen (VOCs) in einem Inkubator, insbesondere einem Inkubator gemäß der Erfindung und dessen möglichen Ausgestaltungen, welcher der Inkubation lebender Zellkulturen dient und der aufweist:
- eine Inkubatorkammer (2) zur Aufnahme von Objekten, insbesondere Zellkulturbehältern, in einem verschließbaren Innenraum der Inkubatorkammer, der eine kontrollierbare Gasatmosphäre aufweist,
- eine Sensoreinrichtung (11; 21; 31; 41) zur Detektion einer Anreicherung von in den Reinigungsmittelrückständen enthaltenen flüchtigen organischen Verbindungen (VOCs), wobei die Sensoreinrichtung mindestens einen VOC-Sensor (11) zur Detektion der VOCs aufweist und der mindestens eine VOC-Sensor mindestens einen Messbereich aufweist, der in Strömungsverbindung mit dem Atmosphärengas des Innenraums stehend angeordnet ist,
wobei das Verfahren die mittels einer elektronischen Steuereinrichtung des Inkubators auszuführenden Schritte aufweist:
A) Aktivieren der Sensoreinrichtung,
B) Erfassen mindestens eines Messwertes, der die Konzentration der in den Reinigungsmittelrückständen enthaltenen VOCs charakterisiert.

Das Verfahren weist vorzugsweise die Schritte auf:
A1) Erfassen eines Öffnens und/oder Schließens der Inkubatortüre mittels eines Türsensors des Inkubators in Form eines Türstatusparameters,
A2) Durchführen des Schrittes A) in Abhängigkeit von dem Türstatusparameter, insbesondere Durchführen des Schrittes A) nach dem Schließen der Inkubatortüre.

Vorzugsweise erfolgt die Aktivierung in Schritt A2), wenn eine zeitabhängige Auswertung des Türparameters ergibt, dass die Inkubatortüre für mindestens die Dauer einer vorbestimmten, insbesondere in einer Datenspeichereinrichtung des Inkubators gespeicherten, Zeitspanne geöffnet war, und dass die Aktivierung in Schritt A2) insbesondere nicht erfolgt, wenn die zeitabhängige Auswertung des Türparameters ergibt, dass die Inkubatortüre für weniger als die Dauer der vorbestimmten Zeitspanne geöffnet war. Übliche Zeitspannen sind Zeitspannen von mehr als 2 Minuten, vorzugsweise mehr als 5 min. Die Zeitspanne kann auch gewählt sein aus den bevorzugten Bereichsspannen 1 min bis 45 min, 2 min bis 45 min, 5 min bis 45 min, 10 min bis 45 min. Die oberen Grenzen der Bereichsangaben können auch variiert werden, ebenso die unteren Grenzen. Die Zeitspanne ist typisch für die Dauer einer Reinigung. Die obere Grenze kann weggelassen werden.

Das Verfahren weist vorzugsweise die Schritte auf:
A1) Ausgabe einer Anleitung zum Reinigen der Inkubatorkammer, beinhaltend mindestens eine Reinigungsanweisung als visuelle Information für den Benutzer an einem Bildschirm des Inkubators,
A2) Erfassen einer Eingabe des Benutzers an einer Benutzerschnittstelleneinrichtung des Inkubators, wobei der Benutzer mittels der Eingabe die Durchführung der Reinigung gemäß der Reinigungsanweisung bestätigt,
A3) automatisches Aktivieren der Sensoreinrichtung gemäß Schritt A) in Abhängigkeit von der Eingabe des Benutzers.

Das Verfahren weist vorzugsweise die Schritte auf:
A1) Erfassen einer Eingabe des Benutzers mittels der Benutzerschnittstelleneinrichtung, wobei der Benutzer mit dieser Eingabe das Aktivieren der Sensoreinrichtung anfordert,
A2) in Abhängigkeit von der Eingabe des Benutzers den Schritt A) durchzuführen.

Das Verfahren weist vorzugsweise die Schritte auf:
A1) Durchführen des Schrittes A) in Abhängigkeit von einer mittels eines Temperatursensors des Inkubators erfassten Temperatur, insbesondere bei Überschreiten oder Unterschreiten eines vordefinierten Schwellenwertes.

Das Verfahren weist vorzugsweise die Schritte auf:
A1) Automatisches Durchführen von Schritt A) vor, während oder nach einer Durchführung einer automatischen Sterilisation der Inkubatorkammer, wobei bei dieser Sterilisation die Inkubatorkammer mittels einer Heizeinrichtung des Inkubators während mindestens einer Hochtemperaturphase, insbesondere auf Temperaturen zwischen 100 °C und 200°C (inklusive dieser Werte), aufgeheizt wird.

Das Verfahren weist vorzugsweise die Schritte auf:
C) Vergleich des mindestens einen Messwerts mit einem vorbestimmten Schwellenwert, der typisch ist für eine gemäß einer reinigungsmittelbasierten, in vorbestimmter Weise ausgeführten Reinigung der Inkubatorkammer,
D) Ausgabe einer Information an den Benutzer mittels einer Benutzerschnittstelleneinrichtung des Inkubators in Abhängigkeit vom Ergebnis dieses Vergleichs in C), wobei die Information besagt, dass eine in vorbestimmter Weise ausgeführte Reinigung der Inkubatorkammer erfasst wurde oder nicht erfasst wurde.

Die Erfindung betrifft unter anderem auch die Verwendung einer Sensoreinrichtung zur Detektion einer Anreicherung von in Reinigungsmittelrückständen enthaltenen flüchtigen organischen Verbindungen (VOCs), die in der Inkubatorkammer eines Inkubators vorhanden sind, welcher der Inkubation lebender Zellkulturen dient, wobei die Sensoreinrichtung mindestens einen VOC-Sensor zur Detektion der VOCs aufweist.

Weitere bevorzugte Ausgestaltungen der Erfindung ergeben sich wie folgt:
Die Sensoreinrichtung weist vorzugsweise eine Mehrzahl N mit N>1 von VOC-Sensoren, vorzugsweise eine Vielzahl N>=10 von VOC-Sensoren auf. Vorzugsweise ist die Anzahl der VOC-Sensoren der Sensoreinrichtung beschränkt auf eine Maximalzahl M=2000, 100, 50, 25 oder 10. Die VOC-Sensoren können alle typenunterschiedlich sein, wodurch sich ein Spektrum an typenunterschiedlichen Messergebnissen und damit eine bessere Differenzierbarkeit der VOC-Detektion oder einer möglichen VOC-Erkennung bzw. einer möglichen VOC-Klassifizierung ergibt. Es können auch mehrere VOC-Sensoren desselben Typs vorgesehen sein. Beispielsweise ist aus der Biologie bekannt, dass die menschliche Nase ca. 380 unterschiedliche Rezeptortypen verwendet, andere Säugetiere eine deutlich höhere Anzahl, so dass dieser Ansatz als technisch "bewährt" angesehen werden kann.

Vorzugsweise ist eine Sensoreinrichtung als elektronische Nase eingerichtet, bei der eine Mehr- oder Vielzahl von VOC-Sensoren, insbesondere zeitgleich, die Gasatmosphäre des Innenraums messen, insbesondere indem zeitgleich die Anlagerung von VOCs aus der Gasatmosphäre an die Messbereiche der VOC-Sensoren erfasst wird. Insbesondere ist eine elektronische Nase geeignet bzw. dazu eingerichtet, dass gesammelte Messsignale der Mehr- oder Vielzahl N von VOC-Sensoren, insbesondere jedes Messsignal eines jeden der VOC-Sensoren, gemeinsam ausgewertet werden, um einen Reinigungsmittelrückstand zu detektieren, insbesondere die Art oder eine Klasse eines Reinigungsmittelrückstands zu erkennen, vorzugsweise auch zu quantifizieren. Eine elektronische Nase ist insbesondere dazu eingerichtet, mindestens zwei in der Gasatmosphäre vorhandene VOCs zu unterscheiden. Eine elektronische Nase ist insbesondere dazu eingerichtet, zwischen verschiedenen Arten oder Klassen von Reinigungsmittelrückständen zu differenzieren. Ferner kann mittels mehrerer VOC-Sensoren eine zuverlässigere und damit auch empfindlichere Detektion von Reinigungsmittelrückständen erreicht werden als mit einem einzigen VOC-Sensor.

Vorzugsweise weist die Sensoreinrichtung mindestens einen VOC-Sensor auf, der für die Detektion von gasförmigen Stoffwechselprodukten von Mikroorganismen, insbesondere Bakterien, Myoplasmen, Pilzen, Hefen eingerichtet ist. Vorzugsweise weist die Sensoreinrichtung mehrere VOC-Sensoren, insbesondere unterschiedlicher Selektivität der Detektion auf.

Vorzugsweise weist die Sensoreinrichtung mindestens einen VOC-Sensor auf, der für die Detektion von gasförmigen Reinigungsmittelrückständen, insbesondere den Zerfallsprodukten bzw. Zersetzungsprodukte der Reinigungsmittel eingerichtet ist. Die Zerfallsprodukte oder Zersetzungsprodukte können insbesondere nach der thermischen Sterilisation entstehen, die sich üblicherweise an das mechanische Reinigen unter Einsatz von Reinigungsmitteln anschließt.

Unterschiedliche Reinigungsmittel erzeugen unterschiedliche volatile Rückstände, die mittels bestimmter, insbesondere kommerziell erhältlicher Sensoren detektierbar sind. Eine Auflistung detektierbarer volatiler Substanzen ist z.B. auf folgender Internetseite auffindbar: https://www.gas-sensing.com/information/volatile organic compound

Nachfolgend gezeigten Tabelle nennt beispielhaft bekannte Reinigungsmittel und mögliche Gassensoren:

| **Reinigungsmittel** | **Gasförmige Nebenprodukte/Reaktionsprodukte/ Zersetzungsprodukte Verdunstung** | **Mögliche Gas-Sensoren** |
|---|---|---|
| Alkohole (Ethanol, Isopropanol) | Verdunstung | MQ2 |
| | | MQ3 |
| | | MQ135 |
| Hypochloride (Bleichmittel, "Chlorreiniger") | Chlor | Dräger Sensor XXS |
| | | Cl2 |
| | | Bürkert Typ 8232 |
| | | EC-Sense EC4-CL2 |
| | | EC-Sense ES1-CL2 |
| | | Figaro FECS45 |
| | | Rainbow RKCL2 |
| Phenole | Verdunstung | Environ Sci Technol 2006 Oct 1;40(19):6058-63. doi: 10.1021/es052322e. |
| Quartäre Ammoniumverbindungen (zB.: kationische Tenside, Benzalkoniumchlorid, Distearyldimethylammonium-chlorid, Didecyldimethylammonium-chlorid, | Thermische Zersetzung zu : Kohlenmonoxid, Stickoxide, Chlorwasserstoff, Ammoniak | Dräger X-am^{®} 8000 |
| Peroxid Verbindungen | Sauerstoff | Amphenol SGX-4OX, Figaro KE-25 viele Weitere |

Die Reinigungsmittel, insbesondere die Reinigungsmittelrückstände, weisen vorzugsweise mindestens eine der folgenden Substanzen bzw. Flüssigkeiten auf, und/oder bestehen zu mindestens 5% aus einer der folgenden Substanzen: Alkohole, insbesondere Ethanol, Isopropanol; Hypochloride, insbesondere Bleichmittel, Chlorreiniger; Phenole; Quartäre Ammoniumverbindungen, insbesondere kationische Tenside, Benzalkoniumchlorid, Distearyldimethylammonium-chlorid, Didecyldimethylammonium-chlorid; Peroxid Verbindungen. Vorzugsweise besteht das Reinigungsmittel, insbesondere die Reinigungsmittelrückstände, aus einer der genannten Flüssigkeiten oder aus einer Mischung von mindestens zweien der genannten Flüssigkeiten. Dabei kann die Mischung auch aus Wasser, insbesondere destilliertem Wasser oder de-ionisiertem Wasser und mindestens einer der genannten Substanzen bestehen. Die Reinigungsmittel, insbesondere die Reinigungsmittelrückstände, weisen vorzugsweise mindestens eine der vorstehend genannten Substanzen in einer wässrigen Mischung auf, bei der mindestens eine dieser Substanzen mit Wasser gemischt ist, insbesondere mit einem Volumenanteil des Wassers von mindestens 10%, 30%, oder 50%. 70% Wasser und 30% Ethanol ist eine besonders bevorzugte Mischung für ein Reinigungsmittel.

Vorzugsweise weist die Sensoreinrichtung mindestens einen VOC-Sensor auf, der für die Detektion von mindestens einer zu den Alkoholen gehörenden chemischen Verbindung eingerichtet ist, vorzugsweise von mehreren unterschiedlichen, zu den Alkoholen gehörenden chemischen Verbindungen eingerichtet ist. In den dieser Erfindung zugrunde liegenden Forschungsarbeiten wurde festgestellt, dass sich Alkohole als Reinigungsmittelrückstände besonders sensibel detektieren lassen. Andererseits sind die entsprechenden kommerziell erhältlichen Alkoholsensoren geeignet empfindlich, um bereits geringe Alkoholspuren zu detektieren.

Vorzugsweise weist die Sensoreinrichtung mindestens einen VOC-Sensor auf, der für die Detektion von mindestens einer zu den Aromaten gehörenden chemischen Verbindung eingerichtet ist, vorzugsweise von mehreren unterschiedlichen, zu den Aromaten gehörenden chemischen Verbindungen eingerichtet ist. In den dieser Erfindung zugrunde liegenden Forschungsarbeiten wurde insbesondere auch festgestellt, dass sich im Rahmen einer Reinigungsmittelrückständedetektion auch Aromaten besonders sensibel detektieren lassen. Andererseits sind die entsprechenden kommerziell erhältlichen Aromatensensoren geeignet empfindlich, um bereits geringe Aromatenspuren zu detektieren.

Vorzugsweise weist die Sensoreinrichtung mindestens einen VOC-Sensor auf, der für die Detektion von mindestens einer zu den Alkanen gehörenden chemischen Verbindung eingerichtet ist, vorzugsweise von mehreren unterschiedlichen, zu den Alkanen gehörenden chemischen Verbindungen eingerichtet ist. In den dieser Erfindung zugrunde liegenden Forschungsarbeiten wurde insbesondere auch festgestellt, dass sich im Rahmen einer Reinigungsmittelrückständedetektion auch Alkane besonders sensibel detektieren lassen. Andererseits sind die entsprechenden kommerziell erhältlichen Alkansensoren geeignet empfindlich, um bereits geringe Alkanspuren zu detektieren.

Besonders bevorzugt ist der VOC-Sensor eingerichtet zur Erfassung von Alkoholen, und Aromaten und/oder Alkanen.

Vorzugsweise weist die Sensoreinrichtung mindestens einen VOC-Sensor auf, der für die Detektion von mindestens einer der chemischen Verbindungen eingerichtet ist, vorzugsweise von mehreren unterschiedlichen chemischen Verbindungen eingerichtet ist, die der Gruppe von chemischen Verbindungen umfassend {Alkohole, Aromaten, Benzole, Alkylbenzole, Alkane, Alkene, Alkane, Aldehyde, Ester, Ketone, Pyrazole, Oxime, Terpene, Säuren, Carbonsäuren (z.B. Phenol), Heterocyclische Amine und Indolen}. In den dieser Erfindung zugrunde liegenden Forschungsarbeiten wurde insbesondere auch festgestellt, dass sich im Rahmen einer Reinigungsmittelrückständedetektion diese Stoffe besonders sensibel detektieren lassen. Andererseits sind die entsprechenden kommerziell erhältlichen Sensoren für verschieden Stoffe geeignet empfindlich, um bereits geringe dieser Stoffspuren zu detektieren.

Ein VOC-Sensor ist insbesondere ein chemischer Sensor für die Detektion von VOCs in einer Gasatmosphäre. Die in der Gasatmosphäre detektierten, gleichartigen oder unterschiedlichen VOCs werden vom Sensor detektiert und in ein elektrisches Signal umgewandelt.

Ein VOC-Sensor kann ein Leitfähigkeitssensor sein, der insbesondere eine in Abhängigkeit von mindestens einem VOC veränderliche elektrische Leitfähigkeit misst. Der VOC-Sensor ist besonders bevorzugt ein Metalloxid-Halbleiter (MOX) Gassensor, auch kurz als MOX-Sensor bezeichnet. Solche chemischen Sensoren besitzen eine Detektionsschicht, mithilfe der eine chemische Interaktion in ein elektrisches Signal transformiert werden kann. Sie sind für einen kontinuierlichen Messbetrieb geeignet.

Die Funktion eines MOX-Sensors basiert insbesondere darauf, dass sich abhängig von der Konzentration des Zielgases die elektrische Leitfähigkeit der gassensitiven Metalloxidschicht bzw. des Halbleiters verändert und damit die Anwesenheit als auch Menge des Zielgases bestimmt wird. Typischerweise besteht ein MOX-Sensor aus vier Elementen: Gassensitive Metalloxidschicht, Elektroden, Heizelement und Isolierungsschicht. Das Heizelement ist von der gassensitiven Metalloxidschicht und den Kontaktelektroden durch die Isolierungsschicht getrennt. Die gassensitive Metalloxidschicht wird durch das Heizelement erhitzt und Sauerstoffmoleküle aus der Umgebung werden an der Oberfläche der gassensitiven Metalloxidschicht adsorbiert. Die adsorbierten Sauerstoffmoleküle fangen Elektronen von den leitenden Bändern des Halbleiters ein und es bilden sich energetische Barrieren, die so einen Teil des Elektronenflusses im Halbleiter blockieren und somit die elektrische Leitfähigkeit verschlechtern bzw. den Widerstand des Gassensors erhöhen. Sobald reduzierende Gase (Zielgase) präsent sind, reagieren diese mit den gebundenen Sauerstoffmolekülen. Die Sauerstoffmoleküle werden von der Oberfläche der gassensitiven Metalloxidschicht gelöst und die Leitfähigkeit steigt bzw. der Widerstand sinkt.

Ein VOC-Sensor kann ein kapazitativer Sensor sein, der insbesondere eine in Abhängigkeit von mindestens einem VOC veränderliche elektrische Kapazität erfasst. Ein VOC-Sensor kann ein optischer Sensor sein, der insbesondere eine in Abhängigkeit von mindestens einem VOC veränderliche optische Eigenschaft misst, z.B. einen sich ändernden Brechungsindex, eine sich ändernde Lichtintensität, oder ein sich änderndes Lichtspektrum, wobei die Wellenlänge des verwendeten Lichtes nicht beschränkt ist und insbesondere auch Infrarot umfasst. Ein VOC-Sensor kann ein massensensitiver Sensor sein, der insbesondere eine in Abhängigkeit von mindestens einem VOC veränderliche Masse misst, z.B. durch Erfassung einer veränderten Schwingung eines mit dem mindestens einen VOC wechselwirkenden Schwingkörpers.

Die Sensoreinrichtung ist vorzugsweise ein Bestandteil des Inkubators, und ist insbesondere in einem Gehäuse oder Gehäuseteil des Inkubators angeordnet.

Vorzugsweise ist eine elektronische Auswertungseinrichtung vorgesehen, die vorzugsweise eine Datenverarbeitungseinrichtung beinhaltet. Die Auswertungseinrichtung ist dazu eingerichtet, mindestens ein Messsignal, insbesondere Messwert, des mindestens einen VOC-Sensors zu erfassen und insbesondere auszuwerten. Die Auswertungseinrichtung ist vorzugsweise ein Bestandteil des Inkubators, und ist insbesondere in einem Gehäuse oder Gehäuseteil des Inkubators angeordnet. Die Auswertungseinrichtung kann auch Bestandteil der Sensoreinrichtung sein, insbesondere, wenn die letztere als Teil eines Nachrüstsystems für einen Inkubator eingerichtet ist, der insbesondere eine solche Sensoreinrichtung noch nicht aufweist.

Die Auswertungseinrichtung, insbesondere der Inkubator, weist vorzugsweise eine Datenspeichereinrichtung auf, die mit der Datenverarbeitungseinrichtung zum Austausch von Daten verbunden ist.

Die Auswertungseinrichtung, insbesondere die Datenverarbeitungseinrichtung, ist vorzugsweise mit einem Programmcode programmierbar, und insbesondere dazu programmiert, die folgenden Schritte, insbesondere gemäß diesem Programmcode, auszuführen:
- Empfangen mindestens eines Messsignals, insbesondere von Messdaten, mindestens eines VOC-Sensors; insbesondere: Empfangen einer Folge von Messsignalen zeitlich, insbesondere für die Dauer einer Messzeit Δt, nacheinander, die insbesondere den zeitlichen Verlauf der Messung mindestens eines VOC-Sensors bilden;
- Vergleich des mindestens einen Messsignals mit mindestens einem Referenzwert;
- Entscheiden, anhand des Ergebnisses dieses Vergleichs, ob eine Veränderung der VOC-Konzentration in der Gasatmosphäre des Innenraums vorliegt, insbesondere eine Veränderung, die für Reinigungsmittelrückstände im Innenraum charakteristisch ist.

Der genannte mindestens eine Referenzwert kann vorbestimmt sein und kann in der Datenspeichereinrichtung gespeichert sein. Der genannte mindestens eine Referenzwert kann auch ein Startwert sein, der aus einer Messung zu Beginn der Messzeit Δt resultiert, während der der mindestens eine VOC-Sensor die VOC-Moleküle in der Gasatmosphäre des Innenraums erfasst. Der Startwert eines VOC-Sensors kann insbesondere dann erfasst werden, wenn der Messbereich des jeweiligen VOC-Sensors initialisiert wurde, insbesondere indem der Messbereich mit einem Spülgas, z.B. Stickstoff N2, gespült wurde. Dieses Spülen erfolgt vorzugsweise solange, bis der zeitliche Verlauf des Messsignals des mindestens einen VOC-Sensors konstant ist oder einen bekannten Referenzverlauf aufweist, z.B. linear zunehmend oder abnehmend ist.

Die Auswertungseinrichtung, insbesondere die Datenverarbeitungseinrichtung, ist vorzugsweise dazu programmiert, die folgenden Schritte, insbesondere gemäß diesem Programmcode, auszuführen:
- Empfangen eines Messsignals, insbesondere eines Messignals, das einen Widerstandswert, insbesondere einen elektrischen Widerstand oder eine elektrische Leitfähigkeit des Messbereichs repräsentiert, insbesondere von Messdaten, des MOX-Sensors; insbesondere: Empfangen einer Folge von Messsignalen zeitlich, insbesondere für die Dauer einer Messzeit Δt, nacheinander, die insbesondere den zeitlichen Verlauf der Messung des eines MOX-Sensors bilden;
- vorzugsweise: Auswerten des Messignals oder der Messignale, um daraus erneut ein oder mehrere Messsignale zu erhalten;
- Vergleich des Messsignals oder der Messsignale mit mindestens einem Referenzwert;
- Entscheiden, anhand des Ergebnisses dieses Vergleichs, ob eine Veränderung der VOC-Konzentration in der Gasatmosphäre des Innenraums vorliegt, insbesondere eine Veränderung, die für Reinigungsmittelrückstände im Innenraum charakteristisch ist.

Eine Steuereinrichtung des Inkubators, insbesondere eine Datenverarbeitungseinrichtung ist vorzugsweise dazu eingerichtet und/oder programmiert, einen VOC-Sensor -oder mehrere- mit einer konstanten Spannung anzusteuern.

Besonders bevorzugt ist eine Steuereinrichtung des Inkubators, insbesondere eine Datenverarbeitungseinrichtung dazu eingerichtet und/oder programmiert, einen VOC-Sensor - oder mehrere- mit sich periodisch ändernder Spannung anzusteuern. Da die Ausbeute chemischer Reaktionen an einem VOC-Sensor von dieser Spannung abhängen kann, oder sogar VOC-spezifisch abhängen kann, ist diese periodische Ansteuerung ein vorteilhafter Betriebsmodus der Sensoreinrichtung.

Eine Steuereinrichtung des Inkubators, insbesondere eine Datenverarbeitungseinrichtung ist vorzugsweise dazu eingerichtet und/oder programmiert, ein Heizelement eines MOX-Sensors mit einer sich periodisch ändernden Spannung anzusteuern, um an der Metalloxidoberfläche eine sich entsprechend periodisch ändernde Temperatur zu erzeugen. Diese Betriebsart einer Sensoreinrichtung wird auch als "temperature cycled operation" ("TCO") bezeichnet. Dazu wird der Heizer hier mit einer, einen stufenförmigen Verlauf aufweisenden, Spannung U-H-Soll angesteuert, die je Heizperiode T mehrere verschiedene Werte vorsieht. Jeder dieser Spannungswerte wird vorzugsweise für einen vorgegebenen Anteil der Heizperiode T eingestellt. Die Heizperiode beträgt vorzugsweise zwischen 1 Sekunden [s] und 60 s, vorzugsweise zwischen 5 s und 30 s, vorzugsweise zwischen 15 s und 25 s, vorzugsweise zwischen 17 s und 23 s, und liegt hier bei 20 s. Aus der Ansteuerung ergibt sich ein sich periodisch änderndes Messsignal mit der Messperiode T. Die Heizperiode kann insbesondere in Abhängigkeit von der thermischen Masse des VOC-Sensors gewählt sein.

Im Falle eines periodischen Messignals erfolgt die Auswertung vorzugsweise, indem eine oder vorzugsweise mehrere Perioden des Messignals statistisch ausgewertet werden, um ein (ausgewertetes) Messsignal zu erhalten. Vorzugsweise ist die Datenverarbeitungseinrichtung dazu programmiert, einen durchschnittlichen Verlauf einer Messperiode zu ermitteln. Dies kann beinhalten, dass die Werte von Messsignalen einer Anzahl M von Messperioden superpositioniert werden und dieser addierte Periodenverlauf dann mit der inversen Anzahl 1/M multipliziert wird. Auf diese Weise wird ein Messsignal geglättet und der Einfluss von Messartefakten wird reduziert. Es kann auch ein Medianfilter zur Glättung verwendet werden. Eine Glättung kann auch erfolgen, dass mehrere aufeinanderfolgende Messsignale, insbesondere einer Messperiode, als Mittelwert zusammengefasst werden, wodurch die Anzahl der Messsignale (dann Mittelwerte) einer Messkurve reduziert wird; dies ist auch als gleitender Mittelwert bekannt.

Vorzugsweise ist die Datenverarbeitungseinrichtung dazu programmiert, aus dem Signal einer einzigen Messperiode oder aus einem durchschnittlichen Verlauf einer Messperiode mindestens einen Sekundärwert abzuleiten, der sich auf eine als Sekundärfeature bezeichnete Charakteristik der Messperiode bezieht. Es kann ein Sekundärwert eine Steigung sein, die zu einem charakteristischen Zeitpunkt der Messperiode vorliegt, beispielsweise dem Zeitpunkt des Umstellens des Spannungswertes. Die charakteristische Steigung kann insbesondere kurz vor oder nach diesen Zeitpunkten erfasst werden, da die Steigung beim Umstellen undefinierbar sein kann. Zum Zwecke der weiteren Auswertung kann der Sekundärwert als das neue Messsignal, wie beschrieben, mit mindestens einem Referenzwert für diesen Sekundärwert verglichen werden.

Vorzugsweise ist die Datenverarbeitungseinrichtung dazu programmiert, einen Mittelwert mehrerer Messsignale zu ermitteln, insbesondere, einen Mittelwert mehrerer oder im Wesentlichen aller Messsignale einer Messperiode zu ermitteln. Zum Zwecke der weiteren Auswertung kann der Mittelwert, wie beschrieben, mit mindestens einem Referenzwert für diesen Mittelwert verglichen werden.

Der Inkubator, insbesondere die Sensoreinrichtung, weist vorzugsweise eine elektronische Steuereinrichtung auf, die insbesondere eine zweite Datenverarbeitungseinrichtung aufweisen kann oder die die Datenverarbeitungseinrichtung der Sensoreinrichtung verwendet und die dazu programmiert ist, mindestens eine Ventil zu steuern, durch dessen Öffnen ein Spülgas, insbesondere N2, über den Messbereich des mindestens einen VOC-Sensors strömt. Dieses Ventil kann insbesondere in Abhängigkeit von den Messignalen des mindestens einen VOC-Sensors geöffnet und/oder geschlossen werden.

Die Auswertungseinrichtung, insbesondere die Datenverarbeitungseinrichtung, ist vorzugsweise programmierbar, und insbesondere dazu programmiert, die folgenden Schritte auszuführen:
- Empfangen mindestens eines Messwerts, insbesondere von Messdaten, von mindestens zwei, mehreren oder allen einer Mehr- oder Vielzahl von VOC-Sensoren; insbesondere für jeden dieser mindestens zwei VOC-Sensoren;
- insbesondere zeitlich parallel: Empfangen einer Folge von Messwerten mindestens eines VOC-Sensors zeitlich, insbesondere für die Dauer einer Messzeit Δt, nacheinander, die insbesondere den zeitlichen Verlauf der Messung mindestens eines VOC-Sensors bilden;
- Vergleich des mindestens einen Messwerts jedes der mindestens zwei VOC-Sensoren mit mindestens einem Referenzwert;
- Entscheiden, anhand des Ergebnisses dieses Vergleichs, ob eine Veränderung der VOC-Konzentration in der Gasatmosphäre des Innenraums vorliegt, insbesondere eine Veränderung, die für Reinigungsmittelrückstände im Innenraum charakteristisch ist.

Der mindestens eine Referenzwert kann insbesondere dadurch ermittelt werden, dass, in einem Zeitraum vor oder nach der Messung des Gasvolumens der Inkubatoratmosphäre, der mindestens eine Messbereich des mindestens einen VOC-Sensors mit einem Spülgas, insbesondere Stickstoff, gespült wird und die Referenzmessung zur Erfassung eines oder mehrerer Referenzwerte am mit Spülgas gespülten Messbereich durchgeführt wird. Der Vergleich von Messwert und Referenzwert -unter optional zusätzlicher Berücksichtigung eines routinemäßig wählbaren Toleranzwertes, führt zuverlässig zur Detektion von VOCs in der Gasatmosphäre einer Inkubatorkammer.

Wie im Rahmen der dieser Erfindung zugrunde liegenden Forschungsarbeiten herausgefunden wurde, lassen sich Anreicherungen von VOCs, also Reinigungsmittelrückstände, in der Gasatmosphäre einer Inkubatorkammer gut nachweisen, da durch die Verdunstung von flüssigen Reinigungsmittelrückständen an Oberflächen des Innenraums eine exponentiell wachsende Anreicherung der Reinigungsmittelrückstände in der Gasatmosphäre mit entsprechend exponentiell wachsender VOC-Freisetzung existiert.

Die Auswertungseinrichtung, insbesondere die Datenverarbeitungseinrichtung, ist vorzugsweise programmierbar, und insbesondere dazu programmiert, die folgenden Schritte oder mindestens einen der folgenden Schritte auszuführen:
- optional: Erzeugen von Ergebnisdaten, die die Information darüber enthalten, ob eine Veränderung der VOC-Konzentration in der Gasatmosphäre des Innenraums vorliegt, insbesondere eine Veränderung, die für Reinigungsmittelrückstände des Innenraums charakteristisch ist;
- optional: Ausgabe einer Information an einen Benutzer des Inkubators in Abhängigkeit von den Ergebnisdaten, ob eine Veränderung der VOC-Konzentration in der Gasatmosphäre des Innenraums vorliegt, insbesondere eine Veränderung, die für Reinigungsmittelrückstände des Innenraums charakteristisch ist; die Ausgabe dieser Information kann in Form von visuellen oder akustischen Signalen erfolgen, die mittels einer geeigneten Ausgabeeinrichtung des Inkubators, insbesondere eines Display, und/oder eines Lautsprechers, erzeugt werden. Diese Ausgabe kann insbesondere als Warnsignal ausgestaltet sein, um den Benutzer vor einer bestehenden Kontamination zu warnen;
- optional: Speichern der Ergebnisdaten in der Datenspeichereinrichtung;
- optional: Übertragen der Ergebnisdaten an eine externe Datenverarbeitungseinrichtung, insbesondere an einen PC, ein Smartphone, einen Tabletcomputer, insbesondere mittels einer drahtgebundenen oder drahtlosen Signalverbindung.

Ein VOC-Sensor dient zur Detektion von flüchtigen organischen Verbindungen (VOCs), wobei der VOC-Sensor einen Messbereich aufweist, der in Strömungsverbindung mit dem Atmosphärengas des Innenraums stehend angeordnet ist. Vorzugsweise ist der Messbereich mindestens eines VOC-Sensors oder mehrerer oder aller VOC-Sensoren in der Inkubatorkammer angeordnet. Vorzugsweise ist der Messbereich mindestens eines VOC-Sensors oder mehrerer oder aller VOC-Sensoren in mindestens einer oder mehreren Messkammer(n) angeordnet. Die Innenraumatmosphäre der Messkammer steht vorzugsweise in Strömungsverbindung mit der Gasatmosphäre des Innenraums der Inkubatorkammer. Diese Strömungsverbindung kann gebildet sein, indem die Inkubatorkammer und die Messkammer durch mindestens einen Strömungskanal verbunden sind. Die Messkammer kann aber auch in der Inkubatorkammer angeordnet sein oder der Innenraum der Messkammer kann, ohne dass ein dedizierter Strömungskanal erforderlich wäre, unmittelbar münden in den Innenraum der Inkubatorkammer. Als Messkammer kann auch der Abschnitt eines durchströmten Strömungskanals angesehen werden, entlang dem der mindestens eine Messbereich des mindestens einen VOC-Sensors angeordnet ist. Eine Messkammer kann eine Zuströmöffnung aufweisen, durch die das zu messende Gas in die Messkammer eintritt, und insbesondere eine Abströmöffnung, durch die das zu messende Gas aus der Messkammer austritt.

Die Sensoreinrichtung weist insbesondere mindestens einen Temperatursensor und/oder einen Luftfeuchtesensor und/oder eine Drucksensor auf, der vorzugsweise zur Messung der Gasatmosphäre in der Messkammer angeordnet ist.

Der mindestens eine Messbereich des mindestens einen VOC-Sensors ist vorzugsweise in einem Abschnitt der Messkammer angeordnet, der, betrachtet entlang einer geraden Verbindung zwischen einem Zuströmöffnung und einer Abströmöffnung der Messkammer, zwischen dieser Zuströmöffnung und dieser Abströmöffnung liegt. Der mindestens eine Messbereich des mindestens einen VOC-Sensors ist vorzugsweise parallel einer Strömungsrichtung, insbesondere der Hauptströmungsrichtung in einer Messkammer angeordnet. Diese Strömungsrichtung folgt insbesondere einer Linie, insbesondere einer geraden Linie, welche die Zuströmöffnung und die Abströmöffnung der Messkammer verbindet. Der mindestens eine Messbereich des mindestens einen VOC-Sensors ist vorzugsweise parallel einer Seitenwand der Messkammer angeordnet und/oder bildet eine Seitenwand der Messkammer. Dabei ist eine Seitenwand insbesondere eine Innenwand der Messkammer, an der die Strömung des zu messenden Gases/der zu messenden Atmosphäre entlang strömt. Durch dieser Maßnahmen wird insbesondere erreicht, dass die Konzentration von VOCs aus der Gasatmosphäre entlang des Messbereichs möglichst konstant ist, indem ein durch Abscheidung von VOCs am Messbereich eventuell erschöpfte Gasatmosphäre durch originale Gasatmosphäre ersetzt wird.

Es ist möglich und bevorzugt, dass die Messbereiche mehrerer VOC-Sensoren parallel zueinander und insbesondere parallel zu einer Strömungsrichtung durch die Messkammer angeordnet sind. Es ist möglich und bevorzugt, dass die Messbereiche mehrerer VOC-Sensoren parallel zueinander und/oder insbesondere parallel zu einer Strömungsrichtung durch die Messkammer angeordnet sind. Messbereich können dabei einen Strömungsabschnitt durch die Messkammer umgeben, können insbesondere konzentrisch um diesen Strömungsabschnitt bzw. die Strömungsrichtung angeordnet sein. Messbereiche sind insbesondere so um den Strömungsabschnitt bzw. die Strömungsrichtung angeordnet, dass ein in der Messkammer befindlicher und durch die Messkammer strömender Gasvolumenabschnitt gleichzeitig an mehreren bzw. allen Messbereichen mehrerer VOC-Sensoren anliegt. Dadurch wird insbesondere sicher gestellt, dass an den Messbereichen dieselbe Gaszusammensetzung vorherrscht, und insbesondere nicht etwa ein vorgelagertes Abscheiden von VOCs an einem stromaufwärts gelegenen Sensor dazu führt, dass der stromabwärts gelegene Sensor eine andere Gaszusammensetzung misst.

Ein Strömungskanal ist insbesondere durch mindestens eine Gasleitung gebildet. Deren eines Ende mündet insbesondere in die Inkubatorkammer, und deren anderes Ende insbesondere in die Messkammer.

Mindestens eine Ventileinrichtung kann vorgesehen sein, um gesteuert durch eine elektronische Steuerungseinrichtung des Inkubators oder der Sensoreinrichtung, den Durchgang des mindestens einen Strömungskanals zu öffnen oder zu schließen, insbesondere auch zu drosseln. Die Ventileinrichtung kann insbesondere mindestens ein Wegeventil aufweisen, insbesondere mindestens ein 3/2-Wegeventil. Der Inkubator bzw. die Sensoreinrichtung kann insbesondere ein Spülgasreservoir aufweisen, in dem Spülgas, insbesondere N2 oder ein Edelgas, zum Spülen der Messkammer enthalten ist und das mittels des Ventils an den mindestens einen Strömungskanal angeschlossen ist. Das mindestens eine Wegeventil kann dazu eingerichtet sein, in einer ersten Schaltstellung den Durchgang durch das Ventil zwischen Inkubatorkammer und Messkammer zu öffnen und gleichzeitig den Durchgang durch das Ventil zwischen Spülgasreservoir und Messkammer zu schließen, und in einer zweiten Schaltstellung den Durchgang durch das Ventil zwischen Inkubatorkammer und Messkammer zu schließen und gleichzeitig den Durchgang durch das Ventil zwischen Spülgasreservoir und Messkammer zu öffnen. Anstelle eines Spülgasreservoirs kann auch ein Anschluss zum Zuführen von Spülgas vorgesehen sein, an dem ein Spülgasreservoir anschließbar ist oder ein Spülgasstrom (z.B. Förderung von -insbesondere gefilterter- Umgebungsluft) zuführbar ist.

Vorzugsweise weist der Inkubator bzw. die Sensoreinrichtung eine Gasfördereinrichtung, insbesondere ein Ventilator oder eine Pumpe auf, mittels der Gasatmosphäre aus der Inkubatorkammer durch den Strömungskanal in die Messkammer befördert wird. Die Messkammer weist vorzugsweise einen Strömungskanal, insbesondere mindestens eine Gasleitung, auf, durch den Gasatmosphäre aus der Messkammer aus dieser herausgeführt wird. Insbesondere wird Gasatmosphäre aus der Messkammer in die Umgebung des Inkubators geführt, insbesondere in einen Gehäusebereich des Inkubators, der zur Umgebung offen ist.

Es ist aber auch möglich und bevorzugt, dass ein Strömungskanal vorgesehen ist, der das aus der Messkammer austretende Gas zurück in die Inkubatorkammer führt. Dadurch wird ein Verlust von Inkubationsgasen, insbesondere CO2, verhindert. Vorzugsweise wird das zurückgeführte Gas durch eine Filtereinrichtung geführt, die insbesondere einen HEPA Filter aufweisen kann. Die Filtereinrichtung ist insbesondere dazu eingerichtet, Stoffe und Partikel, die von der Messkammer dem Gas beigefügt wurden, wieder auszufiltern.

Vorzugsweise ist mindestens ein Strömungskanal, insbesondere eine Gasleitung, welche die Inkubatorkammer und die Messkammer stromaufwärts oder stromabwärts der Messkammer verbindet, insbesondere auch mindestens ein Abschnitt mindestens einer Messkammeraußenseite, mit einer thermischen Isoliereinrichtung thermisch isoliert. Die thermische Isoliereinrichtung kann eine Doppelwand aufweisen, oder eine thermisch isolierende Materialschicht, die insbesondere isolierend wirkende Lufteinschlüsse aufweisen kann.

Vorzugsweise ist mindestens ein Strömungskanal, insbesondere eine Gasleitung, welche die Inkubatorkammer und die Messkammer stromaufwärts oder stromabwärts der Messkammer verbindet, in Kontakt mit einem Temperiermittel, insbesondere einer Heizeinrichtung, um den Strömungskanal zu erwärmen, insbesondere auf die Temperatur der Inkubatorkammer oder höher. Insbesondere kann der mindestens eine Strömungskanal an einer Wand der -ohnehin bereits- temperierten Inkubatorkammer entlang geführt sein. Mit solchen Maßnahmen soll verhindert werden, dass Kondensationseffekte auftreten oder dass VOCs temperaturbedingt verändert werden oder ausfallen.

Die Gasatmosphäre des Innenraums des Inkubators weist insbesondere eine andere Zusammensetzung auf als die Atmosphäre in den Zellkulturbehältern, die in dem Innenraum angeordnet sind. Die Atmosphäre in den Zellkulturbehältern ist insbesondere unmittelbar mit flüssigen Wachstumsmedien (Zellmedien) in Kontakt. Insbesondere haben das in die Messkammer eintretende Gas und die Gasatmosphäre des Innenraums dieselbe Zusammensetzung.

Der Messbereich des mindestens einen VOC-Sensors ist vorzugsweise in Strömungsverbindung mit dem Atmosphärengas des Innenraums stehend angeordnet, indem er in der Inkubatorkammer angeordnet ist, wobei in diesem Falle insbesondere keine gesonderte Messkammer notwendig wäre, aber vorgesehen sein kann. Der mindestens eine Messbereich kann insbesondere unmittelbar an den Innenraum der Inkubatorkammer angrenzen und mit der Gasatmosphäre des Innenraums unmittelbar in Kontakt stehen, wobei insbesondere ein Abwärme entwickelnder Bestandteil des mindestens einen VOC-Sensors außerhalb der Inkubatorkammer, und insbesondere in einer Umgebung der Inkubatorkammer angeordnet ist. Diese Umgebung ist insbesondere nicht in Kontakt mit dem Messbereich und ist insbesondere gekühlt, vorzugsweise luftgekühlt.

Es ist insbesondere bevorzugt, dass der mindestens eine VOC-Sensor teilweise und insbesondere nicht vollständig in der Inkubatorkammer und/oder in der Messkammer angeordnet ist. Es ist insbesondere bevorzugt, dass mindestens ein dem Messbereich eines Sensors gegenüberliegender Abschnitt des mindestens einen VOC-Sensor außerhalb der Inkubatorkammer und/oder der Messkammer angeordnet ist/sind. Es ist insbesondere bevorzugt, dass mindestens ein Heizmittel bzw. alle Heizmittel des mindestens einen VOC-Sensor außerhalb der Inkubatorkammer und/oder der Messkammer angeordnet ist/sind. Auf diese Weise ist der elektrische Anschluss des mindestens einen VOC-Sensors an eine elektrische Steuereinrichtung möglich und insbesondere vereinfacht. Zudem ist das Temperieren bzw. Kühlen der dem Messbereich abgewandten Seite, insbesondere der Heizmittel, des mindestens eine VOC-Sensors möglich und insbesondere vereinfacht.

Mittels der Gasleitung ist insbesondere Atmosphärengas unmittelbar aus der Inkubatorkammer in den Außenraum des Inkubators transportierbar, bzw. wird in dieser Weise unmittelbar transportiert. Die Sensoreinrichtung weist vorzugsweise eine oder vorzugsweise mehrere Messkammern auf, in der mindestens ein Messbereich mindestens eines VOC-Sensors angeordnet ist. Der am einen Ende in die Inkubatorkammer mündende Strömungskanal kann an einem anderen Ende in eine Messkammer münden oder kann aufgeteilt werden, um mit mehreren Enden in mehrere Messkammern zu münden. Insbesondere kann pro Messkammer eine Anzahl N mit 10>=N>=1 von VOC-Sensoren vorgesehen sein, in welche insbesondere die Gasleitung mündet. Es kann genau ein Messbereich eines VOC-Sensors oder es können genau zwei Messbereiche von VOC-Sensoren in einer Messkammer angeordnet sein. Atmosphärengas aus der Inkubatorkammer ist so insbesondere in mindestens eine- vorzugsweise jede- Messkammer transportierbar und wird - insbesondere gesteuert von einer elektrischen Steuereinrichtung- transportiert.

Vorzugsweise weist eine Messkammer eine Abgasleitung auf, die angeordnet ist, um die Abluft aus der Messkammer in einen Außenraum der Inkubatorkammer bzw. des Inkubators zu befördern.

Vorzugsweise weist die Sensoreinrichtung eine Mehrzahl von VOC-Sensoren auf, deren Messbereiche, insbesondere deren Adsorptionsflächen, in Kontakt mit einem Innenraum der Messkammer angeordnet sind.

Vorzugsweise weist die Sensoreinrichtung eine Mehrzahl von VOC-Sensoren auf, deren Messbereiche, insbesondere deren Adsorptionsflächen zur Adsorption von VOCs an den Messbereich, in Kontakt mit einem Innenraum der Messkammer angeordnet sind, wobei die VOC-Sensoren eine Heizseite aufweisen, die jeweils außerhalb der Messkammer angeordnet ist.

Vorzugsweise weist die Messkammer eine torusförmig ausgebildeten Innenraum auf, insbesondere in Form eines geschlossenen Torus, insbesondere mit mindestens einer Zufluss- und mindestens einer Abflussöffnung. Der Torus kann parallel einer Ebene liegen, die Zuflussöffnung kann im Wesentlichen diesseits der Ebene und insbesondere diesseits des Torus angeordnet sein, und die die Abflussöffnung kann im Wesentlichen jenseits der Ebene und insbesondere jenseits des Torus angeordnet sein. Zu- und Abflussöffnung können aber auch in der Ebene liegen oder diese kreuzen.

Insbesondere kann der Inkubator bzw. die Sensoreinrichtung ein Fördermittel zur Gasförderung aufweisen, mittels der ein im Innenraum der Messkammer befindliches, zuvor der Inkubatorkammer entnommenes, Atmosphärengas zirkuliert werden kann. Dabei kann die Zuflussöffnung und/oder die Abflussöffnung einen -insbesondere von einer elektrischen Steuereinrichtung steuerbaren- Verschluss aufweisen, mit dem die entsprechende Öffnung wahlweise verschließbar ist. Durch Zirkulieren des Gases wird ein Gasaustausch an den Messbereichen/Adsorptionsflächen erzielt, und eine längerfristige Abführung von zu messender Gasatmosphäre aus der Inkubatorkammer kann vermieden werden. Dadurch kann der Verbrauch von Gas, z.B. CO2, und Energie vermieden werden, die zum Wiederherstellen der Inkubatoratmosphäre in der Inkubatorkammer und/oder dem Druckausgleich andernfalls erforderlich sein könnten.

Vorzugsweise ist der Inkubator und/oder dessen elektronische Steuereinrichtung dazu eingerichtet, in Abhängigkeit von dem durch einen Strömungskanal aus der Inkubatorkammer in Richtung einer Messkammer abfließenden Volumenstrom einen entsprechenden zufließenden Volumenstrom mindestens eines Inkubatorgases, insbesondere einer festgelegten Mischung von Inkubatorgasen, z.B. N2 und CO2, zu steuern. Dadurch wird die Inkubatoratmosphäre -auch während einer Messung mittels der Sensoreinrichtung- möglichst unverändert gehalten. Auch die Temperatur der Inkubatoratmosphäre, die sich durch diese Gasab- und zuführung eventuell ändern bzw. sinken kann, kann durch Temperieren mittels der Temperaturregeleinrichtung des Inkubators, insbesondere dessen Temperiereinrichtung(en) und Temperatursensor(en), konstant gehalten werden bzw. nachgeregelt werden, insbesondere auf eine Zieltemperatur, z.B. 37 °C.

Vorzugsweise ist die Sensoreinrichtung als elektronische Nase ausgestaltet. Dazu weist sie insbesondere eine elektronische Steuereinrichtung sowie eine Mehrzahl von vorzugsweise typenunterschiedlichen VOC-Sensoren auf, sowie insbesondere eine Spüleinrichtung, mittels der mindestens eine Messkammer durch ein Spülgas gespült werden kann.

Vorzugsweise weist die elektronische Steuereinrichtung einer als elektronischen Nase ausgestalteten Sensoreinrichtung oder eines Inkubators eine Datenverarbeitungseinrichtung mit mindestens einem Datenspeicher auf, die dazu programmiert ist, mindestens einen, mehrere oder jeden der folgenden Schritte auszuführen. Analog kann das erfindungsgemäße Verfahren diese Schritte aufweisen:
i) Speichern eines Messdatensatzes im dem Datenspeicher, der die, insbesondere zeitabhängig, insbesondere innerhalb desselben mindestens einen Zeitabschnitts, erfassten, Messwerte der Anzahl N>1 von VOC-Sensoren beinhaltet, wobei ein Messwert für das detektierte Messignal des jeweiligen VOC-Sensors charakteristisch ist, das in Gegenwart eines aus der Inkubatorkammer stammenden und am Messbereich des VOC-Sensors anliegenden, insbesondere vorbeiströmenden, Volumens der Gasatmosphäre erfasst wurde;
ii) Ermitteln von ersten Ergebnismessdaten aus einem Vergleich des Messdatensatzes mit einem Referenzdatensatz, insbesondere unter Verwendung einer Differenz des Messdatensatzes und des Referenzdatensatzes, der die, insbesondere zeitabhängig erfassten, Referenzmesswerte der Anzahl N>1 von VOC-Sensoren beinhaltet, wobei ein Referenzmesswert für das detektierte Messignal des jeweiligen VOC-Sensors charakteristisch ist, das in Gegenwart eines aus einer Spüleinrichtung stammenden und am Messbereich des VOC-Sensors anliegenden, insbesondere vorbeiströmenden, Spülgases erfasst wurde;
iii) optional: Erkennen eines charakteristischen Datenmusters in dem die Ergebnismessdaten enthaltenden Ergebnismessdatensatz, wobei das charakteristische Datenmuster ein bestimmtes, im Atmosphärengas nachgewiesenes VOC, insbesondere auch dessen Konzentration oder Menge, repräsentiert.

Das charakteristische Datenmuster in dem die Ergebnismessdaten enthaltenden Ergebnismessdatensatz kann vorzugsweise durch folgende Auswertung berücksichtigt werden: eine Kontamination kann insbesondere dann vorliegen, dass die Messwerte einer bestimmten Untermenge von VOC-Sensoren der Sensoreinrichtung -optional unter Berücksichtigung eines Toleranzwertes- von ihrem jeweiligen Referenzwert abweichen, - optional kann ein relativer Grad der Abweichung berücksichtigt werden-, dass aber die Messwerte der anderen VOC-Sensoren der Sensoreinrichtung -optional unter Berücksichtigung eines Toleranzwertes- nicht von ihrem jeweiligen Referenzwert abweichen. Das charakteristische Datenmuster kann zuvor durch Messungen an einem oder mehreren Testgasen mit definiertem VOC-Gehalt ermittelt werden.

Der Schritt iii) kann vorzugsweise beinhalten, dass ein mittels maschinellem Lernen ermittelter Klassifikationsalgorithmus, insbesondere ein künstliches neuronales Netzwerk, zum Klassifizieren des charakteristischen Datenmusters verwendet wird.

Vorzugsweise weist die Steuereinrichtung eine Datenverarbeitungseinrichtung mit mindestens einem Datenspeicher auf, die dazu programmiert ist, mindestens den ersten, oder mehrere oder alle der folgenden Schritte auszuführen. Analog kann das erfindungsgemäße Verfahren diese Schritte aufweisen:
i) Speichern eines Testmessdatensatzes in einem Datenspeicher, der, insbesondere zeitabhängig, insbesondere innerhalb desselben mindestens einen Zeitabschnitts, erfasste, Testmesswerte der Anzahl N>1 von VOC-Sensoren beinhaltet, wobei ein Testmesswert für das detektierte Messignal des jeweiligen VOC-Sensors charakteristisch ist, das in Gegenwart eines der Messkammer zugeführten und am Messbereich des VOC-Sensors anliegenden, insbesondere vorbeiströmenden, Volumens eines vorbekannten Testgases mit, insbesondere nach Art und/oder Menge, vorbekanntem VOC-Gehalt erfasst wurde;
ii) Ermitteln von zweiten Ergebnismessdaten aus einem Vergleich des Testmessdatensatzes mit einem Referenzdatensatz, insbesondere unter Verwendung einer Differenz des Testmessdatensatzes und des Referenzdatensatzes, der die, insbesondere zeitabhängig, insbesondere innerhalb desselben mindestens einen Zeitabschnitts, erfassten, Referenzmesswerte der Anzahl N>1 von VOC-Sensoren beinhaltet, wobei ein Referenzmesswert für das detektierte Messignal des jeweiligen VOC-Sensors charakteristisch ist, das in Gegenwart eines aus einer Spüleinrichtung stammenden und am Messbereich des VOC-Sensors anliegenden, insbesondere vorbeiströmenden, Spülgases erfasst wurde;
iii) Speichern eines die zweiten Ergebnismessdaten enthaltenden zweiten Ergebnismessdatensatzes, der ein nun bekanntes, für das Testgas charakteristisches Datenmuster aufweist.

Mittels den vorstehend genannten Schritten kann insbesondere ein Verfahren realisiert werden, mittels dem die Präsenz bestimmter VOCs in der Gasatmosphäre erkannt werden kann, insbesondere die Konzentration mindestens eines VOC's in der Gasatmosphäre abgeschätzt werden kann.

Vorzugsweise wird ein Schritt iv) ausgeführt, in dem die in iii) erhaltenen zweiten Ergebnismessdaten als gelabelte Daten verwendet werden, um einen lernfähigen Klassifikationsalgorithmus durch maschinelles Lernen zu trainieren, insbesondere ein neuronales Netzwerk, der/das nachfolgend zum Klassifizieren gemessener charakteristischer Datenmusters verwendbar ist. Mittels diesem Schritt kann auch insbesondere ein Verfahren realisiert werden, mittels dem die Präsenz bestimmter VOCs in der Gasatmosphäre erkannt werden kann, insbesondere die Konzentration mindestens eines VOC's in der Gasatmosphäre abgeschätzt werden kann.

Vorzugsweise weist der Inkubator ein Informationsausgabesystem auf, insbesondere ein Display, einen Lautsprecher oder eine Datenschnittstelle zu einem externen datenverarbeitenden Gerät, um in Abhängigkeit von der mittels der Sensoreinrichtung erfassten Detektion von VOCs eine Information über diese Detektion auszugeben, insbesondere um eine Warninformation an einen Benutzer oder ein überwachendes System auszugeben.

Die Erfindung betrifft auch ein Laborüberwachungssystem zur Erfassung der Kontamination mindestens einer Inkubatorkammer, aufweisend
* mindestens einen erfindungsgemäßen Inkubator;
* mindestens ein extern zu dem mindestens einen Inkubator angeordnetes, datenverarbeitendes Gerät, das insbesondere in einer Datenaustauschverbindung mit dem mindestens einen Inkubator steht, insbesondere über ein Intranet oder das Internet;
wobei das datenverarbeitende Gerät programmiert ist, die von dem mindestens einen Inkubator erhaltenen, mittels der Sensoreinrichtung des Inkubators durch die Detektion ermittelten Messdaten über eine mögliche Kontamination der Inkubatorkammer zu erfassen und in einer Datenspeichereinrichtung zu speichern, insbesondere um diese Messdaten an ein weiteres Gerät zu kommunizieren, insbesondere an einen PC, ein Mobilfunkgerät, ein Smartphone oder einen Tabletcomputer.

Die Erfindung betrifft auch ein Verfahren zur Erfassung der Kontamination in der Inkubatorkammer eines Inkubators, insbesondere eines erfindungsgemäßen Inkubators, aufweisend die Schritte:
* Erfassung von Messdaten mittels einer Sensoreinrichtung, die mindestens einen VOC-Sensor zur Detektion von flüchtigen organischen Verbindungen (VOCs) aufweist, wobei der VOC-Sensor einen Messbereich aufweist, der in Strömungsverbindung mit dem Atmosphärengas des Innenraums stehend angeordnet ist;
* Ermitteln einer möglichen Kontamination der Gasatmosphäre des Innenraums durch Auswertung der Messdaten.

Die Erfindung betrifft auch ein Nachrüstsystem mit einer nachrüstbaren Sensoreinrichtung zur Detektion einer möglichen Kontamination der Gasatmosphäre des Innenraums einer Inkubatorkammer, wobei die Sensoreinrichtung mindestens einen VOC-Sensor (11) zur Detektion von flüchtigen organischen Verbindungen (VOCs) aufweist, wobei der VOC-Sensor einen Messbereich aufweist, der in Strömungsverbindung mit dem Atmosphärengas des Innenraums stehend anordenbar ist, und wobei die Sensoreinrichtung vorzugsweise eine Gasleitung aufweist, die zwischen dem Innenraum und dem Messbereich anordenbar ist, und vorzugsweise eine Pumpe, um ein Volumen der Gasatmosphäre des Innenraums der Inkubatorkammer durch die Gasleitung zum Messbereich zu befördern. Die Erfindung betrifft auch eine solche nachrüstbare Sensoreinrichtung für einen Inkubator. Das Nachrüstsystem bzw. die nachrüstbare Sensoreinrichtung umfasst ferner insbesondere eine elektronische Auswertungseinrichtung, die vorzugsweise eine Datenverarbeitungseinrichtung beinhaltet. Die Auswertungseinrichtung ist vorzugsweise dazu eingerichtet, mindestens ein Messsignal, insbesondere Messwert, des mindestens einen VOC-Sensors zu erfassen und insbesondere auszuwerten. Alternativ und/oder zusätzlich umfasst das Nachrüstsystem einen Programmcode, bei dessen Ausführung eine Datenverarbeitungseinrichtung, insbesondere des Inkubators, den insbesondere einen Messwert des mindestens einen VOC-Sensors erfasst und insbesondere auswertet. Die möglichen Schritte eines Programmcodes wurden bzw. werden hier bereits/noch beschrieben.

Die Erfindung betrifft auch eine Inkubatoranordnung, aufweisend einen Inkubator mit einer Inkubatorkammer und einer nachrüstbaren Sensoreinrichtung wie oben beschrieben, die zur Detektion einer möglichen Kontamination der Gasatmosphäre des Innenraums einer Inkubatorkammer an dem Inkubator oder in der Inkubatorkammer des Inkubators angeordnet ist.

Der Inkubator ist ein Laborgerät bzw. ein Laborinkubator. Ein Inkubator bezeichnet insbesondere ein Laborgerät mit einer Inkubatorkammer, deren Atmosphäre vom Inkubator auf eine vorgegebene Zieltemperatur regelbar ist bzw. geregelt wird. Insbesondere handelt es sich um ein Laborgerät, mit dem kontrollierte Klimabedingungen für verschiedene biologische Entwicklungs- und Wachstumsprozesse geschaffen und erhalten werden können. Der Inkubator kann vorzugsweise ein Shaker, also ein Inkubator mit einer Bewegungseinrichtung zur Bewegung von in der Inkubatorkammer angeordneten Objekten, sein oder diesen beinhalten. Der Inkubator kann eine Zellkultivierungsvorrichtung sein, ein Microbial incubator (auch ohne CO₂). Der Inkubator dient insbesondere der Schaffung und Erhaltung eines Mikroklimas mit geregelten Gas-, und/oder Luftfeuchtigkeits- und/oder Temperatur-Bedingungen in der Inkubatorkammer, wobei diese Behandlung zeitabhängig sein kann. Der Labor-Inkubator, insbesondere eine Behandlungseinrichtung des Labor-Inkubators, kann insbesondere einen Zeitgeber aufweisen, insbesondere eine Zeitschaltuhr, eine Heiz-/Kühleinrichtung und vorzugsweise eine Einstellung für die Regelung eines der Inkubatorkammer zugeführten Austauschgases, eine Einstelleinrichtung für die Zusammensetzung des Gases in der Inkubatorkammer des Inkubators, insbesondere zur Einstellung des CO₂ und/oder des O₂ und/oder des N2-Gehalts Gehalts des Gases und/oder eine Einstelleinrichtung zur Einstellung der Luftfeuchtigkeit in der Inkubatorkammer des Inkubators. Der Inkubator, insbesondere eine Behandlungseinrichtung des Inkubators, weist insbesondere die Inkubatorkammer auf, ferner vorzugsweise eine Regeleinrichtung mit mindestens einem Regelkreis, dem als Stellglied die mindestens eine Heiz-/Kühleinrichtung und als Messglied mindestens eine Temperaturmesseinrichtung zugeordnet sind. Mittels der Regeleinrichtung kann die Temperatur im Inkubator geregelt werden. Je nach Ausführungsform kann darüber auch die Luftfeuchte geregelt werden. Eine mit Wasser gefüllte Wanne in der Inkubatorkammer kann geheizt oder gekühlt werden, um über die Verdunstung die Luftfeuchtigkeit einzustellen. Alternativ und/oder zusätzlich kann ein Wasserverdampfer als Bestandteil des Inkubators vorgesehen sein, mittels dem die Luftfeuchtigkeit in der Atmosphäre der Inkubatorkammer eingestellt wird. CO₂-Inkubatoren dienen insbesondere der Kultivierung tierischer bzw. humaner Zellen. Inkubatoren können Wendevorrichtungen zum Wenden des mindestens einen Zellkulturbehälters und/oder eine Schütteleinrichtung zum Schütteln bzw. Bewegen der des mindestens einen Zellkulturbehälters aufweisen.

Eine Sensoranordnung des Inkubators, die insbesondere einer Regeleinrichtung zugeordnet sein kann, weist insbesondere mindestens einen Temperatursensor auf, vorzugsweise eine Vielzahl von Temperatursensoren. Ein Temperatursensor kann beispielsweise ein Pt 100- oder Pt 1000-Temperaturfühler sein. Eine Sensoreinrichtung weist vorzugsweise einen Sensor zur Ermittlung einer relativen Gaskonzentration auf, insbesondere zur Ermittlung des Gehalts an CO₂ und/oder O₂ und/oder N₂. Eine Sensoreinrichtung weist vorzugsweise einen Sensor zur Ermittlung der relativen Luftfeuchtigkeit auf.

Ein Inkubator weist vorzugsweise eine bzw. eine einzige Inkubatorkammerauf. Diese kann in Kompartimente unterteilt sein. Kompartimente können durch -insbesondere gelochte-Lagerplatten getrennt sein, wobei insbesondere ein Gasaustausch zwischen den Kompartimenten ermöglicht ist. Eine Lagerplatte, insbesondere deren untere Seite, kann zum Halten der Sensoreinrichtung oder eines Strömungskanals eingerichtet sein mittels dem Inkubatoratmosphäre in eine Messkammer geführt wird und kann insbesondere eine Halterung für diese Teile aufweisen.

Die Inkubatorkammer weist Kammerwände bzw. Kammerinnenwände und genau eine oder mindestens eine Kammeröffnung auf, über die die Objekte bzw. Zellkulturbehälter im Inneren der Inkubatorkammer platzierbar und entnehmbar sind. Diese Kammeröffnung ist durch ein mit der Inkubatorkammer beweglich verbundenes Verschlusselement verschließbar, insbesondere eine mittels Scharnier an der Inkubatorkammer beweglich montierte Inkubatortüre, insbesondere eine oder mehrere Kammertüren. Ein Inkubator kann eine oder mehrere Innentüren aufweisen, die insbesondere transparent sein können, und kann eine -insbesondere nicht transparente- Außentür aufweisen, welche insbesondere die Inkubatorkammer und gegebenenfalls mindestens einer innere Inkubatortüre, welche die Kammeröffnung verschließt bzw. öffnet, zur Umgebung hin thermisch isoliert.

In der verschlossenen Position der Kammeröffnung ist das Innere (synonym: der Innenraum) der Inkubatorkammer gegen die Umgebung vorzugsweise in der Weise isoliert, so dass im Inneren eine gewünschte, vom Inkubator gesteuerte Atmosphäre einstellbar, insbesondere regelbar ist. Der Begriff "Gasatmosphäre des Innenraums der Inkubatorkammer" bezeichnet nicht den Innenraum von in der Inkubatorkammer angeordneten im Wesentlichen verschlossenen hohlen Objekten, und bezeichnet insbesondere nicht den Behälterinnenraum eines in der Inkubatorkammer angeordneten Behälters, dessen Öffnung verschlossen ist, insbesondere abgedeckt ist, oder nicht gasdicht oder gasdicht verschlossen ist. Dieser Behälterinnenraum, z.B. eines Zellkulturbehälters, weist typischerweise ein flüssiges Medium auf und einen oberhalb dieser Flüssigkeit gelegenen Luftüberstand. In der geöffneten Position der Kammeröffnung ist über diese Öffnung ein Gasaustausch zwischen der Umgebung des Inkubators und dem Inneren der Inkubatorkammer möglich. Die Kammeröffnung befindet sich typischer Weise in einer die Kammeröffnung umlaufenden Frontwand.

Die Inkubatorkammer weist vorzugsweise mehrere Wände bzw. Innenwandflächen auf, die insbesondere einstückig und insbesondere kantenfrei miteinander verbunden sein können. Die Wände bzw. Innenwandflächen sind vorzugsweise im Wesentlichen planar geformt, können aber auch alle oder zum Teil eine geschwungene Form aufweisen. Die Inkubatorkammer ist vorzugsweise quaderartig geformt, kann aber auch anders geformt sein, z.B. sphärisch, ellipsoid, polyederförmig. Die Wände bzw. Innenwandflächen sind vorzugsweise aus einem korrosionsarmen Material gefertigt, insbesondere Edelstahl, Kupfer, Messing, oder ein Kunststoff, insbesondere ein Verbundkunststoff. Dadurch wird die Reinigung/ Sterilisation des Kammerinneren mittels des Reinigungsmittels erleichtert. Unabhängig von der Kammeröffnung, die dem Bestücken / Entnehmen von Objekten bzw. Zellkulturbehältern dient, kann die Inkubatorkammer mindestens einen Port zum Durchführen einer entsprechend dimensionierten Vorrichtung bzw. einer Gasleitung und/oder einer Kabelverbindung aus dem Inneren der Inkubatorkammer an deren Außenseite oder in die Umgebung des Inkubators aufweisen. Ein Port beinhaltet insbesondere eine Öffnung in einer Kammerwand der Inkubatorkammer, zum Durchführen einer entsprechend dimensionierten Vorrichtung bzw. einer Gasleitung und/oder einer Kabelverbindung aus dem Inneren der Inkubatorkammer an deren Außenseite oder in die Umgebung des Inkubators

Eine typische Größe des Inneren einer Inkubatorkammer liegt zwischen 50 und 400 Litern.

Der Inkubator kann genau eine Inkubatorkammer aufweisen, kann aber auch mehrere Inkubatorkammern aufweisen, deren Atmosphäre (Temperatur, relative Gaskonzentration, Luftfeuchte) insbesondere individuell oder gesammelt einstellbar sein kann. Ein Inkubator kann mehrere Inkubatorkammern aufweisen, die jeweils eine eigene Kammeröffnung und eine eigene Kammertüre zum Verschließen der Kammeröffnung aufweisen können, insbesondere einen eigenen Port. Es können mehrere Sensoreinrichtungen vorgesehen sein, insbesondere ein pro Kammer.

Der Inkubator kann ein Gehäuse aufweisen, dass die Inkubatorkammer teilweise oder vollständig umgibt. Das Gehäuse kann im Wesentlichen quaderförmig ausgebildet sein, und kann insbesondere derart ausgebildet sein, dass der Inkubator stapelbar ist. Die Sensoreinrichtung und/oder der mindestens eine VOC-Sensor ist vorzugsweis in dem Gehäuse, oder einem Gehäuseabschnitt angeordnet, insbesondere unbeweglich mit diesem verbunden. Auf diese Weise bildet die Sensoreinrichtung einen integralen Bestandteil des Inkubators. Sie kann aber auch als Modul, insbesondere als Teil eines Nachrüstsystems im Gehäuse anordenbar sein, insbesondere unbeweglich mit diesem verbindbar sein.

Ein Lagerbereich des Inkubators ist insbesondere durch eine Lagerplatte, insbesondere einen Regalblecheinsatz realisiert, die/der insbesondere aus Edelstahl oder Kuper oder ähnlichem bestehen kann oder dieses Material aufweist. Eine Lagerplatte dient als Bodenplatte, insbesondere als Zwischenbodenplatte. Die Lagerplatte kann der Inkubatorkammer entnehmbar sein ("Lagerplatteneinsatz") oder kann fest eingesetzt mit dieser verbunden sein. Die Inkubatorkammer kann Halteabschnitte oder einen Haltrahmen zum Halten eines oder mehrerer Lagerplatteneinsätze oder einsetzbarer Instrumente aufweisen. Eine Lagerplatte kann an ihrer Unterseite zum Halten einer Sensoreinrichtung oder mindestens einer Gasleitung eingerichtet sein, insbesondere eine Halterung für diese Sensoreinrichtung oder Gasleitung aufweisen. Alternativ oder zusätzlich kann mindestens eine der Innenwände der Inkubatorkammer zum Halten eines oder mehrerer Lagerplatteneinsätze oder einsetzbarer Instrumente eingerichtet sein. Dazu kann eine in die Wand integrierte Haltestruktur vorgesehen sein, insbesondere ein oder mehrere Vorsprünge, Rinnen oder Stege. Eine Lagerplatte vergrößert die verfügbare Lagerfläche in der Inkubatorkammer.

Ein Halterahmen für die mindestens eine Lagerplatte ist ebenfalls vorzugsweise aus einem nicht-korrosiven Material gefertigt, vorzugsweise Edelstahl. Der Halterahmen ist vorzugsweise als stehendes Objekt ausgelegt, indem er mindestens einen Sockelabschnitt aufweist, der auf der Bodenwand der Inkubatorkammer ruht. Er kann sich aber auch an den Seitenwänden der Inkubatorkammer abstützen und/oder an der Deckenwand der Inkubatorkammer aufgehängt sein.

Eine Lagerplatte erstreckt sich vorzugsweise - und insbesondere im Wesentlichen vollständig- über einen horizontalen Querschnitt der Inkubatorkammer.

Vorzugsweise weist der Inkubator eine Behandlungseinrichtung zur Behandlung des mindestens einen Zellkulturbehälters auf. Der Begriff "Behandlung" bedeutet insbesondere, dass ein Objekt, insbesondere eine Zellkultur oder ein Zellkulturbehältnis bewegt, und/oder transportiert und/oder untersucht und/oder verändert wird, insbesondere physikalisch, chemisch, biochemisch oder auf andere Weise verändert wird.

Eine Behandlungseinrichtung kann eine Bewegungseinrichtung sein, mittels der das Zellmedium in mindestens einem Zellkulturbehälter in Bewegung gehalten wird, vorzugsweise über ein Bewegungsprogramm, das vom Steuerprogramm gesteuert wird. Eine Bewegungseinrichtung kann eine Schüttel- oder Schwenkeinrichtung sein. Eine Bewegungseinrichtung weist vorzugsweise eine Trägereinrichtung auf, insbesondere eine Platte, auf der ein oder mehrere Zellkulturbehälter abgestellt und/oder fixiert werden. Eine Bewegungseinrichtung weist vorzugsweise eine Antriebseinrichtung auf, insbesondere im Fall einer Schütteleinrichtung beispielsweise einen Oszillatorantrieb, mittels dem das gewünschte Bewegungsprogramm umgesetzt wird. Die Gestaltung des Bewegungsprogramms kann vom Wachstumsstadium der Zellen einer Zellkultur abhängen und kann von der Zellart, insbesondere einer Zelllinie abhängen. Die Gestaltung und/oder Steuerung der Behandlung, insbesondere des Bewegungsprogramms, kann von den Zellüberwachungsdaten abhängen. Die Eine Behandlungseinrichtung kann eine Schwenkeinrichtung sein, mittels der mindestens ein Zellkulturbehälter geschwenkt wird. Die Bestandteile der Schwenkeinrichtung können denen der Schütteleinrichtung entsprechen, sind aber für eine Schwenkbewegung eingerichtet.

Eine Behandlungseinrichtung kann auch eine Transporteinrichtung sein, mittels der mindestens ein Zellkulturbehälter in der Inkubatorkammer transportierbar ist. Die Transporteinrichtung kann eine Lifteinrichtung sein, aufweisend eine Trägereinrichtung, auf der der mindestens eine Zellkulturbehälter platzierbar ist. Die Lifteinrichtung weist vorzugsweise einen Bewegungsmechanismus und/oder einen elektrisch ansteuerbaren Antriebsmechanismus zum Antreiben des Bewegungsmechanismus auf. Die Transporteinrichtung kann ferner einen beweglichen und elektrisch ansteuerbaren Greifarm zum Greifen und Halten mindestens eines Zellkulturbehälters sein. Die Transporteinrichtung kann ein Förderband zum Bewegen des mindestens einen darauf platzierten Zellkulturbehälters aufweisen. Durch den Transport kann der mindestens eine Zellkulturbehälter in der Inkubatorkammer bewegt werden, insbesondere zu einer Bearbeitungsposition in einer Bearbeitungsstation in der Inkubatorkammer, und von dieser Bearbeitungsposition weg. Die Steuereinrichtung kann dazu eingerichtet sein, die Transporteinrichtung in Abhängigkeit von Ergebnisdaten zu steuern, die von einer Auswertungseinrichtung aus den Messwerten der Sensoreinrichtung gewonnen wurden.

Die Sensoreinrichtung, insbesondere eine Gasleitung, kann zudem an einer in der Inkubatorkammer befindlichen Transporteinrichtung befestigbar sein bzw. befestigt sein. Die Sensoreinrichtung kann an einem Positioniermechanismus befestigbar sein oder befestigt sein, mittels dem die Sensoreinrichtung in der Inkubatorkammer bewegbar und positionierbar ist. Der Positioniermechanismus kann einen beweglichen Roboterarm beinhalten und ist vorzugsweise elektrisch steuerbar, insbesondere durch ein Steuerprogramm der Steuereinrichtung. Auf diese Weise kann eine VOC-Konzentration an unterschiedlichen Orten in der Inkubatorkammer nacheinander mit einer oder mit wenigen Sensoreinrichtungen nacheinander gemessen werden. Der Positioniermechanismus kann als in die Inkubatorkammer einsetzbares Bauteil ausgebildet sein. Die Energiezufuhr dieses Bauteils kann über eine Kabelverbindung mit dem Inkubator erfolgen, vorzugsweise über ein durch eine Wandöffnung, z.B. einen Port, oder über eine solche Kabelverbindung zu einer externen Spannungsquelle. Die Steuereinrichtung kann dazu eingerichtet sein, den Positioniermechanismus in Abhängigkeit von Ergebnisdaten zu steuern, die von einer Auswertungseinrichtung aus den Messwerten der Sensoreinrichtung gewonnen wurden.

Als Behandlungseinrichtung lässt sich auch die Temperiereinrichtung der Inkubatorkammer verstehen, mit der die Atmosphäre im Inneren der Inkubatorkammer auf den gewünschten Wert, insbesondere 37°C geregelt wird. Der Begriff Temperieren bezieht sich auf das Erhöhen und Senken der Atmosphärentemperatur durch Heizen und Kühlen. Vorzugsweise wird die Temperatur im Inneren über eine Temperaturänderung der Wände des Inkubators eingestellt. Temperatursensoren der entsprechenden Temperaturregeleinrichtung sind an wenigstens einer Position im Inneren und / oder außerhalb der Inkubatorkammer, insbesondere an einer Wand der Inkubatorkammer verteilt.

Der Inkubator weist vorzugsweise eine Benutzerschnittstelleneinrichtung auf, über die der Benutzer Daten an die Datenverarbeitungseinrichtung bzw. die Steuereinrichtung eingeben kann, und/oder über die Informationen an den Benutzer ausgegeben werden können. Vorzugsweise ist der Inkubator bzw. diese Benutzerschnittstelleneinrichtung dazu eingerichtet, dass der Benutzer eine von den Messwerten der Sensoreinrichtung, insbesondere den Ergebnisdaten einer Auswertungseinrichtung, abhängige Information erhalten kann. Vorzugsweise ist der Inkubator bzw. diese Benutzerschnittstelleneinrichtung dazu eingerichtet, dass der Benutzer mindestens einen Betriebsparameter zum Betreiben des Inkubators oder der Sensoreinrichtung an dieser Benutzerschnittstelleneinrichtung eingeben kann und/oder entsprechende Informationen von dieser erhalten kann. Auf diese Weise kann eine einzige Benutzerschnittstelleneinrichtung vom Benutzer dazu verwendet werden, um den Inkubator und auch die mindestens eine Sensoreinrichtung zu beeinflussen, oder zu steuern, oder von dieser Informationen zu erhalten. Insbesondere kann die Sensoreinrichtung dazu eingerichtet sein, dem Benutzer auf eine mittels der Benutzerschnittstelleneinrichtung des Inkubators erfolgte Abfrage des Benutzers Messwerte oder Ergebnisdaten anzuzeigen, oder eine aus Messwerten abgeleitete statistische Information, und/oder einen Zeitverlauf von Messwerten oder Ergebnisdaten.

Eine gerätegesteuerte Behandlung des Inkubators ist vorzugsweise eine programmgesteuerte Behandlung, also eine durch ein Programm gesteuerte Behandlung. Unter einer programmgesteuerten Behandlung einer Probe ist zu verstehen, dass der Vorgang der Behandlung im Wesentlichen durch Abarbeiten einer Mehrzahl oder Vielzahl von Programmschritten erfolgt. Vorzugsweise erfolgt die programmgesteuerte Behandlung unter Verwendung mindestens eines Programmparameters, insbesondere mindestens eines vom Benutzer gewählten Programmparameters. Ein von einem Benutzer gewählter Parameter wird auch als Nutzerparameter bezeichnet. Die programmgesteuerte Behandlung erfolgt vorzugsweise mittels der digitalen Datenverarbeitungseinrichtung, die insbesondere Bestandteil der Steuereinrichtung ist. Die Datenverarbeitungseinrichtung kann mindestens einen Prozessor, d.h. eine CPU aufweisen, und/oder mindestens einen Mikroprozessor aufweisen. Die programmgesteuerte Behandlung wird vorzugsweise entsprechend den Vorgaben eines Programms gesteuert und/oder durchgeführt, insbesondere eines Steuerprogramms. Insbesondere ist bei einer programmgesteuerten Behandlung zumindest nach Erfassung der benutzerseitig erforderlichen Programmparameter im Wesentlichen keine Benutzertätigkeit erforderlich. Eine gerätegesteuerte Behandlung des Inkubators kann insbesondere in Abhängigkeit von Messwerten oder Ergebnisdaten der mindestens einen Sensoreinrichtung erfolgen.

Unter einem Programmparameter wird eine Variable verstanden, die innerhalb eines Programms oder Unterprogramms, für mindestens eine Durchführung (Aufruf) des Programms oder Unterprogramms gültig, in vorbestimmter Weise eingestellt werden kann. Der Programmparameter wird, z.B. vom Benutzer, festgelegt und steuert das Programm oder Unterprogramm und bewirkt eine Datenausgabe in Abhängigkeit von diesem Programmparameter. Insbesondere beeinflusst und/oder steuert der Programmparameter und/oder steuern die vom Programm ausgegebenen Daten die Steuerung des Geräts, insbesondere die Steuerung der Behandlung mittels der mindestens einen Behandlungseinrichtung.

Unter einem Programm wird insbesondere ein Computerprogramm verstanden. Ein Programm ist eine Folge von Anweisungen, insbesondere bestehend aus Deklarationen und Instruktionen, um auf einer digitalen Datenverarbeitungseinrichtung eine bestimmte Funktionalität, Aufgaben- oder Problemstellung bearbeiten und/oder lösen zu können. Ein Programm liegt in der Regel als Software vor, die mit einer Datenverarbeitungseinrichtung verwendet wird. Das Programm kann insbesondere als Firmware vorliegen, im Fall der vorliegenden Erfindung insbesondere als Firmware der Steuereinrichtung des Inkubators oder des Systems. Das Programm liegt meist auf einem Datenträger als ausführbare Programmdatei, häufig im sogenannten Maschinencode vor, die zur Ausführung in den Arbeitsspeicher des Rechners der Datenverarbeitungseinrichtung geladen wird. Das Programm wird als Abfolge von Maschinen-, d. h. Prozessorbefehlen von dem/den Prozessoren des Computers verarbeitet und damit ausgeführt. Unter 'Computerprogramm' wird insbesondere auch der Quelltext des Programms verstanden, aus dem im Verlauf der Steuerung des Laborgerätes der ausführbare Code entstehen kann.

Eine Benutzerschnittstelleneinrichtung kann Bestandteil eines Inkubators sein, oder ein Modul. Eine Benutzerschnittstelleneinrichtung weist jeweils vorzugsweise auf: eine Steuereinrichtung für die Benutzerschnittstelleneinrichtung; eine Kommunikationseinrichtung zur Herstellung einer Datenverbindung mit einem Laborgerät, insbesondere einem Inkubator, über eine Schnittstelleneinrichtung desselben; eine Eingabeeinrichtung zur Erfassung von Benutzereingaben eines Benutzers; eine Ausgabeeinrichtung, insbesondere eine Anzeige und/oder ein Display, zur Ausgabe von Informationen an den Benutzer, insbesondere ein berührungsempfindliches Display. Dabei ist die Steuereinrichtung der Benutzerschnittstelleneinrichtung vorzugsweise dazu eingerichtet, über die Datenverbindung Daten mit der Steuereinrichtung des Inkubators auszutauschen.

Ein in der Inkubatorkammer lagerbares Objekt ist insbesondere ein Zellkulturbehälter. Ein Zellkulturbehälter ist insbesondere transparent. Er ist insbesondere aus Kunststoff gefertigt, insbesondere PE oder PS gefertigt und weist insbesondere eine planare Bodenplatte auf, welche die Wachstumsfläche der Zellen bildet. Diese kann eine Oberflächenbehandlung zur Förderung der Adhärenz von Zellen aufweisen. Der Zellkulturbehälter kann mit einer PE-Kappe oder Gasaustauschkappe, insbesondere einem Deckel mit optional enthaltenem Filter, verschließbar oder verschlossen sein bzw. versehen sein. Der Zellkulturbehälter ist insbesondere stapelbar. Geeignet ist insbesondere eine Eppendorf Zellkulturflasche.

Der Inkubator weist vorzugsweise eine elektrische Steuereinrichtung (synonym: Steuerungseinrichtung) auf, die insbesondere eine Regeleinrichtung beinhalten kann. Eine Datenverarbeitungseinrichtung ist vorzugsweise Bestandteil einer Steuereinrichtung des Inkubators, die Funktionen des Inkubators steuert. Die Funktionen der Steuereinrichtung sind insbesondere durch elektronische Schaltkreise implementiert. Die Steuereinrichtung kann einen Mikroprozessor aufweisen, der die Datenverarbeitungseinrichtung beinhalten kann. Die Steuereinrichtung und/oder die Datenverarbeitungseinrichtung ist vorzugsweise zur Durchführung eines Steuerungsverfahrens ausgebildet, das auch als Steuerungssoftware oder Steuerungsprogramm bezeichnet wird. Die Funktionen des Inkubators und/oder der Steuereinrichtung und/oder der Auswertungseinrichtung können in Verfahrensschritten beschrieben werden. Sie können als Bestandteile eines Computerprogramm(code)s des Steuerungsprogramms realisiert sein, insbesondere als Unterprogramme des Steuerungsprogramms.

Eine Steuereinrichtung weist im Rahmen der vorliegenden Erfindung generell insbesondere die Datenverarbeitungseinrichtung, insbesondere eine Recheneinheit (CPU) zum Verarbeiten von Daten und/oder einen Mikroprozessor auf oder ist die Datenverarbeitungseinrichtung. Die Datenverarbeitungseinrichtung der Steuereinrichtung des Inkubators ist vorzugsweise auch zum Steuern eines Behandlungsprozesses und/oder von individuellen Behandlungen eingerichtet, die von einer oder mehreren insbesondere optionalen Behandlungseinrichtungen des Inkubators durchgeführt werden.

Die Datenverarbeitungseinrichtung ist alternativ vorzugsweise eine außerhalb des Inkubators und getrennt von diesem angeordnete Vorrichtung, auch als externe Vorrichtung bzw. externe Datenverarbeitungseinrichtung bezeichnet. Die Datenverarbeitungseinrichtung und der Inkubator stehen vorzugsweise in einer Datenverbindung und sind vorzugsweise Bestandteile eines Netzwerks zum Datenaustausch. Die Datenverarbeitungseinrichtung und der Inkubator sind in diesem Fall insbesondere Bestandteile eines erfindungsgemäßen Laborüberwachungssystems zur Erfassung der Anreicherung von VOCs, insbesondere zur Erfassung der Kontamination.

Der Inkubator weist insbesondere eine Regeleinrichtung mit mindestens einem Regelkreis, dem als Stellglied die mindestens eine Temperiereinrichtung und als Messglied mindestens ein Temperatursensor zugeordnet sind. Je nach Ausführungsform kann darüber auch die Luftfeuchte geregelt werden, obwohl die Luftfeuchtigkeit selber nicht durch einen Luftfeuchte-Sensor (rH-Sensor) gemessen wird und die Luftfeuchtigkeit nicht Eingangsgröße des Regelkreises ist. Eine mit Wasser gefüllte Wanne in der Inkubatorkammer kann geheizt oder gekühlt werden, um über die Verdunstung die Luftfeuchtigkeit einzustellen. CO2-Inkubatoren dienen insbesondere der Kultivierung tierischer bzw. humaner Zellen. Inkubatoren können Wendevorrichtungen zum Wenden des mindestens einen Zellkulturbehälters und/oder eine Schütteleinrichtung zum Schütteln bzw. Bewegen der des mindestens einen Zellkulturbehälters aufweisen.

Es ist bei der Anordnung einer Sensoreinrichtung an oder in der Inkubatorkammer zu beachten zu beachten, dass der Betrieb von elektrischen Einrichtungen innerhalb der Inkubatorkammer zu Abwärme führt, welche die Kammeratmosphäre in inakzeptablem Maße erwärmen kann. Die Steuereinrichtung des Inkubators kann deshalb insbesondere dazu eingerichtet sein, den Betrieb der elektrischen Einrichtungen, insbesondere der mindestens einen Sensoreinrichtung innerhalb oder an der Inkubatorkammer in Abhängigkeit von mittels Temperatursensoren erfassten Temperaturen der Kammeratmosphäre zu steuern. Die Steuereinrichtung des Inkubators kann insbesondere dazu eingerichtet sein, die Temperierung der Kammeratmosphäre mittels der mindestens einen Temperiereinrichtung und den Betrieb der elektrischen Einrichtungen innerhalb der Inkubatorkammer in Abhängigkeit voneinander zu steuern, um die unerwünschte Erwärmung der Kammeratmosphäre zu kompensieren.

Die Steuerungseinrichtung kann dazu eingerichtet sein, dass ein Programmparameter oder ein Steuerungsparameter des Inkubators automatisch in Abhängigkeit von anderen Daten gewählt wird. Eine von einem Steuerungsparameter gesteuerte Behandlung der mindestens einen Zellkultur in mindestens einem Zellkulturbehälter entspricht bei einem Inkubator insbesondere einer Klimabehandlung, der die mindestens eine Zellkultur unterzogen wird. Mögliche Parameter, insbesondere Programmparameter, insbesondere Nutzerparameter, die zur Beeinflussung einer Klimabehandlung verwendet werden, definieren insbesondere die Temperatur des Inkubatorraums, in dem die mindestens eine Probe inkubiert wird, die relative Gaskonzentration von O2- und/oder CO2 und/oder N2 im Inkubationsinnenraum, die Luftfeuchtigkeit im Inkubationsinnenraum und/oder mindestens einen Ablaufparameter, der den Ablauf, insbesondere die Reihenfolge, eines aus mehreren Schritten bestehenden Inkubationsbehandlungsprogramms beeinflusst oder definiert.

Die Temperiereinrichtung kann eine kombinierte Heiz- / Kühleinrichtung sein. Sie ist vorzugsweise nur eine Heizeinrichtung. Diese kann insbesondere die Wärme über einen elektrischen Widerstandsdraht erzeugen.

Zu den vom Inkubator gesteuerten Vorgängen gehören zudem alle Temperierschritte, die den physikalischen Zustand "Temperatur der Inkubatoratmosphäre" beeinflussen, insbesondere auch Schritte einer optionalen automatischen Sterilisation mittels Hochtemperaturphasen bei ca. 100 °C bis 120 °C oder bis 180°C oder 200°C, die bei leerer Inkubatorkammer durchgeführt werden.

Zu den vom Inkubator gesteuerten Vorgängen können ferner Gasaustauschvorgänge gehören, bei denen gemäß einem Volumenstrom mit einem vorbestimmten Volumen pro Zeit Teile der Inkubatoratmosphäre ausgetauscht werden, um insbesondere als Bestandteil eines Regelvorgangs die gewünschte Gaszusammensetzung der Inkubatoratmosphäre einzustellen, insbesondere wenn sich diese nach einem Öffnen der Inkubatortüre in unerwünschter Weise geändert hat oder ein Absaugen von Gasatmosphäre der Inkubatorkammer in einer Messkammer die Kammeratmosphäre, insbesondere den Kammerdruck, unerwünscht ändert. Der entsprechende zu messende physikalische Zustand ist in diesem Fall die relative Gaskonzentration oder die relative Luftfeuchtigkeit. Der Inkubator bzw. dessen Steuereinrichtung ist vorzugsweise dazu eingerichtet, die zu einem bestimmten Zeitpunkt als Sollwert eines Temperaturregelkreises angelegte die relative Gaskonzentration oder den infolge der relativen Gaskonzentration detektierten Gaswerte, insbesondere CO2 und/oder O2-Werte, zu regeln. Zu dieser Gasregelung lässt sich auch die Regelung der Luftfeuchtigkeit rechnen, die mittels eines Luftfeuchtesensors einer Sensoranordnung erfolgt, insbesondere mittels eines Sensors zur Messung der relativen Luftfeuchtigkeit.

Zu den vom Inkubator selbst gesteuerten Vorgängen können ferner Gasbewegungsvorgänge gehören, bei denen beispielsweise gemäß einem Volumenstrom mit einem vorbestimmten Volumen pro Zeit und insbesondere auch in einer oder mehrere variabler oder konstanter Strömungsrichtungen in der Inkubatorkammer Teile der Inkubatoratmosphäre bewegt werden. Dies kann insbesondere zu einer gleichmäßigeren Atmosphäre innerhalb der Inkubatorkammer führen, um insbesondere unterschiedlich positionierte Zellkulturbehälter im zeitlichen Mittel mit derselben Atmosphäre zu beaufschlagen.

Die Erfindung bezieht sich auch auf die Verwendung einer Sensoreinrichtung, wie in diesem Dokument beschrieben, zur Detektion einer Anreicherung, insbesondere Kontamination, von flüchtigen organischen Verbindungen (VOCs) in einer Gasatmosphäre in einem Innenraum eines Laborgeräts zur Behandlung von flüssigen, insbesondere biologischen Laborproben, insbesondere lebenden Zellen, insbesondere in der Gasatmosphäre des Innenraums der Inkubatorkammer eines Inkubators, wobei die VOCs freigesetzt wurden durch :
- Reinigungsmittelrückstände ;
wobei die Sensoreinrichtung mindestens einen VOC-Sensor zur Detektion der VOCs aufweist und der mindestens eine VOC-Sensor mindestens einen Messbereich aufweist, der in Strömungsverbindung mit dem Atmosphärengas des Innenraums stehend angeordnet ist. Der mindestens eine VOC-Sensor, dessen Ansteuerung und die Auswertung von dessen Messsignalen, kann gemäß der Beschreibung in diesem Dokument erfolgen, und kann insbesondere an die Erfordernisse zur Detektion der jeweiligen, aus den genannten Quellen stammenden, VOCs angepasst sein.

Weitere bevorzugte Ausgestaltungen des erfindungsgemäßen Inkubators, der Sensoreinrichtung und des Verfahrens, ergeben sich aus der nachfolgenden Beschreibung der Ausführungsbeispiele in Zusammenhang mit den Figuren und deren Beschreibung. Gleiche Bauteile der Ausführungsbeispiele werden im Wesentlichen durch gleiche Bezugszeichen gekennzeichnet, falls dies nicht anders beschrieben wird oder sich nicht anders aus dem Kontext ergibt. Es zeigen:
Fig. 1a zeigt eine perspektivische seitlich-frontale Ansicht eines erfindungsgemäßen Inkubators gemäß Ausführungsbeispiel, im geschlossenen Zustand der Gehäusetüre.
Fig. 1b zeigt eine perspektivische seitlich-rückwärtige Ansicht des Inkubators der Fig. 1a.
Fig. 1c zeigt eine perspektivische seitlich-frontale Ansicht des Inkubators der Fig. 1a, im geöffneten Zustand der Gehäusetüre.
Fig. 2 zeigt eine perspektivische seitlich-frontale Ansicht des Inkubators der Fig. 1a, mit ausgeblendeter Gehäusetüre und in einem Querschnitt entlang einer Ebene, die parallel zur Seitenwand und zentral durch den Inkubator verläuft.
Fig. 3a bis 3k zeigen jeweils einen anderen Inkubator gemäß einem jeweils bevorzugten Ausführungsbeispiel der Erfindung.
Fig. 4a zeigt eine Sensoreinrichtung mit Messkammer gemäß einem Ausführungsbeispiel der Erfindung, die insbesondere im Inkubator 1 der Figure 1a bis 2 verwendet werden kann.
Fig. 4b zeigt die eine Sensoreinrichtung mit Messkammer der Fig. 6a in einer Schnittansicht, ohne eingesetzte VOC-Sensoren und ohne deren Kabel.
In Fig 5 ist: der schematische Aufbau der zu Fig. 4a, 4b zuordenbaren Sensoreinrichtung gezeigt und deren Verbindung zu einer Steuereinrichtung des Inkubators.
Fig. 6 zeigt den schematischer Aufbau eines MOX-Sensors, der in einer erfindungsgemäßen Sensoreinrichtung verwendbar ist.
Fig. 7 zeigt schematisch die Gassteuerung bei einer VOC-Messung, die von den in den Fig. 4a bis 6 gezeigten Komponenten ausführbar ist.
Fig. 8 zeigt Diagramme mit Messwerten zu einer Messung von VOCs aus DH5□, die in Zellkulturflaschen in der Inkubatorkammer des erfindungsgemäßen Inkubators wuchsen.
Fig. 9 zeigt Diagramme mit Messwerten zu einer Messung von VOCs aus CHO-CD-Medium, die in Zellkulturflaschen in der Inkubatorkammer des erfindungsgemäßen Inkubators wuchsen.
Fig. 10 zeigt Diagramme mit Messwerten zu einer Messung von VOCs aus DH5a in CHO-CD-Medium, die in Zellkulturflaschen in der Inkubatorkammer des erfindungsgemäßen Inkubators wuchsen.
Fig. 11 zeigt Diagramme mit Messwerten zu einer Messung von VOCs aus DH5□ und CHO-S in CHO-CD-Medium, die in Zellkulturflaschen in der Inkubatorkammer des erfindungsgemäßen Inkubators wuchsen.
Fig. 12 zeigt Diagramme mit Messwerten zu einer Messung von VOCs aus DH5a in CHO-CD-Medium, die in Zellkulturflaschen in der Inkubatorkammer des erfindungsgemäßen Inkubators wuchsen.. Die aufgenommen Messsignale der Gassensoren werden über die Zeit aufgetragen und der ggf. vorliegende Alarmzeitpunkt markiert (vertikale schwarze Markierung).
Fig. 13a zeigt einen erfindungsgemäßen Inkubator gemäß einer weiteren bevorzugten Ausführungsform, mit nur einem, als MOX-Sensor ausgestalteten, VOC-Sensor.
Fig. 13b zeigt schematisch den MOX-Sensor der Fig. 13a.
Fig. 14 zeigt den stufenförmigen Verlauf der periodischen Ansteuerung (temperature cycled operation, TCO-Modus) des MOX-Sensors der Fig. 13b und des sich daraus ergebenden periodischen Verlaufs der elektrischen Leitfähigkeit des Messbereichs.
Fig. 15 zeigt den Verlauf der Messsignale einer Messperiode eines normalisierten Messzyklus, abgeleitet aus einer Messsignalkurve in einer Messperiode in Fig. 14.
Fig. 16a zeigt die Messsignale aus einer als Turbine gestalteten Sensoreinrichtung mit mehreren MOX-Sensoren, nachdem eine Bakterienprobe in das Kammerinnere eingebracht wurde.
Fig. 16b zeigt die Messsignale aus einer mit Einzelsensor gestalteten Sensoreinrichtung mit einem MOX-Sensor, gemäß Fig. 13a,b, nachdem, wie in Fig. 16a, eine Bakterienprobe in das Kammerinnere eingebracht wurde, wobei das Messsignal im TCO-Modus ermittelt und ein Sekundärfeature als Messsignal verwendet wurde.
Fig. 17a zeigt die Messsignale mehrerer Experimente, aus einer mit Einzelsensor gestalteten Sensoreinrichtung mit einem MOX-Sensor, gemäß Fig. 13a,b, nachdem eine Ethanolprobe in das Kammerinnere eingebracht wurde, wobei ein Messpunkt bzw. ein Messsignal als Mittelwert der Messsignale einer Messperiode im TCO-Modus ermittelt wurde.
Fig. 17b zeigt die Kurven aus Fig. 17b nach einer weiteren, mathematische Umformungen beinhaltenden, Auswertung.
Fig. 18 zeigt die Kurven aus Fig. 17b, nach Subtraktion eines Referenzverlaufs.
Fig. 19 zeigt die Maxima der Kurven aus Fig. 18, eingetragen in einem Diagramm, dessen Abszisse die Größe "Alkoholkonzentration der Probe" zeigt.
Fig. 20 zeigt ein Diagramm mit Messwerten zu einer Messung von VOCs aus verschiedenen Reinigungsmitteln, deren Rückstände auf einer Oberfläche in der Inkubatorkammer lokalisiert waren.

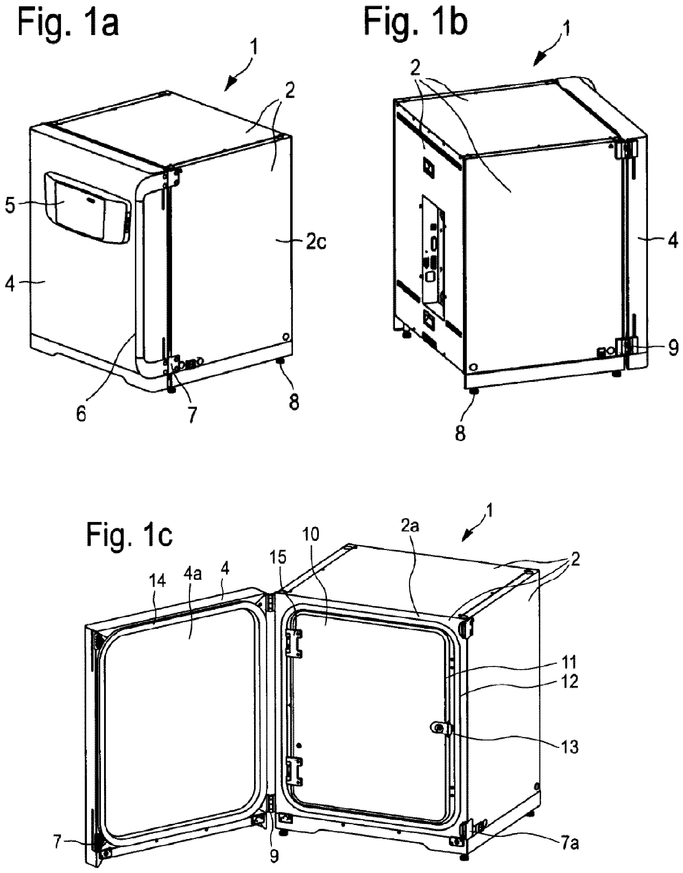

Fig. 1a zeigt die den Inkubator 1 für das Wachstum von Zellkulturen, hier ein als CO2-Inkubator ausgebildeten Labortemperierschrank für das Wachstum von eukaryotischen Zellen. Der Inkubator 1 hat ein Gehäuse 2 mit einem von mindestens einer Gehäusewand 2 umgebenen Gehäuseinneren und eine im Gehäuse angeordnete temperierbare Inkubatorkammer 3 (auch "Kammer 3") mit einem von mindestens einer Kammerwand umgebenen Kammerinneren zur Aufnahme der Laborproben. Die Außenwände des Gehäuses sind so miteinander verbunden, dass sie alle weiteren Bestandteile des Inkubators tragen, insbesondere auch die Sensoreinrichtung 30. Das Gehäuse ruht auf Sockeln 8. Im bestimmungsgemäßen Gebrauch sind die Außenseiten der Seitenwände 2c des Gehäuses, die Frontwand 2a, die Rückwand 2b sowie die Außenseite der Gehäusetüre 4 und deren Innenseite 4a, sowie die Seitenwände der Kammer, der Kammerfrontwand 3a, und die Kammerrückwand 3b vertikal, also parallel zur Gravitationsrichtung angeordnet. Die obere Außenseite 2d und die nicht sichtbare Bodenseite des Gehäuses und die Bodenwand und Deckenwand der Kammer sind entsprechend horizontal angeordnet. Als Richtung "nach unten" ist im Kontext der Beschreibung der Erfindung immer die Richtung der Gravitation gemeint, anhand der eine bestimmungsgemäß betriebener Inkubator ausgerichtet ist, die Richtung "nach oben" ist die entgegengesetzte Richtung. Die Richtung "nach vorne" bezeichnet die horizontale Richtung zur Vorderseite der geschlossenen Gehäusetüre, die Richtung "nach hinten" bezeichnet die horizontale Richtung zur Rückseite des Inkubators.

Die Kammer ist aus Edelstahl gefertigt, das Gehäuse aus lackiertem Metallblech. In der Kammer befinden sich auf der Oberfläche einer Kammerinnenwand Reinigungsmittelrückstände, die mittels der Sensoreinrichtung detektierbar sind, wenn sie sich in der Gasatmosphäre der Inkubatorkammer anreichern. Gegebenenfalls sind auch Zersetzungsprodukte (ein synonymer Begriff dafür sind auch Zerfallsprodukte) der Reinigungsmittelrückstände, die durch starke Erhitzung im Rahmen der Sterilisation entstehen, detektierbar, wenn sie sich in der in der Gasatmosphäre der Inkubatorkammer anreichern. Diese Zersetzungsprodukte werden als mögliche Reinigungsmittelrückstände im Sinne der vorliegenden Anmeldung verstanden.

Die Gehäusetüre 4 trägt eine Benutzerschnittstelleneinrichtung 5, die hier ein berührungsempfindliches Display umfasst, das vom Benutzer zum Lesen und Eingeben von Informationen verwendet wird, insbesondere zur Ausgabe von mittels der Sensoreinrichtung 20 gewonnen Informationen. Die Gehäusetüre weist zwei Scharniere 9 auf, die die Gehäusetüre mit dem Gehäuse 2 verbinden. Mittels einer Verriegelungseinrichtung 7; 7a, 7b wird die Gehäusetüre in der geschlossenen Position gehalten. Die Gehäusetüre weist einen Türgriff 6 auf.

In Fig. 1c ist die Gehäusetüre 4 geöffnet gezeigt. Die Kammertüre 10 ist mittels der Scharniere 15 an der Kammerfrontwand 3a befestigt, und wird in der gezeigten Position von einem Handverschluss 13 geschlossen gehalten, so dass das Kammerinnere nicht zugänglich ist. Aufgrund der Transparenz der Kammertüre 10 ist das Innere für den Benutzer in dieser Position aber sichtbar. Die Kammertüre wird durch eine umlaufende elastische Dichtung 11 der Kammertüre gasdicht an der Kammerfrontwand gehalten. Die Innenseite 4a der Gehäusetüre weist eine umlaufende elastische Dichtung 14 auf, die im geschlossenen Zustand der Gehäusetüre an der Gehäusefrontwand und der dort umlaufenden Dichtung 12 bündig anliegt und eine gasdichte Abschirmung des Bereichs zwischen Kammertüre 10 und Gehäusetüre 4a erzielt.

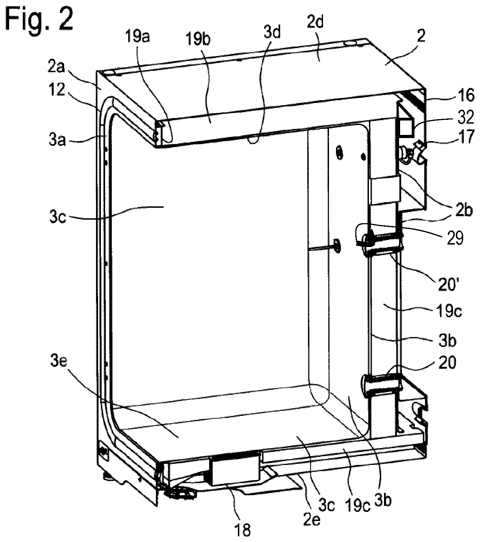

Wie in Fig. 2 teilweise sichtbar ist, weist der Inkubator zwei Temperiereinrichtungen auf, die den Kammerinnenraum 3 temperieren, d.h. deren Temperatur durch eine Temperaturregelung einstellen. Ein Teil der dazu notwendigen Bauteile 18 sind zwischen der Gehäusebodenwand 2e und der Kammerbodenwand 3e angeordnet. Die Heizwendel eines oberen Heizkreises (nicht gezeigt) sind mit der Außenseite der Kammerdeckenwand 3d und einem oberen Bereich der Kammerseitenwände, hier etwa dem oberen 2/3 entlang der Höhe der Seitenwände 3c der Kammer, thermisch gekoppelt und verbunden. Die Heizwendel eines unteren Heizkreises (nicht gezeigt) sind mit der Außenseite der Kammerbodenwand 3e und einem unteren Bereich der Kammerseitenwände, hier etwa dem unteren 1/3 entlang der Höhe der Seitenwände 3c der Kammer, thermisch gekoppelt und verbunden.

Eine thermische Isoliereinrichtung 19 (19a, 19b, 19c) ist zwischen Kammer und Gehäuse vorgesehen. Sie isoliert die Kammer, mit daran anliegenden Temperiereinrichtungen von dem Gehäuse, das an seiner Außenseite direkt mit der Umgebung in Verbindung steht. Der Inkubator arbeitet normalerweise bei Außentemperaturen zwischen 18 °C und 28 °C. Die Temperiereinrichtungen bzw. die Temperaturregelung arbeiten in diesem Bereich besonders effizient. Die Isoliereinrichtung umfasst ein U-förmig gebogenes Isolierelement 19b aus Glaswolle bzw. Mineralwolle, das die Kammerdeckenplatte und die beiden Kammerseitenwände 3c umgibt. Er mündet zum Boden und an die Rückwand hin an Isolierplatten 19c aus PIR-Schaum (Polyisocyanurat-Schaum), und wird zur Frontseite von Gehäuse und Kammer durch einen umlaufenden Nadelvliesstreifen 19a abgeschlossen, der an der Innenseite der Gehäusefrontwand 2a, der Kammerfrontwand 3a und der Dichtung 12 anliegt. Die thermische Isolierung der Kammer nach außen wird durch die erfindungsgemäßen Maßnahmen optimiert.

An der Gehäuserückwand 2b ist eine doppelte Gehäuserückwand 16 zur Abdeckung rückseitig angebrachter Komponenten, insbesondere der Messkammer 31 einer Sensoreinrichtung 30 befestigt. Die Rückwand ist mittels eines Griffs 17 abnehmbar.

Der Inkubator weist an seiner Rückseite zwei Zugangsports 20, 20' auf, die es ermöglichen, durch Öffnungen 20h, 20'h in der Rückwand der Kammer Leitungen, insbesondere mindestens eine Gasleitung zwischen Messkammer 32 und Inkubatorkammer 3, und/oder Kabel ins Innere der Kammer 3 zu verlegen, beispielsweise um eine optional im Inneren angeordnete Sensoreinrichtung anzusteuern. Wenn ein Zugangsport nicht benötigt wird, ist er von einem Stopfen 25 aus thermisch isolierendem Material, z.B. Silikonschaum, gefüllt.

Vorzugsweise mündet eine Gasleitung 29 im Innenraum der Inkubatorkammer 3, führt durch die Öffnung 20h der Kammerrückwand und/oder eine Öffnung im Port 20, zwischen Isoliermaterial 19c der thermischen Isoliereinrichtung 19 entlang der Kammerrückwand, um von dieser mittelbar temperierten Kammerwand temperiert zu werden, und tritt erst dann weg von der Kammerrückwand, durch das Isoliermaterial 19c hindurch in die vorzugsweise vorgesehene Messkammer 32, die hier in dem von der Gehäuserückwand 16 abgetrennten Bereich des Inkubators 1 angeordnet und mit diesem verbunden ist. Eine Abluftleitung der Messkammer 32 (nicht sichtbar) führt vorzugsweise durch die Gehäuserückwand 16 in die Umgebung des Inkubators.

Bevorzugte andere Ausgestaltungen einer Sensoreinrichtung, von mindestens einem VOC-Sensor, und einer Messkammer und deren bevorzugten Anordnungen am Inkubator, insbesondere am Inkubator 1, werden anhand der nachfolgenden Figuren beschrieben.

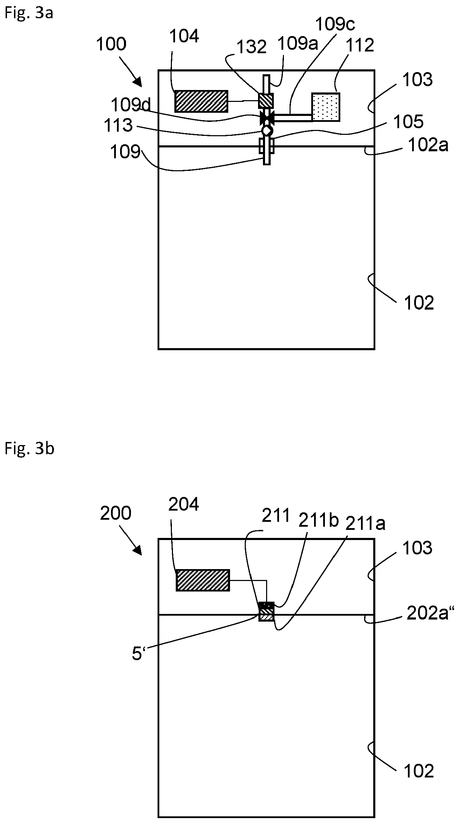

Fig. 3a zeigt: Einen Inkubator 200, mit einer Inkubatorkammer 102 und einem außerhalb der Inkubatorkammer gelegenen Gehäuseabschnitt 103 ("Außenbereich") des Inkubators; diese Bestandteile weist jeder Inkubatoren in den Figuren 3a bis 3k auf. In Fig. 3a wird per Förderung der Kammeratmosphäre mittels Fördermittel 113 (Pumpe oder Ventilator) der Gasaustausch aus dem Inneren der Kammer 102 mittels Gasleitung 109 via Port 105 durch die Kammerwand 102a zu einer Messkammer 132 der VOC-Sensoreinrichtung 130 bewirkt, die mit Steuerung 104 im Außenbereich 103 lokalisiert ist, und Abgase der VOC-Messkammer 132 werden über die Abluftleitung 109a an die Umgebung abgegeben; ein Spülgas 112 wird über eine Spülgasleitung 109c und das Ventil 109d zur VOC-Messkammer geleitet, um diese vor einer Messung zu spülen.

Fig. 3b zeigt: einen Inkubator 200: Eine VOC-Sensor-Steuerung (204) ist im Außenbereich 103 lokalisiert, der Messbereich, nämlich die MOX-Seite 211a des VOC-Sensors 211 liegt im Innenbereich der Kammer 102, eine Heizseite 211b des VOC-Sensors 211 liegt im Außenbereich 103, der VOC-Sensor 211 ist dichtend in Loch 205' in Kammerwand 202a" eingebaut.

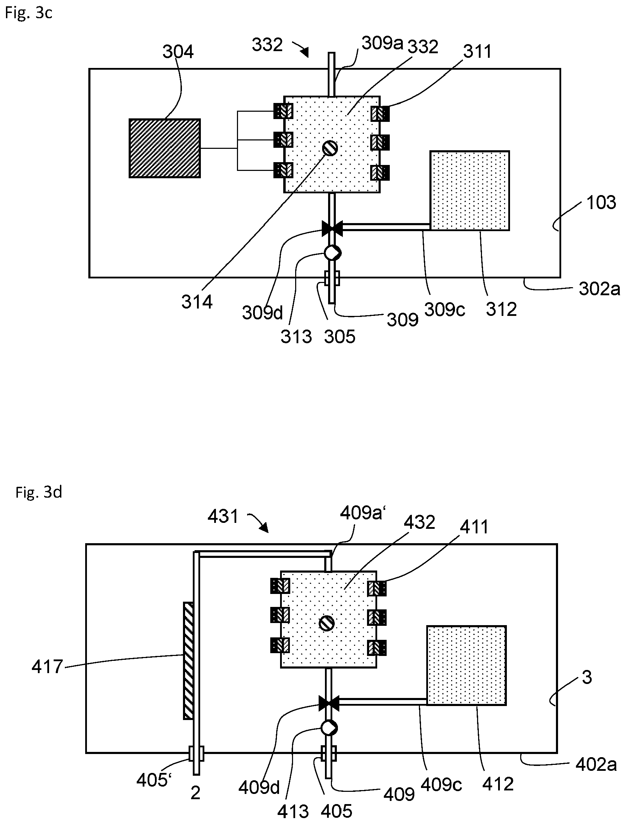

Fig. 3c zeigt: einen Inkubator 300: Per Pumpen 313-Förderung erfolgt der Gasaustausch aus dem Inneren der Kammer 102 mittels Gasleitung 309 via dem Port 305 durch die Kammerwand 302a zu einer Messkammer 332 der VOC-Sensoreinrichtung bzw. e-Nase 331 mit ihren 6 VOC-Sensoren 311, die mit Steuerung 304 im Außenbereich 103 lokalisiert ist, und Abgase von VOC-Messkammer 332 werden über die Abluftleitung 309a an die Umgebung abgegeben; Spülgas 312 wird vor einer Messung über die Spülgasleitung 309c und Ventil 309d zur VOC-Messkammer gefördert.

Fig. 3d zeigt: einen Inkubator 400: Per Pumpen 413-Förderung erfolgt der Gasaustausch aus dem Inneren der Kammer 102 mittels Gasleitung 409 via dem Port 405 durch die Kammerwand 402a zu einer Messkammer 43 der VOC-Sensoreinrichtung bzw. e-Nase 431 mit ihren 6 VOC-Sensoren 411, die mit Steuerung 404 im Außenbereich 103 lokalisiert ist, und Abgase von der VOC-Messkammer 432 werden über die Abluftleitung 409a' und optional den Filter 415 via dem Port 405' zurück durch die Kammerwand 402a in die Kammer 102 geführt; Spülgas 412 wird über die Spülgasleitung 409c und das Ventil 409d zur VOC-Messkammer befördert. Optional ist eine Beheizung bzw. Heizungen 417 der Leitungen 409 vorgesehen, um Kondensation zu verhindern.

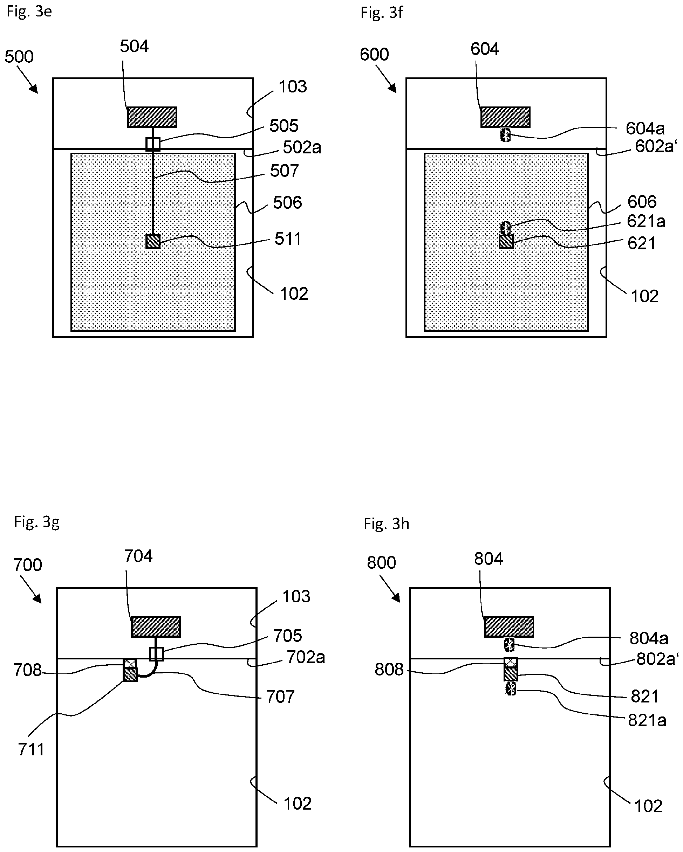

Fig. 3e zeigt: einen Inkubator 500: ein VOC-Sensor 511 ist auf einem Regalbrett 506 in der Kammer 102 angeordnet und mit dem Kabel 507 und kabel-basierter Signalverbindung via dem Port 505 durch eine Kammerwand 502a zu einer VOC-Steuerung 504 im Außenbereich 103 verbunden.

Fig. 3f zeigt: einen Inkubator 600: ein VOC-Sensor 621 ist auf einem Regalbrett 606 in der Kammer 10 montiert und mit kabelloser, funk 621a, 604a-basierter Signalverbindung durch eine portfreie Kammerwand 602a' mit einer VOC-Steuerung 604 im Außenbereich 103 verbunden.

Fig. 3g zeigt: einen Inkubator 700: ein VOC-Sensor 711 ist mittels Magnethefter 708 in der Kammer 102 an der Kammerwand 702a befestigt und mit einer Kabel 707-basierter Signalverbindung via dem Port 705 durch die Kammerwand 702a mit einer VOC-Steuerung 704 im Außenbereich 103 verbunden.

Fig. 3h zeigt: einen Inkubator 800: ein VOC-Sensor 821 ist mittels Magnethefter 808 in der Kammer 102 mit der Kammerwand 802a' verbunden mit kabelloser, funk 821a, 804a-basierter Signalverbindung durch eine portfreie Kammerwand 802a' mit einer VOC-Steuerung 804 im Außenbereich 103 verbunden.

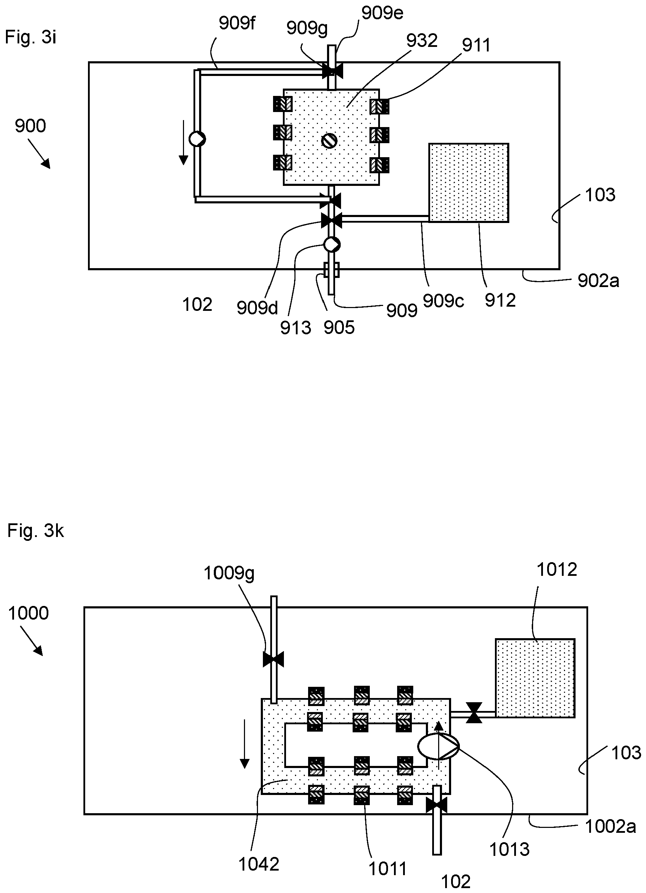

Fig. 3i zeigt: einen Inkubator 900: gezeigt ist eine Messkammer mit Ringverlauf und zirkulierendem Kammergas: Per Pumpen 913-Förderung erfolgt der Gasaustausch aus dem Inneren der Kammer 102 mittels der Gasleitung 909 via dem Port 905 durch die Kammerwand 902 zu einer Messkammer 932 der VOC-Sensoreinrichtung bzw. e-Nase 931 mit ihren hier 6 VOC-Sensoren 911d, die mit der Steuerung 904 im Außenbereich 103 lokalisiert ist, und nach Schließen der Ventile 909d und 909g werden Abgase von der VOC-Messkammer 932 über Zirkulierleitungen 909f mittels Pumpe 916 zirkulierend durch die Messkammer 932 gefördert, um eine kontinuierliche Konvektion des VOC-enthaltenden Kammergases ohne größeren Verlust von Kammer 102-Gas bzw. Atmosphäre zu ermöglichen; Spülgas 912 wird über eine Spülgasleitung 909c und ein Ventil 909d zur VOC-Messkammer befördert. Infolge des zirkulierenden Gasvolumens wird über dieses hinaus kein Gasvolumen der Kammer 102 entnommen, wodurch der Kammergasverlust minimiert ist.

Fig. 3k zeigt: einen Inkubator 1000: gezeigt ist eine zirkular gestaltete Messkammer für Messung an zirkulierendem Gas: Per Pumpen 1013-Förderung erfolgt der Gasaustausch aus dem Inneren der Kammer 102 mittels der Gasleitung 1009d via dem Port 1005 durch die Kammerwand 1002a zu einer ringförmigen Messkammer 1042 der VOC-Sensoreinrichtung bzw. e-Nase 1041 mit ihren hier 12 VOC-Sensoren 1011, und nach Schließen der entsprechenden Ventile werden Abgase zirkulierend durch die VOC-Messkammer 1042 mittels der Pumpe 1013 gefördert, um kontinuierliche Konvektion des VOC-enthaltenden Kammergases ohne größeren Verlust von Kammer 102-Gas bzw. Atmosphäre zu ermöglichen; ein Spülgas 1012 wird über die Spülgasleitung 1009c und das Ventil 1009d zur VOC-Messkammer befördert. Vorteile: das Kammergas wird auf kürzestem Weg der Kammer entnommen, es erfolgt somit kaum Kondensation; entnommen werden muss der Kammer 102 aber nur das für die schmale ringförmige VOC-Messkammer benötigte Volumen; und während der Messung zirkuliert das Gasvolumen; in Folge geht im Vergleich zum Aufbau mit Gaszuleitung und Abgasleitung zur Umgebung weniger Kammergas verloren.

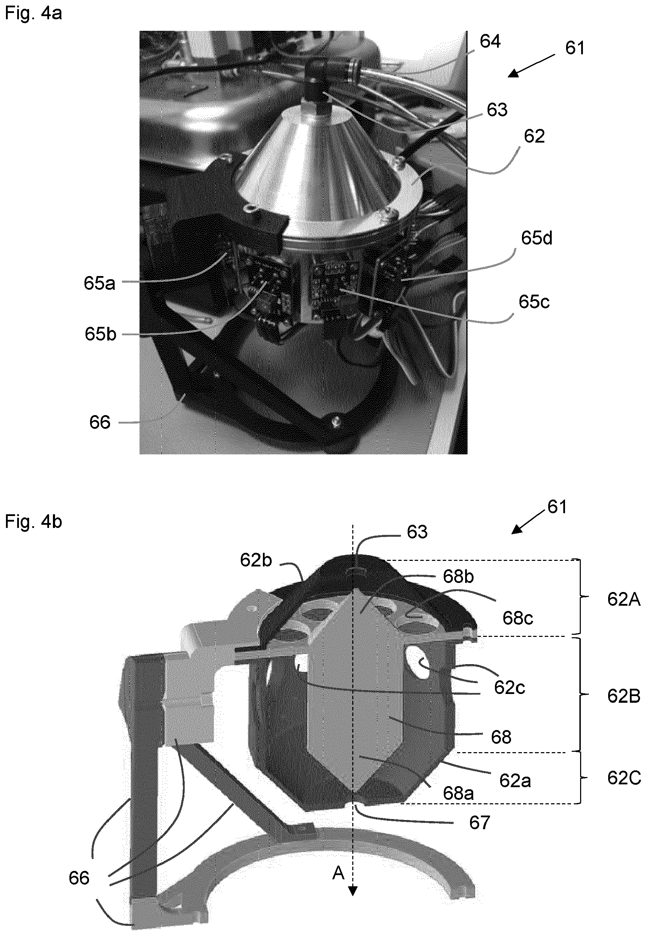

Fig. 4a zeigt eine Sensoreinrichtung 61 mit Messkammer 62 gemäß einem Ausführungsbeispiel der Erfindung in einer perspektivischen Seitenansicht von schräg oben, die insbesondere im Inkubator 1 der Figuren 1a bis 2 verwendet werden kann. Die Messkammer ist ein hohlspindelförmiger Körper, der in der Fig. 4a an seinem oberen Ende eine Zuflussöffnung 63 für den Zufluss von Atmosphärengas aus der Inkubatorkammer mittels der Gasleitung 64 erlaubt, und einen Abfluss durch die Abflussleitung 67. Die Messkammer ist mittels einer Halterung 66 am Inkubator befestigbar, insbesondere verschraubbar, verlötbar oder anders unbeweglich am Inkubator, insbesondere einer Gehäusewand oder Kammerwand verbindbar. Die Messkammer 62 weist einen Teilhohlkörper 62a auf, in den ein Gasführungskörper 68 gesetzt wird, und der mit einem Deckelteil 62b abgedeckt wird. Die Teile 62a, 6sb und 68 sind fest miteinander verbunden. An der Außenseite der Messkammer 62 sieht man die Rückseiten 65a bis 65i der -hier neun- MOX-VOC-Sensoren, die jeweils einen Messbereich bzw. eine beheizte MOX-Adsorptionsfläche (nicht sichtbar) an ihrer Vorderseite aufweisen, die der zentralen Längsachse A der Messkammer zugewandt und parallel zu dieser angeordnet sind.

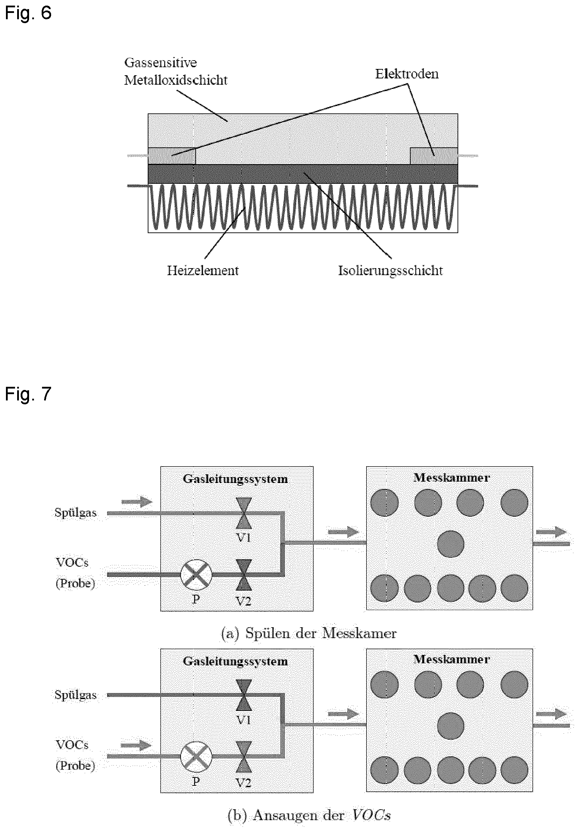

Fig. 4b zeigt eine durch Schnitt des Modells geöffnete Ansicht der Sensoreinrichtung 61 mit Messkammer der Fig. 6a in einer perspektivischen Seitenansicht, ohne eingesetzte VOC-Sensoren und ohne deren Kabel.

Die Messkammer 62 weist, wie in Fig. 4b gezeigt ist einen oberen hohlkonusförmigen Abschnitt 62A auf, einen mittleren hohlzylinderförmigen Abschnitt 62B und einen unteren hohlkonusförmigen Abschnitt 62C. An der Spitze des hohlkonusförmigen Abschnitt 62A ist die Zuflussöffnung 63 für das Gas vorgesehen, an der Spitze des hohlkonusförmigen Abschnitt 62C ist die Abflussöffnung 63 für das Gas vorgesehen. Die Hauptströmungsrichtung A ergibt sich aus der geraden Verbindung der Zentren der kreisförmigen Zuflussöffnung 63 und der kreisförmigen Abflussöffnung 67. An der der Zuflussöffnung zugewandten Spitze des Abschnitts 68b des Gasführungskörpers ist ein Umgebungssensor (nicht sichtbar; Druck, Temperatur, Luftfeuchte) angeordnet, der -ebenso wie die neun VOC-Sensoren, mit der elektronischen Auswertungseinrichtung für den Signalaustausch verbunden sind.

Der Gasführungskörper ist spindelförmig und so koaxial zur Achse A mit dem hohlspindelförmigen Verlauf der Außenwand der Messkammer 62 angeordnet, dass sich zwischen der Innenseite der Außenwand der Messkammer 62 und der Außenseite des Gasführungskörpers ein hülsenförmiger, bzw. ein Strömungskanal mit ringförmigen Querschnitten ergibt. Das Gas wird auf diese Weise gleichförmig und insbesondere unter Eliminierung von Wirbelbildung -also möglichst laminar- an den Messbereichen der VOC-Sensoren 65a-65i vorbeigeführt, die an den kreisförmigen Öffnungen 62c der Außenwand der Messkammer 62 dicht anliegen, so dass an jeder der neun Öffnungen 62c dieselbe Fläche einer MOX-Adsorptionsfläche des Messbereichs des jeweiligen VOC-Sensors im Kontakt mit dem parallel zur Strömungsrichtung A vorbeiströmendem Gas steht. Durch die Führungselemente und die Gleichförmigkeit des Gasstroms wird die mittels der Sensoreinrichtung 61 durchgeführte Messung besonders sensibel, zudem reproduzierbar und zuverlässig. Indem die Rückseite der MOX-VOC-Sensoren außerhalb der Messkammer und nicht im Kontakt mit dieser angeordnet sind, wird der Wärmeübertrag zwischen den Heizelementen der Sensoren und der Messkammer minimiert, die Rückseiten sind zudem einfach per Konvektion/Luftstrom kühlbar.

Ausführungsbeispiel eines Inkubators mit Sensoreinrichtung und elektronischer Nase

Der nachfolgend beschriebene erfindungsgemäße Inkubator weist den Aufbau gemäß den Figuren 1a bis 2 auf und verwendet eine Sensoreinrichtung 61 aus Fig. 4a, 4b (zur Realisierung der Sensoreinrichtung 30). Die Sensoreinrichtung 61 ist als elektronische Nase mit insgesamt neun verschiedenen VOC-Sensoren ausgebildet.

### Grundlegendes zu VOCs:

Aus VOC-haltigen Reinigungsmitteln werden VOCs freigesetzt. Die Sensoreinrichtung 61 ist nach dem Prinzip einer Elektronische Nase aufgebaut und misst VOCs. Sie ermöglicht einen Rückschluss auf die Reinigungsmittelrückstände in der Inkubatorkammer, die mit Änderungen der VOC-Konzentration in der Inkubatorkammer verbunden sind.

Es werden hier insgesamt 9 Gassensoren mit möglichst unterschiedlicher Selektivität verbaut. Diese reagieren somit unterschiedlich auf mögliche VOCs und erzeugen damit ein charakteristisches Messsignalmuster. Das Messsignalmuster kann zeitabhängig erfasst werden. Das Messsignalmuster enthält Informationen, die analysiert und in eine semantische Aussage gebracht werden sollen. Jeder dieser neun Gassensoren kann auch in einer Sensoreinrichtung mit weniger als neun Gassensoren oder mit nur einem einzigen Gassensor verwendet werden.

Durch die Vielfalt der Gassensoren kann eine bessere Unterscheidung zwischen verschiedenen Reinigungsmittelbestandteilen getroffen werden. Bspw. entsteht teilweise bei verschiedenen Reinigungsmitteln ein ähnliches Messsignalmuster, aber der Signalverlauf einzelner Gassensoren unterscheidet sich charakteristisch. Der Vorteil der Verwendung mehrerer verschiedener Gassensoren ist also die Erhöhung des Informationsgehalts der Messung.

Die Sensoreinrichtung 61 verfügt über eine Messkammer (MK), ein Gasleitungssystem (GS) und eine Verarbeitende Einheit (VE). Die MK beinhaltet die Gassensoren (VOC-Sensoren), welche die VOCs messen. Das GS leitet die VOCs mithilfe von Aktuatoren zur MK. Die VE steuert das GS, liest die Gassensoren und den Umgebungssensor aus, verarbeitet die Daten und stellt eine Kommunikationsschnittstelle zum Inkubator bereit. Die VE beinhaltet eine elektronische Steuerungseinrichtung der Sensoreinrichtung, welche die eine Datenverarbeitungseinrichtung aufweisende Auswertungseinrichtung beinhaltet. Ein Mikrocontroller der Steuerungseinrichtung ist hier der Raspberry Pi 3 B (Raspberry). Die Kommunikationsschnittstelle ermöglicht den Informationsfluss zwischen einem AI-Modul und Messkammer und somit die Steuerung der Sensoreinrichtung 61 mithilfe des Nutzerinterfaces.

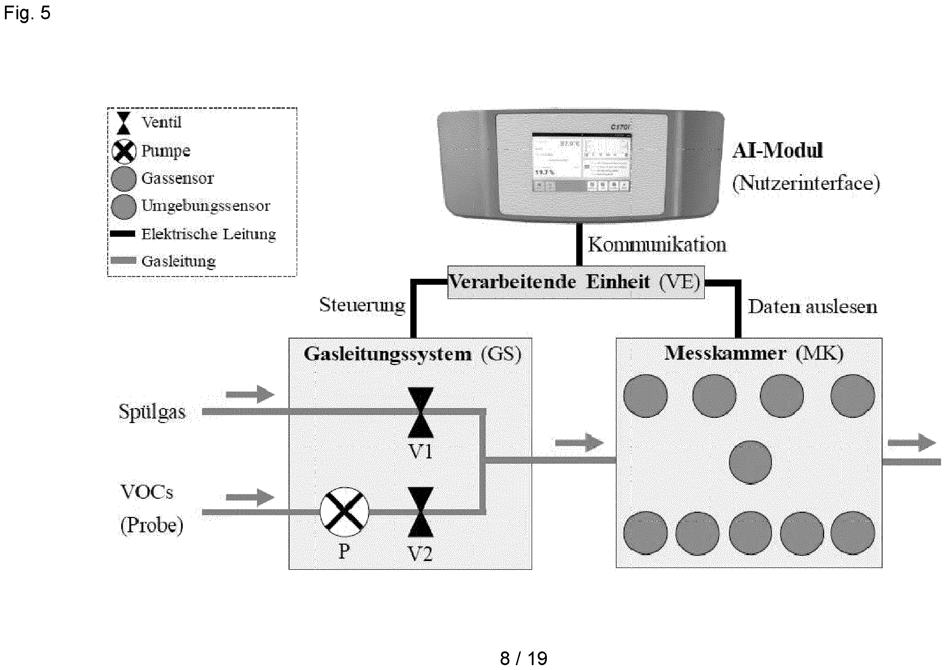

In Fig 5 ist gezeigt: der schematische Aufbau der Sensoreinrichtung 61 und deren Verbindung zu einer Steuereinrichtung des Inkubators, auch bezeichnet als AI-Modul. Die MK beinhaltet die Gassensoren, den Umgebungssensor, einen Ein- und einen Ausgang. Das GS leitet mithilfe von Gasleitungen der Ventile und der Pumpe entweder Spülgas oder VOCs (also Kammeratmosphärengas) in die MK. Die VE ist über elektrische Leitungen mit dem GS und der MK verbunden. Sie steuert das GS, ließt die Gassensoren und den Umgebungssensor aus, verarbeitet die Daten und stellt eine Kommunikationsschnittstelle zum Inkubator bereit. Die Kommunikationsschnittstelle ermöglicht den Informationsfluss zwischen AI-Modul und Sensoreinrichtung 61 und somit die Steuerung der Sensoreinrichtung 61 mithilfe des Nutzerinterfaces (Benutzerschnittstelleneinrichtung).

Damit vor jeder VOC-Analyse in diesem Ausführungsbeispiel möglichst immer dieselben Startbedingungen geschaffen werden, wird vor dem Einleiten der VOCs die MK gespült. Der Spülvorgang ist vorteilhaft für das Generieren von vergleichbaren Messergebnissen. Wenn die Sensoreinrichtung 61 nicht benutzt wird, können entweder Gase aus der Umgebung in die MK eindringen oder VOCs der vergangenen VOC-Messung in der MK zurückbleiben. Vorzugsweise wird immer ein Spülgas konstanter Zusammensetzung verwendet. Um den Anteil von sich ändernden Gasen im Spülgas gering zu halten, wird vorzugsweise Stickstoff 5.0 als Spülgas verwendet. Dieser besitzt hier einen Reinheitsgrad von >99.999%.

Die MK hat einen Ein- und Ausgang (Zu- und Abfluss). Über den Eingang werden Spülgas oder VOCs in die MK eingeleitet. Vor dem Eingang befindet sich eine Y-Kopplung, welche die Spül- und VOC-Leitung zusammenführt. Über den Ausgang entweichen die Gase wieder aus der MK. Der Ein- und Ausgang befinden sich vorzugsweise gegenüber und mittig auf einer jeweiligen Außenwand der MK, um einen möglichst gleichmäßigen Gasdurchfluss und -verteilung zu gewährleisten.

Als Gassensoren (VOC-Sensoren) werden vorzugsweise MOX-Sensoren verwendet.

Es existieren verschiedene Analysemethoden, um VOCs zu detektieren, wozu u.A. auch die Elektronische Nase gehört. Eine Elektronische Nase nutzt ein Sensor-Array, um mithilfe von Mustererkennung einen Fingerabdruck für einen gegebenen Geruch zu generieren und diesen von Fingerabdrücken anderer Gerüche zu unterscheiden. Auf diese Weise mimt eine Elektronische Nase das olfaktorische System von Säugetieren nach und erlaubt das Erkennen von Gerüchen als Ganzes und damit die Quelle des Geruchs ausgemacht werden kann. So kann bspw. die Identifikation von Reinigungsmitteln erfolgen, indem aufgrund der detektierten Mixtur von charakteristischen VOCs ein Rückschluss auf die Quelle gezogen wird.

Das Messsystem einer elektronische Nase ist insbesondere aufgebaut aus einer Probenleitenden Einheit, Detektionseinheit als auch Berechnungseinheit und die verwendeten Gassensoren werden vorzugsweise so gewählt, dass diese sensitiv für die auftretenden Gasmoleküle sind, aber die einzelnen Gassensoren unterschiedlich stark auf diese reagieren. Hier werden Metalloxid-Halbleiter (MOX) Gassensoren verwendet, welche zu der Klasse der chemischen Sensoren gehören. Chemische Sensoren besitzen eine Detektionsschicht, mithilfe einer chemischen Interaktion in ein elektrisches Signal transformiert werden kann und sind darüber hinaus nicht nur kostengünstig, sondern können auch im kontinuierlichen Messbetrieb verwendbar.

Der Aufbau und die Funktionsweise von MOX-Sensoren: Der Sensormechanismus basiert darauf, dass abhängig von der Konzentration des Zielgases die elektrische Leitfähigkeit der gassensitiven Metalloxidschicht bzw. des Halbleiters verändert und damit die Anwesenheit als auch Menge des Zielgases bestimmt wird. Typischerweise besteht ein MOX-Sensor aus vier Elementen: Gassensitive Metalloxidschicht, Elektroden, Heizelement und Isolierungsschicht (siehe Fig. 6).

Fig. 6 zeigt den schematischer Aufbau eines MOX-Sensors. Ein MOX-Sensor besteht aus 4 Elementen: Gassensitive Metalloxidschicht, Kontaktelektroden, Heizelement und Isolierungsschicht. Das Heizelement ist von der gassensitiven Metalloxidschicht und den Kontaktelektroden durch die Isolierungsschicht getrennt. Die gassensitive Metalloxidschicht wird durch das Heizelement erhitzt und Sauerstoffmoleküle aus der Umgebung werden an der Oberfläche der gassensitiven Metalloxidschicht adsorbiert. Die adsorbierten Sauerstoffmoleküle fangen Elektronen von den leitenden Bändern des Halbleiters ein und es bilden sich energetische Barrieren, die so einen Teil des Elektronenflusses im Halbleiter blockieren und somit die elektrische Leitfähigkeit verschlechtern bzw. den Widerstand des Gassensors erhöhen. Sobald reduzierende Gase (Zielgase) präsent sind, reagieren diese mit den gebundenen Sauerstoffmolekülen. Die Sauerstoffmoleküle werden von der Oberfläche der gassensitiven Metalloxidschicht gelöst und die Leitfähigkeit steigt bzw. der Widerstand sinkt.

Hersteller geben in der Regel Umgebungsbedingungen an, in denen die MOX-Sensoren valide Messwerte liefern und wie stark sie von diesen beeinflusst werden. Um zu überblicken, wie sehr die Umweltbedingungen einen Einfluss auf die VOC-Messungen haben, wird zusätzlich ein Umgebungssensor mittig in der MK angebracht. Zu den relevanten Umweltbedingungen zählen die Feuchtigkeit, Temperatur und der Umgebungsdruck.

Der Prozess einer VOC-Messung ist vorzugsweise in zwei Phasen eingeteilt - das Spülen und Einleiten der VOCs. Der Inkubator mit Sensoreinrichtung wird zunächst für die Messung initialisiert, es wird gespült. Die Gassensoren werden für die Messung vorzugsweise kontinuierlich ausgelesen und zwischengespeichert. Bei Bedarf kann der Nutzer die zwischengespeicherten Daten dauerhaft abspeichern und ggf. exportieren. Die VOC-Messung selbst wird vom Nutzer oder automatisch vom Inkubator durch das Steuern des GS gestartet oder beendet. Die zu steuernden Elemente sind die Ventile und die Pumpe. Zum Starten der VOC-Messung wird im Beispiel der Spülvorgang eingeleitet. Ventil 1 (V1) ist geöffnet, Ventil 2 (V2) geschlossen und die Pumpe (P) deaktiviert (siehe Fig. 7 oben). Wenn der Spülvorgang beendet und die Zufuhr der VOCs gestartet werden soll, wird Ventil 1 (V1) geschlossen, Ventil 2 (V2) geöffnet und die Pumpe (P) gestartet (siehe Fig. 7 unten). Zum Beenden der VOC-Messung wird die Pumpe (P) ausgeschaltet und Ventil 2 (V2) geschlossen. Die Sensoreinrichtung wird vorzugsweise über ein Netzteil des Inkubators mit Spannung versorgt. Dies betrifft die VE, die Gassensoren der MK, die Ventile und die Pumpe des GS.

Fig. 7 zeigt schematisch die Gassteuerung bei einer VOC-Messung. Diese ist vorzugsweise ist in Spülen und Ansaugen von VOCs aufgeteilt. Gas fördernde Bauteile werden grün markiert und nicht fördernde rot. Während des Spülvorgangs ist Ventil 1 geöffnet, Ventil 2 geschlossen, die Pumpe deaktiviert und das Spülgas fließt durch die Messkammer. Während des Ansaugens der VOCs ist Ventil 1 geschlossen, Ventil 2 geöffnet, die Pumpe aktiviert und die VOCs fließen durch die Messkammer.

Die MK besteht aus einer Aluminium-Spritzgusskammer mit anschraubbaren Deckel und beinhaltet die typenunterschiedlichen Gassensoren (MQ 1, MQ 2, MQ 3, MQ 4, MQ 5,MQ 6, MQ7,MQ 8, MQ9 und MQ135 bzw. Bezugszeichen 65a-i; herkömmlich bezogen von der HANWEI ELETRONICS CO.,LTD) und den Umgebungssensor (BME680). Der Umgebungssensor stellt die geforderten Umweltparameter Temperatur, Feuchtigkeit und Druck bereit und wurde innerhalb der MK platziert. Die Gassensoren wurden so gewählt, dass sie für die potentiell auftretenden Stoffgruppen der VOCs größtenteils selektiv sind und wurden gemäß des aufgestellten Konzepts anliegend an die MK platziert. Die Anschlussstellen zwischen Gassensoren und MK wurden mit Silikon abgedichtet. Die jeweilige Selektivität der Gassensoren kann in Tabelle 1 unter der Details-Spalte eingesehen werden.

**Tabelle 1 Hardwareteile der Messkammer (MK)|**

| Messkammer (*MK*) | | |
|---|---|---|
| Bauteil | Bezeichnung | Details |
| Gassensor | MQ 2 | Alkane (Butan, Propan, Methan) |
| | | Alkohole |
| | | Wasserstoff |
| Gassensor | MQ 3 | Alkohole |
| Gassensor | MQ 4 | Alkane (Methan) |
| Gassensor | MQ 5 | Alkane (Methan, Propan, Butan) |
| Gassensor | MQ 6 | Alkane (Methan, Propan, Butan) |
| Gassensor | MQ 7 | Oxide (Kohlenstoffmonoxid) |
| Gassensor | MQ 8 | Wasserstoff |
| Gassensor | MQ 9 | Kohlenstoffmonoxid |
| | | Alkane (Methan, Propan, Butan) |
| Gassensor | MQ 135 | Alkohole |
| | | Benzole (Benzol) |
| | | Amine (Ammoniak) |
| | | Oxide (Kohlenstoff-, Stickstoffdioxid) |
| Umgebungssensor | Adafruit BME680 | Temperatur, Feuchtigkeit, Druck |
| Spritzgusskammer | Hammond | Aluminium, 170 × 120 × 55 mm |
| Silikon | | Abdichten Gassensoren |

Die VOCs gehören zu den Stoffgruppen der Benzole, Alkylbenzole, Ketone, Alkohole, Alkane, Terpene, Säuren, Carbonsäuren, Ester, Aldehyde, Alkene, Heterocyclische Amine und Indolen. Der größte Anteil der aufgeführten VOCs gehört zu der Stoffgruppe der Alkohole. Ein Teil der verwendeten Gassensoren sind nach den Angaben des Herstellers für Gase der Stoffgruppen der Alkohole, Alkane, Benzole und Amine selektiv. Dem entsprechend sollten die Gassensoren MQ2, MQ3, MQ4, MQ5, MQ6, MQ9 und MQ135 auf die VOCs der DH5 ansprechen und ein Anstieg der Messsignale zu verzeichnen sein. Da der größte Anteil der entstehenden VOCs zur Gruppe der Alkohole gehören, erzeugen die Gassensoren MQ2, MQ3 und MQ135 höhere Messsignale als die anderen Gassensoren.

**Tabelle 2 DH5α emittierte VOCs (in IUPAC-Schreibweise) und deren chemische Klassifizierung [4].**

| IUPAC-Name | Chemische Klassifizierung |
|---|---|
| 1,2.3-Trimethylbenzol | Benzole, Alkylbenzole |
| 1-(4-Methylphenyl)ethanon | Benzole, Ketone |
| 1,4-Xylen | Benzole, Alkylbenzole |
| 2-(4-Methyl-3-cyclohexen-1-yl)-2-propanol | Alkohole, Terpene |
| 2-Ethyl-1-hexanol | Alkohole |
| 2-Phenylethanol | Alkohole |
| 3-Hydroxy-2-butanon | Alkohole, Ketone |
| 2,3-Butandiol | Alkohole |
| 2-Decanol | Alkohole |
| Dodecan | Alkane |
| Octadecan | Alkane |
| Hexansäure | Säuren, Carbonsäuren |
| Nonansäure | Säuren, Carbonsäuren |
| Octansäure | Säuren, Carbonsäuren |
| Ethyl-octanoat | Ester |
| 3-Methylbutyl-acetat | Ester |
| Lauraldehyd | Aldehyde |
| 2-Methylpentanal | Aldehyde |
| 4-Methyl-1-hexen | Alkene |
| 1H-Indol | Indole, Heterocyclische Amine |

Chinese Hamster Ovary Zellkulturen (CHO) sind häufig im Laboralltag anzutreffende Zelltypen. Die von CHO abgegebenen VOCs gehören zu den Stoffgruppen der Alkane, Aldehyde, Ester, Benzole, Ketone, Pyrazole, Oxime und Alkohole. Der größte der aufgeführten VOCs gehört zu der Stoffgruppe der Alkane. Ein Teil der verwendeten Gassensoren sind nach den Angaben des Herstellers für Gase der Stoffgruppen der Alkohole, Alkane und Benzole selektiv. Dem entsprechend sollten die Gassensoren MQ2, MQ3, MQ4, MQ5, MQ6, MQ9 und MQ135 auf die VOCs der CHOs ansprechen und ein Anstieg der Messsignale zu verzeichnen sein. Da der größte Anteil der entstehenden VOCs zur Gruppe der Alkane gehören, sollten die Gassensoren MQ2, MQ4, MQ5, MQ6, und MQ9 höhere Messsignale als die anderen Gassensoren erzeugen. Wie auch bei den DH5 müssen die Zellkulturen bei jedem Versuch eine gleichbleibende Wachstumsdynamik aufweisen. Im Gegensatz zu den DH5 wurden die CHOs nicht eigenständig gezüchtet, sondern vom Anmelder bereitgestellt und nach den internen Standardprozeduren gezüchtet.

Die Auswertungseinrichtung (VE) verwendet einen Algorithmus, um zu erkennen, ob in einer Testprobe (CHO mit/ohne DH5a) eine Kontamination vorliegt oder nicht. Der entwickelte Algorithmus basiert auf der sequentiellen CUSUM-Analysetechnik (auch CUSUM Control Chart genannt) und wurde zuerst von Page vorgestellt. Auch ein Kl-Algorithmus, insbesondere ein neuronales Netz wäre zur Auswertung möglich. Die CUSUM-Analysetechnik wird genutzt, um die Abweichungen eines laufenden Prozess zu überwachen. xᵢ sei die i-te Beobachtung des Prozesses. Der Prozess wird in zwei Zustände eingeteilt - entweder ist er unter Kontrolle oder nicht. Wenn der Prozess unter Kontrolle ist, unterliegt xᵢ einer Normalverteilung mit einem Mittelwert µ0 und einer Standardabweichung σ. µ0 wird häufig als Zielwert interpretiert, dem xᵢ möglichst nahe sein muss, damit der Prozess unter Kontrolle bleibt.

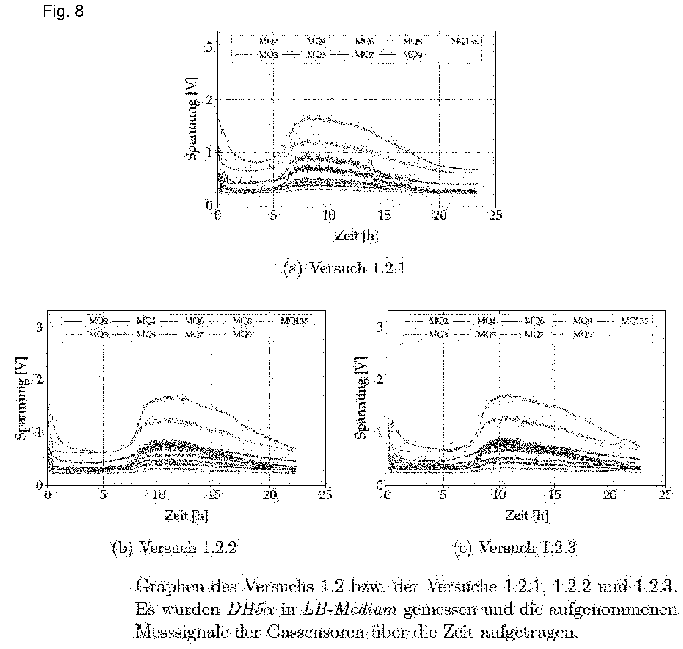

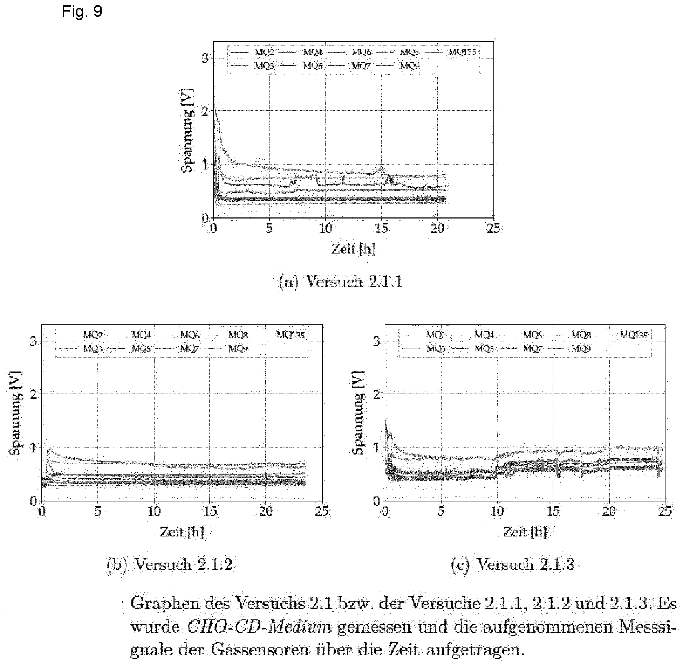

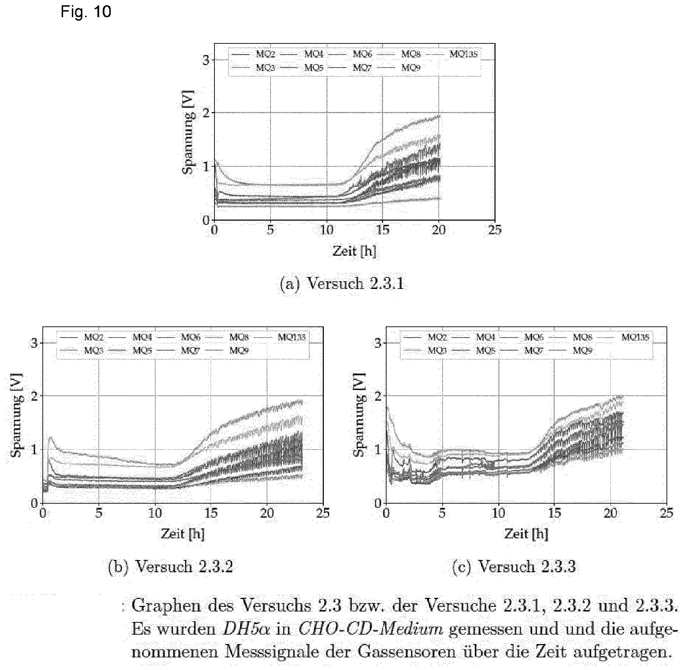

Fig. 8 zeigt Diagramme mit Messwerte zu einer Messung von VOCs aus DH5a, die in Zellkulturflaschen in der Inkubatorkammer des erfindungsgemäßen Inkubators wuchsen. Es wurden DH5a in LB-Medium gemessen und die aufgenommenen Messsignale der Gassensoren über die Zeit aufgetragen. Zur Erfassung der in Fig. 8 (analog: Fig. 9, 10) gezeigten Messdaten führt eine Steuereinrichtung des Inkubators, insbesondere mittels der Programmierung einer Datenverarbeitungseinrichtung des Inkubators, folgende Schritte aus: innerhalb eines ersten Zeitraums ab Start der Messung zum Zeitpunkt Null wird die Messkammer, welche die Messbereiche der typenunterschiedlichen MOX-Sensoren aufweist, mit einem Spülgas, hier Stickstoff, gespült. Dabei messen die MOX-Sensoren (jeweils in Form eines Spannungswertes) innerhalb dieses ersten Zeitraums im Wesentlichen zeitlich parallel und sukzessive Referenzmesswerte, während das Spülgas an den Messbereichen vorbeiströmt. Dieser erste Zeitraum liegt in Fig. 8a bei 5 Stunden, in Fig. 8b und 8c bei 7 Stunden. In einem sich unmittelbar an den ersten Zeitraum anschließenden zweiten Zeitraum wird eine als Zuluftkanal dienende Gasleitung mittels eines Ventils geöffnet, das gleichzeitig den Zustrom von Spülgas in die Messkammer stoppt. Dadurch strömt ein Volumen der Gasatmosphäre in die Messkammer - und aus dieser beispielsweise durch einen Abluftkanal wieder ab. Das Volumen strömt während des zweiten Zeitraums an den Messbereichen vorbei. Dabei messen die MOX-Sensoren innerhalb dieses zweiten Zeitraums im Wesentlichen zeitlich parallel und sukzessive Messwerte.

Der zweite Zeitraum liegt in Fig. 8a zwischen dem Ende der 5. Stunde, in Fig. 8b und 8c zwischen dem Ende der 7. Stunde ab Start der Messung bis jeweils zum Ende des Zeitraums 22,5 Stunden ab Start der Messung. Für jeden MOX-Sensor bilden die innerhalb des ersten Zeitraums ermittelten Referenzmesswerte eine "Basislinie", im Vergleich zu der der Messwert betrachtet wird: die Differenz aus Messwert und Referenzmesswert zu einem bestimmten Zeitpunkt kann als Ergebnis der Messung der Reinigungsmittelrückstände betrachtet werden (Ergebnismessdaten). Ist diese Differenz Null, liegen keine Reinigungsmittelrückstände vor. Ist sie hier im Beispiel größer Null, liegen Reinigungsmittelrückstände vor. Die meisten MOX-Sensoren detektieren Reinigungsmittelrückstände durch von Null verschiedene Ergebnismessdaten.

Bei dieser Art Messung (Fig. 8, 9, 10) und nachfolgender Auswertung ist die Datenspeichereinrichtung zur Durchführung der folgenden Schritte programmiert: i) Speichern eines Messdatensatzes im einem Datenspeicher, der hier Messwerte der Anzahl N=9 >1 von VOC-Sensoren beinhaltet, wobei ein Messwert für das detektierte Messignal des jeweiligen VOC-Sensors charakteristisch ist, das in Gegenwart eines aus der Inkubatorkammer stammenden und am Messbereich des VOC-Sensors anliegenden Volumens der Gasatmosphäre erfasst wurde; ii) Ermitteln von ersten Ergebnismessdaten aus einem Vergleich des Messdatensatzes mit einem Referenzdatensatz, insbesondere unter Verwendung einer Differenz des Messdatensatzes und des Referenzdatensatzes, der, insbesondere zeitabhängig erfasste, Referenzmesswerte der Anzahl N>1 von VOC-Sensoren beinhaltet, wobei ein Referenzmesswert für das detektierte Messignal des jeweiligen VOC-Sensors charakteristisch ist, das in Gegenwart eines aus einer Spüleinrichtung stammenden und am Messbereich des VOC-Sensors anliegenden Spülgases erfasst wurde;

Optional kann auch der Schritt vorgesehen sein: iii) Erkennen eines charakteristischen Datenmusters in dem die Ergebnismessdaten enthaltenden Ergebnismessdatensatz, wobei das charakteristische Datenmuster ein bestimmtes, im Atmosphärengas nachgewiesenes VOC bzw. VOC-Gemisch, insbesondere auch dessen Konzentration oder Menge, repräsentiert. Beispielsweise können die Ergebnismesswerte der typenunterschiedlichen MOX-Sensoren, insbesondere unter Berücksichtigung eines gemeinsamen Skalierungsfaktors, bzw. unter Berücksichtigung eines Normierungsfaktors, das charakteristische Datenmuster zu einem Zeitpunkt oder zu mehreren Zeitpunkten der Messung erfassen.

Fig. 9 zeigt Diagramme mit Messwerte zu einer Messung von VOCs aus CHO-CD-Medium, die in Zellkulturflaschen in der Inkubatorkammer des erfindungsgemäßen Inkubators wuchsen. CHO-CD-Medium wurden mittels der Sensoreinrichtung 61 gemessen und die aufgenommenen Messsignale der Gassensoren über die Zeit aufgetragen.

Fig. 10 zeigt Diagramme mit Messwerten zu einer Messung von VOCs aus DH5a in CHO-CD-Medium, die in Zellkulturflaschen in der Inkubatorkammer des erfindungsgemäßen Inkubators wuchsen. Es wurden DH5a in CHO-CD-Medium gemessen und die aufgenommenen Messsignale der Gassensoren über die Zeit aufgetragen.

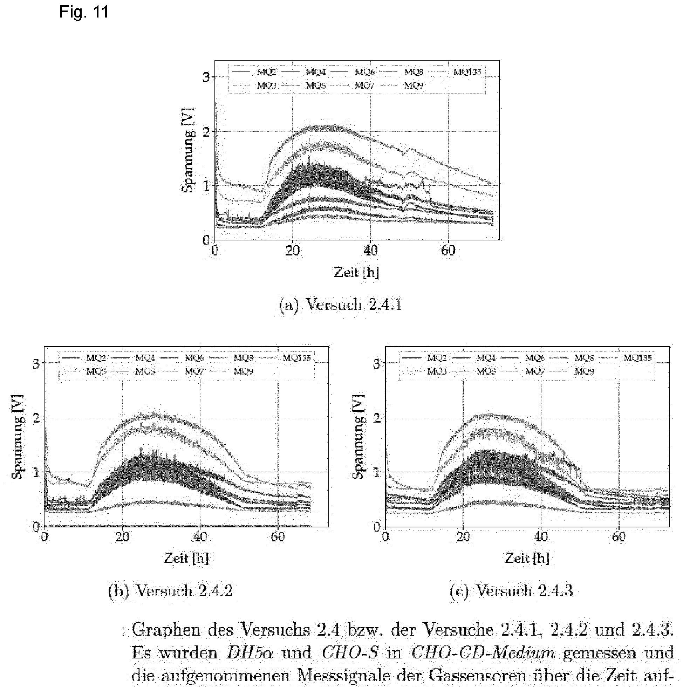

Fig. 11 zeigt Diagramme mit Messwerten zu einer Messung von VOCs aus DH5a und CHO-S in CHO-CD-Medium, die in Zellkulturflaschen in der Inkubatorkammer des erfindungsgemäßen Inkubators wuchsen. Es wurden DH5a in und CHO-S in CHO-CD-Medium gemessen und die aufgenommenen Messsignale der Gassensoren über die Zeit aufgetragen.

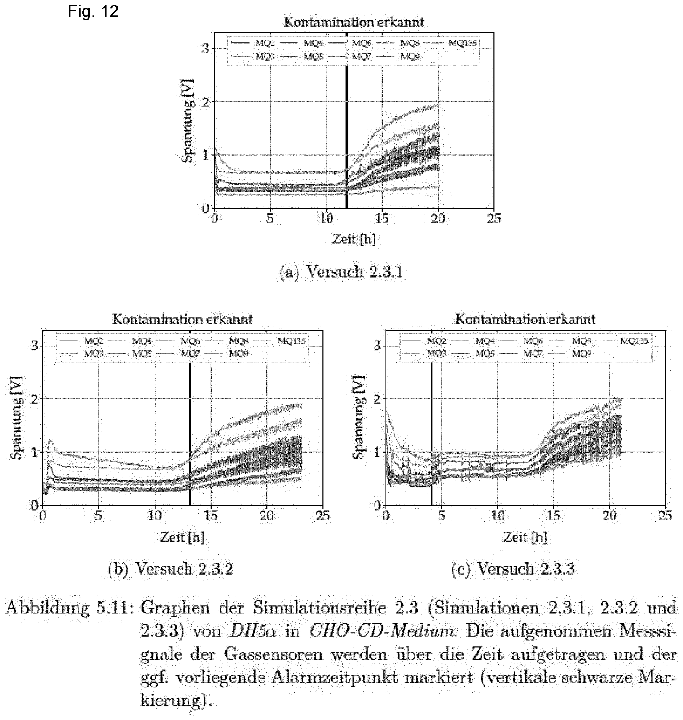

Fig. 12 zeigt Diagramme mit Messwerten zu einer Messung von VOCs aus DH5a in CHO-CD-Medium, die in Zellkulturflaschen in der Inkubatorkammer des erfindungsgemäßen Inkubators wuchsen. Die aufgenommen Messsignale der Gassensoren werden über die Zeit aufgetragen und der ggf. vorliegende Alarmzeitpunkt markiert (vertikale schwarze Markierung).

Anhand der Versuchsergebnisse ist gezeigt, dass Sensoreinrichtung 61 in der Lage ist, eine Anreicherung von Reinigungsmittelrückständen zu erkennen. Das gassensorische System kann also dazu beitragen, dass Reinigungsmittelrückstände nicht unerkannt bleiben und somit weiterführende Probleme in den Anwendungsbereichen der Zellkultivierung vermieden werden. Die Integration des gassensorischen Systems in den CO2-Inkubator war erfolgreich und die Verwendung des gassensorischen Systems im Laborumfeld des CO2-Inkubators wurde erleichtert.

### Ausführungsbeispiel eines Inkubators mit einer nur einen VOC-Sensor aufweisenden Sensoreinrichtung

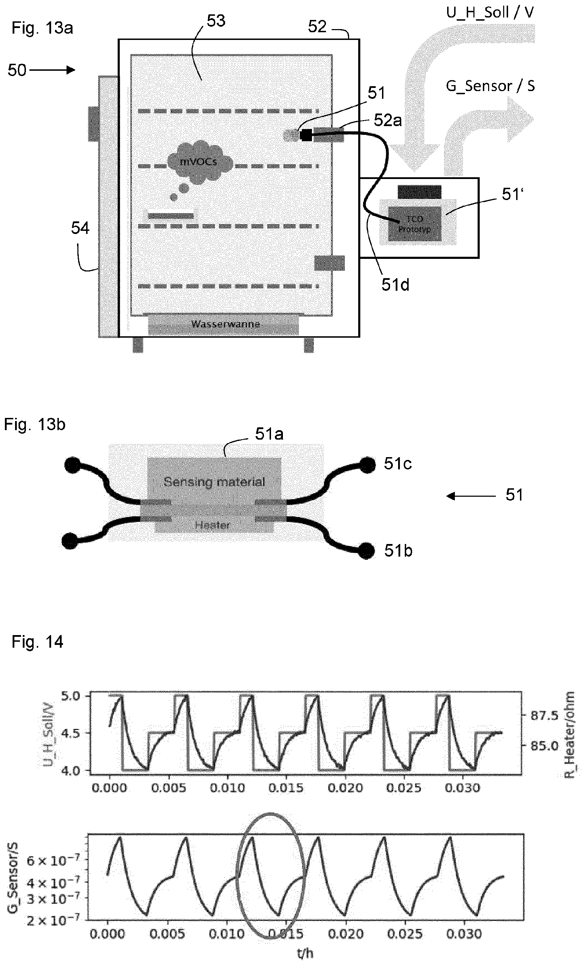

Fig. 13 zeigt einen Inkubator 50 zur Inkubation lebender Zellkulturen, aufweisend ein Gehäuse 52, darin eine Inkubatorkammer 53 zur Aufnahme von Objekten, insbesondere Zellkulturbehältern, in einem mittels der Inkubatortüre 54 verschließbaren Innenraum der Inkubatorkammer, der eine kontrollierbare Gasatmosphäre aufweist, eine Sensoreinrichtung zur Detektion einer Anreicherung, insbesondere einer Reinigung verursacht, von flüchtigen organischen Verbindungen (VOCs) aus Reinigungsmittelrückständen in der Gasatmosphäre des Innenraums, wobei die Sensoreinrichtung genau einen VOC-Sensor 51 zur Detektion der VOCs aufweist und der VOC-Sensor 51 einen Messbereich 51a aufweist. Bei dem VOC-Sensor 51 handelt es sich um einen Dickfilmsensor, MOX-Sensor, unter der Bezeichnung Figaro TGS2602 kommerziell erhältlich über Figaro USA, Inc.. Ein solcher Sensor kann auch in einem Inkubator mit einer mehr als einen VOC-Sensor aufweisenden Sensoreinrichtung verwendet werden. Dies gilt auch für die Aspekte von dessen Montage im Inkubator, der Ansteuerung mittels konstanter oder veränderlicher Spannung und der Auswertung der Messsignale.

Der Sensor 51 ist fest an der Innenwand der Kammer 53 montiert, so dass die als Messbereich dienende Metalloxidoberfläche in Strömungsverbindung mit dem Atmosphärengas des Innenraums stehend angeordnet ist. Ein elektrisches Kabel 51d führt durch einen Port 52a der Kammerwand in einen Gehäusebereich des Gehäuses 52, in dem eine elektrische Steuereinrichtung 51' angeordnet ist, zu welcher der Sensor 51 mittels des Kabels 51d verbunden ist. Mittels der Steuereinrichtung 51' wird der Sensor 51 gesteuert und ausgewertet. Das Heizelement des Sensors 51 wird von der Steuereinrichtung mit einer Messspannung U_H_Soll, gemessen in Volt, betrieben, die an Elektroden 51b des Sensors 51 anliegen (Fig. 13b). Ein elektrischer Widerstandswert, zum Beispiel ein elektrischer Widerstand R_Sensor, gemessen in Ohm, oder eine elektrische Leitfähigkeit G_Sensor, gemessen in Siemens, wird zudem über die Elektroden 51c erfasst. Das entsprechende Messsignal wird von der Steuereinrichtung 51' ausgewertet. Es ist möglich, dass der vom Sensor ausgegebene Wert des elektrischen Widerstands als Spannung in Volt ausgegeben wird, wobei aus diesem ausgegebenen Wert der gewünschte Messwert R_Sensor oder G_Sensor gemäß einer vorbekannten Abhängigkeit, insbesondere einer Proportionalität, ableitbar ist; insbesondere ist dieser ausgegebene Spannungswert zum gewünschten Messwert R_Sensor oder G_Sensor proportional.

Die Steuereinrichtung 51' dient als Auswertungseinrichtung, und enthält die Datenverarbeitungseinrichtung. Sie ist mit einem Programmcode programmierbar, und dazu programmiert, die folgenden Schritte, insbesondere gemäß diesem Programmcode, auszuführen:
- Empfangen eines Messsignals R/G_Sensor, insbesondere von Messdaten, des MOX-Sensors; insbesondere: Empfangen einer Folge von Messsignalen zeitlich, insbesondere für die Dauer einer Messzeit Δt, nacheinander, die insbesondere den zeitlichen Verlauf der Messung des eines MOX-Sensors bilden;
- Vergleich des Messsignals mit einem Referenzwert;
- Entscheiden, anhand des Ergebnisses dieses Vergleichs, ob eine Veränderung der VOC-Konzentration in der Gasatmosphäre des Innenraums vorliegt, insbesondere eine Veränderung, die für Reinigungsmittelrückstände im Innenraum charakteristisch ist.

Die Steuereinrichtung 51' ist dazu eingerichtet, das Heizelement des Sensors 51 mit einer sich periodisch ändernden Spannung U-H-Soll anzusteuern, um an der Metalloxidoberfläche eine sich entsprechend periodisch ändernde Temperatur zu erzeugen. Diese Betriebsart einer Sensoreinrichtung wird auch als "temperature cycled operation" ("TCO") bezeichnet. Dazu wird der Heizer hier mit einer, einen stufenförmigen Verlauf aufweisenden, Spannung U-H-Soll angesteuert, die je Heizperiode T mehrere verschiedene Werte vorsieht, hier die Spannungswerte 4,0 Volt, 4,5 Volt und 5,0 Volt. Dies ist in Fig. 14 gezeigt.

Jeder dieser Spannungswerte wird für einen vorgegebenen Anteil der Heizperiode T eingestellt. Die Heizperiode beträgt vorzugsweise zwischen 5 Sekunden [s] und 30 s, vorzugsweise zwischen 15 s und 25 s, vorzugsweise zwischen 17 s und 23 s, und liegt hier bei 20 s. Aus der Ansteuerung ergibt sich ein sich periodisch änderndes Messsignal R/G_Sensor mit der Messperiode T.

Im Falle eines periodischen Messignals erfolgt die Auswertung vorzugsweise, indem eine oder vorzugsweise mehrere Perioden des Messignals statistisch ausgewertet werden. Vorzugsweise ist die Datenverarbeitungseinrichtung dazu programmiert, einen durchschnittlichen Verlauf einer Messperiode zu ermitteln. Dies kann beinhalten, dass die Werte einer Anzahl M von Messperioden superpositioniert werden und dieser addierte Periodenverlauf dann mit der inversen Anzahl 1/M multipliziert wird. Auf diese Weise wird ein Messsignal geglättet und der Einfluss von Messartefakten wird reduziert.

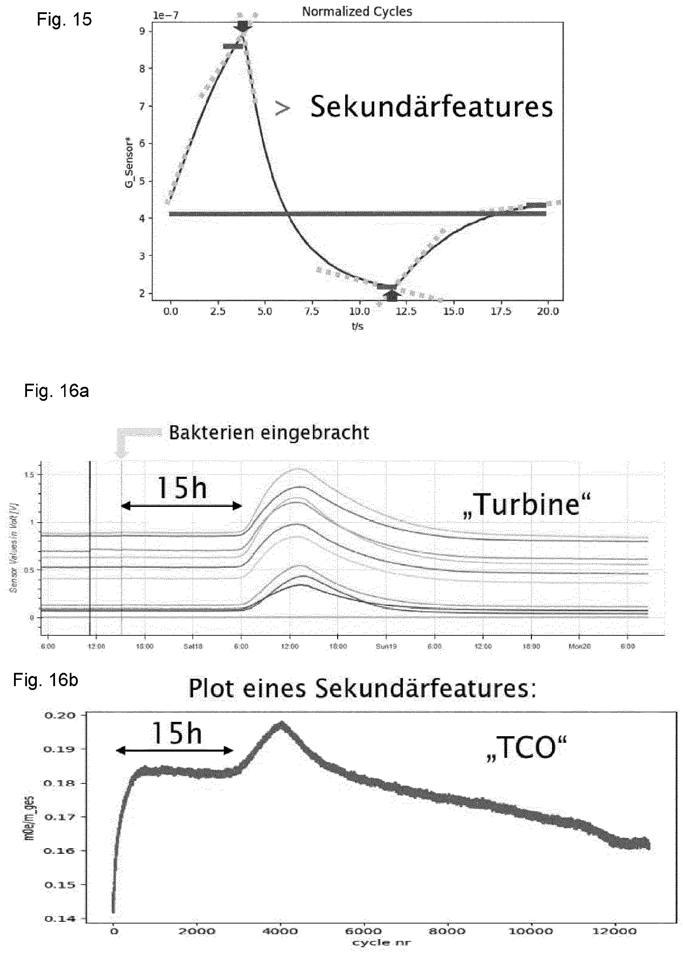

Vorzugsweise ist die Datenverarbeitungseinrichtung dazu programmiert, aus dem Signal einer einzigen Messperiode oder aus einem durchschnittlichen Verlauf einer Messperiode mindestens einen Sekundärwert abzuleiten, der sich auf eine als Sekundärfeature bezeichnete Charakteristik der Messperiode bezieht. Wie in Fig. 15 gezeigt ist, kann ein Sekundärwert eine Steigung sein, die zu einem charakteristischen Zeitpunkt der Messperiode vorliegt, beispielsweise dem Zeitpunkt des Umstellens des Spannungswertes. In Fig. 15 ist zu sehen, dass die charakteristische Steigung kurz vor oder nach diesen Zeitpunkten erfasst werden kann. Zum Zwecke der weiteren Auswertung kann der Sekundärwert, wie beschrieben, mit mindestens einem Referenzwert für diesen Sekundärwert verglichen werden.

Vorzugsweise ist die Datenverarbeitungseinrichtung dazu programmiert, einen Mittelwert mehrerer Messsignale zu ermitteln, insbesondere, einen Mittelwert mehrerer oder im Wesentlichen aller Messsignale einer Messperiode zu ermitteln. Zum Zwecke der weiteren Auswertung kann der Mittelwert, wie beschrieben, mit mindestens einem Referenzwert für diesen Mittelwert verglichen werden.

In Fig. 16a ist der zeitliche Verlauf von Messwerten gezeigt, der anhand der Sensoren einer Sensoreinrichtung 61 vom Typ "Turbine" ermittelt wurde. Das Messignal jedes Sensors reagiert auf die Zugabe der VOC-Quelle, in den Kammerinnenraum, mit zeitlicher Verzögerung, indem nach ca. 15 Stunden ein Anstieg des Messsignals erfassbar ist. In Fig. 16b wurde im vergleichbaren Experiment ein Einzelsensor (Figaro, wie in Fig. 13a, 13b gezeigt) im Modus TCO betrieben. Ein Messpunkt ist jeweils ein Mittelwert eines Zyklus bzw. einer Messperiode T=20 s, die entsprechend einer Sensoransteuerung gemäß Fig. 14 vorgegeben wurde. Dabei wurde ein Sekundärfeature der Messsignalperiode ausgewertet, vorliegend der Quotient mOe / m_ges. Die Buchstabenfolge "mOe" beschreibt ein Sekundärfeature. Jeder Messzyklus besteht aus mehreren Temperaturzyklen. Jeder Temperaturzyklus hat eine Nummer startend mit 0. Jeder Temperaturzyklus hat einen Start "s" und ein Ende "e". Es gibt zwei Klassen der Features: Mittelwert "m" und Steigung "s". Demnach bedeutet der Quotient: m0e = Mittelwert am Ende des Nullten Zyklus' / m_ges = Mittelwert über einen gesamten Messzyklus

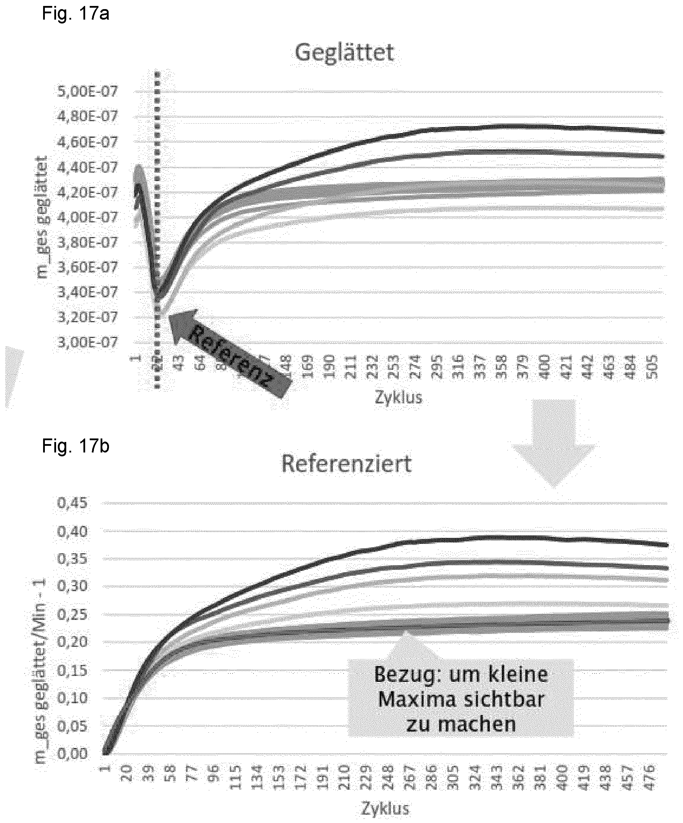

Fig. 17a zeigt, zum besseren Vergleich überlagert, eine Reihe von unterschiedlichen Kurven ausgewerteter Messsignale (elektrische Leitfähigkeit der Messchicht, in Siemens), die in unterschiedlichen Experimenten erfasst wurden. Der Messaufbau entspricht der Vorrichtung der Figuren 13a, b, die im TCO Modus betrieben wurde, Messperiode 20 s. Die durch ihre Farbe bzw. Graduierung unterscheidbaren Kurven entsprechen jeweils einem Experiment. Variiert wurde der Volumenprozentgehalt an Ethanol (z.B. zwischen 0,1% und 0,4%) in einem Ethanol-Wassergemisch mit vorgegebenem Gesamtvolumen, das immer in demselben offenen Behälter im Kammerinneren platziert wurde. Der Referenzzeitpunkt ist der Moment, in dem die Kammertüre nach dem kurzen Einsetzen der Probenbehälter wieder geschlossen wurde. In Fig. 17a ist, für jeden Zyklus (Messperiode) der -hier geglättete- Mittelwert "m_ges geglättet" der Messsignale der Messperiode aufgetragen. In Fig. 17b ist der Wert "m_ges geglättet" zusätzlich durch den Minimumwert der jeweiligen Kurve zum Referenzzeitpunkt dividiert, und von diesem Quotienten der Wert 1 subtrahiert (m_ges geglättet / Min - 1), zur Normierung der Messsignale.. Alle Messsignale werden auf diesen Wert normiert (mges/min) - in dem Falle ist das Minimum dann gleich 1. Subtrahiert man dann 1 von den Messwerten, wird die Kurve auf Null gezogen.

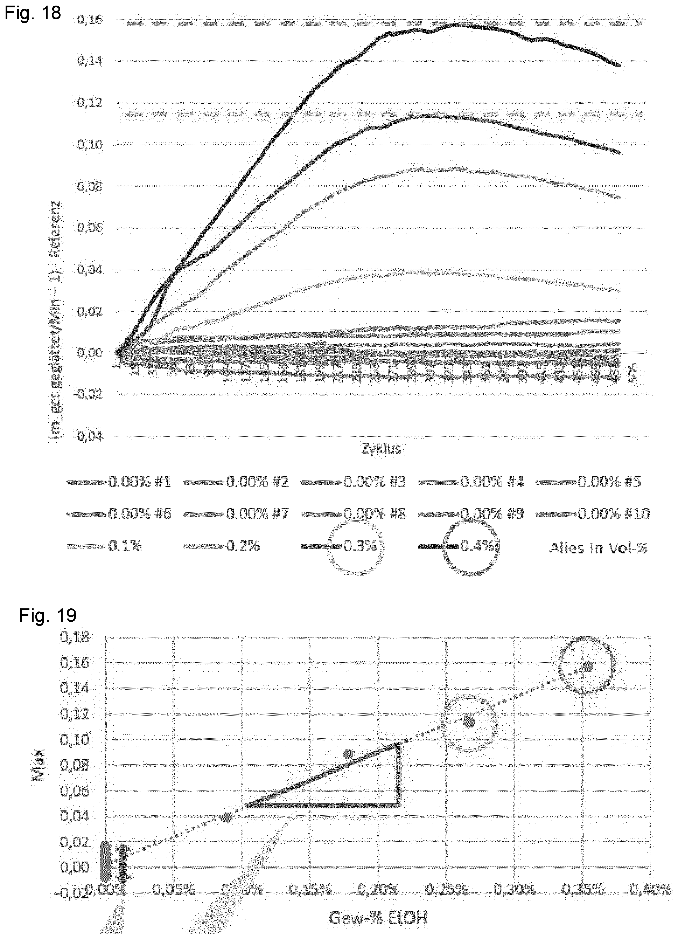

In Fig. 18 sind die Maxima der Messkurven aus Fig. 17b gegenüber der jeweiligen Alkoholkonzentration (Ethanol in Wasser, Gew%) aufgetragen. Einerseits ist erkennbar, dass die Messanordnung effizient geeignet ist, um mittels des chemischen MOX-Sensors die EtOH-VOC im Kammerinnenraum zu detektieren und zu quantifizieren, denn die Maxima der ausgewerteten Größe (m_ges geglättet / Min - 1) liegen auf einer Geraden. Aus jedem weiteren vergleichbaren Experiment (m_ges geglättet / Min - 1) unbekannter Ethanolkonzentration lässt sich aus der Lage des Maximums der Kurve und der Geradensteigung die Ethanolkonzentration der Probe ermitteln. Das Experiment ist für die Erfassung von Bakterien in einem Kammerinneren hochrelevant, da ein Hauptbestandteil der bei den bakteriellen Stoffwechselvorgängen freigesetzten VOC's Alkohole sind. Die Geradensteigung selber ist zudem eine Maß für die Sensitivität der Messanordnung - größere Steigungen implizieren eine leichtere Unterscheidbarkeit der ausgewerteten Größe. Eine optionale Abschätzung einer Nachweisgrenze, der Erfassungsgrenze und der Bestimmungsgrenze für die Messanordnung/das Messverfahren lässt sich mittels DIN 32645 ermitteln. Dabei wird hinsichtlich der Fig. 18 die Steigung berücksichtigt, die Drift des Messsignale über die Zeit, und das Rauschen der Messsignale.

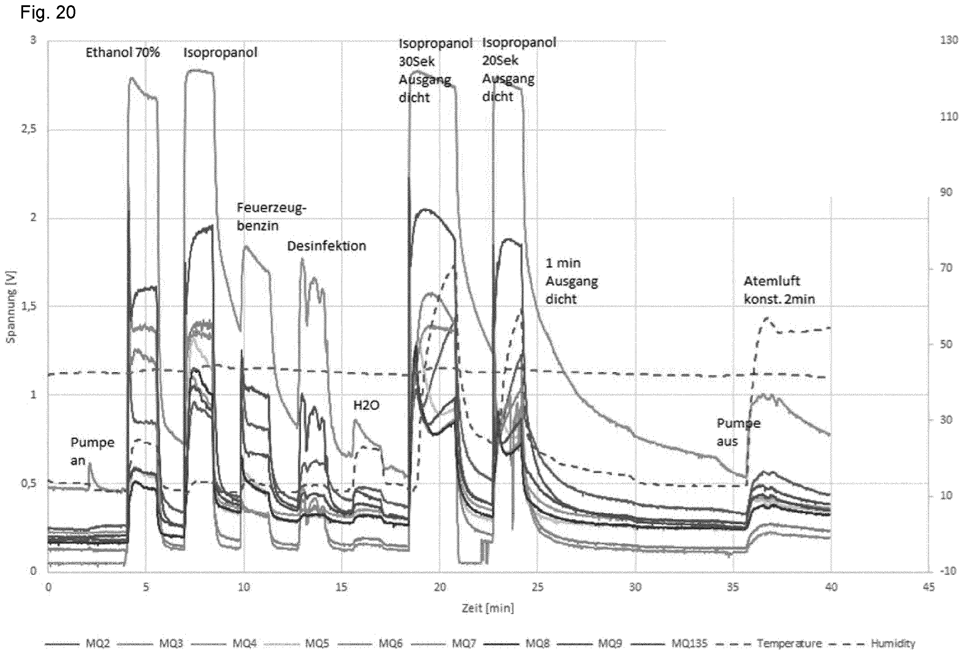

Fig. 20 zeigt ein Diagramm mit Messwerten zu einer Messung von VOCs aus verschiedenen Reinigungsmitteln, deren Rückstände auf einer Oberfläche in der Inkubatorkammer lokalisiert waren. Die Proben der Reinigungsmittel, deren organische Verbindungen generell bevorzugt mindestens zwei Kohlenstoffatome aufweisen, wurden im Abstand von einigen Minuten hintereinander durch Verreiben auf einer Oberfläche im Inkubator aufgebracht. Die Messkammer wurde mit Spülgas gespült, bevor eine Probe durch eine neue Probe ersetzt wurde. Nacheinander wurden folgende Proben eingebracht und gemessen: Ethanol in Wasser (70 vol%); Isopropanol, Feuerzeugbenzin (=Leichtbenzin, eine Mischung verschiedener Kohlenwasserstoffe mit fünf bis sieben Kohlenstoffatomen (Isopentan bis ≈Toluol, Siedebereich ca. 25 bis 100 °C)); Desinfektionsmittel (beinhaltend Propan-2-ol, Propan-1-ol, 1-Tetradecanol und Mecetroniumetilsulfat); H2O (im offenen Behälter; zur Kontrolle); Isopropanol (für 30 Sek. bzw. 20 Sekunden in einer offenen Flasche in die Inkubatorkammer gegeben); Atemluft (für 2 min in die Messkammer eingebracht; zur Kontrolle). Die Messungen wurden mit den bereits erläuterten 9 MOX Sensoren ausgeführt, wie sie bereits im Aufbau der Messungen zu den Figuren 8 bis 12 verwendet wurden. Mit jedem der Sensoren konnte erfolgreich ein Messignal der Proben erfasst werden, wenn auch mit unterschiedlicher Empfindlichkeit.

Das erfindungsgemäße Verfahren, das bei den Messungen der Fig. 20 zur Anwendung kam, erwies sich somit als robust und zuverlässig. Zur Anwendung kam ein Verfahren zur Detektion einer Anreicherung von in Reinigungsmittelrückständen enthaltenen flüchtigen organischen Verbindungen (VOCs) in einem Inkubator 1; 50; 200; 300; 400; 500; 600; 700; 800; 900; 1000, insbesondere einem erfindungsgemäßen Inkubator, welcher der Inkubation lebender Zellkulturen dient und der aufweist:
- eine Inkubatorkammer 2 zur Aufnahme von Objekten, insbesondere Zellkulturbehältern, in einem verschließbaren Innenraum der Inkubatorkammer, der eine kontrollierbare Gasatmosphäre aufweist,
- eine Sensoreinrichtung 11; 21; 31; 41 zur Detektion einer Anreicherung von in den Reinigungsmittelrückständen enthaltenen flüchtigen organischen Verbindungen (VOCs) im Atmosphärengas der Gasatmosphäre, wobei die Sensoreinrichtung mindestens einen VOC-Sensor 11 zur Detektion der VOCs aufweist und der mindestens eine VOC-Sensor mindestens einen Messbereich aufweist, der in Strömungsverbindung mit dem Atmosphärengas des Innenraums stehend angeordnet ist,
   wobei das Verfahren die mittels einer elektronischen Steuereinrichtung des Inkubators auszuführenden Schritte aufweist:
   A) Aktivieren der Sensoreinrichtung,
   B) Erfassen mindestens eines Messwertes, der die Konzentration der in den Reinigungsmittelrückständen enthaltenen VOCs charakterisiert.

Das Aktivieren der Sensoreinrichtung beinhaltet insbesondere im Fall der MOX-Sensoren, dass deren Messbereiche durch die sensoreigenen Heizeinrichtungen elektrisch beheizt werden, und eine Basislinie des Messsignals erfasst wird, in Bezug auf die die von Proben verursachten Messsignale erfasst werden.

Der bei den Messungen zur Figur 20 verwendete Inkubator war erfindungsgemäß ein Inkubator 1; 50; 200; 300; 400; 500; 600; 700; 800; 900; 1000, welcher der Inkubation lebender Zellkulturen dient und der aufweist:
- eine Inkubatorkammer 2 zur Aufnahme von Objekten, insbesondere Zellkulturbehältern, in einem verschließbaren Innenraum der Inkubatorkammer, der eine kontrollierbare Gasatmosphäre aufweist,
- wobei insbesondere eine Oberfläche im Innenraum der Inkubatorkammer 2 und/oder die Gasatmosphäre im Innenraum der Inkubatorkammer 2 aus einer Reinigung der Oberfläche resultierende Reinigungsmittelrückstände aufweist oder vorzugsweise aufweisen kann,
- eine Sensoreinrichtung 11; 21; 31; 41 zur Detektion einer Anreicherung von in den Reinigungsmittelrückständen enthaltenen flüchtigen organischen Verbindungen (VOCs), wobei die Sensoreinrichtung mindestens einen VOC-Sensor 11 zur Detektion der VOCs aufweist und der mindestens eine VOC-Sensor mindestens einen Messbereich aufweist, der in Strömungsverbindung mit dem Atmosphärengas des Innenraums stehend angeordnet ist.

Die insbesondere bei den Messungen zur Figur 20 verwendete Messkammer weist folgende Bauteile bzw. Gassensoren auf. Diese weisen gemäß Hersteller eine Empfindlichkeit zur Detektion der nachfolgend genannten Verbindungen auf:

### Messkammer (MK)

**Tabelle : Hardwareteile der Messkammer (MK)**

| Bauteil Bezeichnung | Details | |
|---|---|---|
| GassensorMQ 2 | Alkane (Butan, Propan, Methan) | |
| | Alkohole | |
| | Wasserstoff | |
| GassensorMQ 3 | Alkohole | |
| GassensorMQ 4 | Alkane (Methan) | |
| GassensorMQ 5 | Alkane (Methan, Propan, Butan) | |
| GassensorMQ 6 | Alkane (Methan, Propan, Butan) | |
| GassensorMQ 7 | Oxide (Kohlenstoffmonoxid) | |
| GassensorMQ 8 | Wasserstoff | |
| Gassensor MQ 9 | Kohlenstoffmonoxid | |
| | Alkane (Methan, Propan, Butan) | |
| GassensorMQ | 135 Alkohole | |
| | Benzole (Benzol) | |
| | Amine (Ammoniak) | |
| | Oxide (Kohlenstoff-, Stickstoffdioxid) | |
| Umgebungssensor | Adafruit BME680 | Temperatur, Feuchtigkeit, Druck |
| Spritzgusskammer Hammond | | Aluminium, 170 × 120 × 55 mm |
| Silikon | Abdichten Gassensoren | |

## Patentansprüche

1. Inkubator (1; 50; 200; 300; 400; 500; 600; 700; 800; 900; 1000), welcher der Inkubation lebender Zellkulturen dient und der aufweist:
• eine Inkubatorkammer (2) zur Aufnahme von Objekten, insbesondere Zellkulturbehältern, in einem verschließbaren Innenraum der Inkubatorkammer, der eine kontrollierbare Gasatmosphäre aufweist,
• wobei eine Oberfläche im Innenraum der Inkubatorkammer (2) und/oder die Gasatmosphäre im Innenraum der Inkubatorkammer (2), insbesondere aus einer Reinigung der Oberfläche resultierende, Reinigungsmittelrückstände aufweist,
• eine Sensoreinrichtung (11; 21; 31; 41) zur Detektion einer Anreicherung von in den Reinigungsmittelrückständen enthaltenen flüchtigen organischen Verbindungen (VOCs), wobei die Sensoreinrichtung mindestens einen VOC-Sensor (11) zur Detektion der VOCs aufweist und der mindestens eine VOC-Sensor mindestens einen Messbereich aufweist, der in Strömungsverbindung mit dem Atmosphärengas des Innenraums stehend angeordnet ist.

2. Inkubator gemäß Anspruch 1, wobei der mindestens eine oder die VOC-Sensoren MOX-Sensoren sind, die zur Detektion der Reinigungsmittelrückstände geeignet sind.

3. Inkubator gemäß Anspruch 1 oder 2, der eine elektronische Steuereinrichtung, eine Inkubatortüre (4) zum Verschließen der Inkubatorkammer (2) und einen elektronischen Türsensor aufweist, mit dem das Öffnen und/oder Schließen der Inkubatortüre erfassbar ist, wobei die elektronische Steuereinrichtung dazu programmiert ist,
a) das Öffnen und/oder Schließen der Inkubatortüre mittels des Türsensors als Türstatusparameter zu erfassen,
b) in Abhängigkeit von dem Türstatusparameter, insbesondere nach dem Schließen der Inkubatortüre, die Sensoreinrichtung automatisch zu aktivieren,
c) mittels der Sensoreinrichtung mindestens einen Messwert zu erfassen, der die Konzentration der in den Reinigungsmittelrückständen enthaltenen VOCs charakterisiert.

4. Inkubator gemäß Anspruch 3, wobei die Aktivierung in Schritt b) erfolgt, wenn eine zeitabhängige Auswertung des Türparameters ergibt, dass die Inkubatortüre für mindestens die Dauer einer vorbestimmten, insbesondere in einer Datenspeichereinrichtung des Inkubators gespeicherten, Zeitspanne geöffnet war, und dass die Aktivierung in Schritt b) insbesondere nicht erfolgt, wenn die zeitabhängige Auswertung des Türparameters ergibt, dass die Inkubatortüre für weniger als die Dauer der vorbestimmten Zeitspanne geöffnet war.

5. Inkubator gemäß Anspruch 1 oder 2, der eine elektronische Steuereinrichtung und eine Benutzerschnittstelleneinrichtung aufweist, über die ein Benutzer Daten an die Datenverarbeitungseinrichtung bzw. die Steuereinrichtung eingeben kann, und über deren Bildschirm Informationen an den Benutzer ausgegeben werden können, wobei die Steuereinrichtung dazu programmiert ist,
i) zur Ausgabe einer Anleitung zum Reinigen der Inkubatorkammer mindestens eine Reinigungsanweisung als visuelle Information für den Benutzer am Bildschirm auszugeben,
ii) eine Eingabe des Benutzers an der Benutzerschnittstelleneinrichtung zu erfassen, mit der der Benutzer die Durchführung der Reinigung gemäß der Reinigungsanweisung bestätigt,
iii) in Abhängigkeit von der Eingabe des Benutzers die Sensoreinrichtung automatisch zu aktivieren,
iv) mittels der Sensoreinrichtung mindestens einen Messwert zu erfassen, der die Konzentration der in den Reinigungsmittelrückständen enthaltenen VOCs charakterisiert.

6. Inkubator gemäß Anspruch 1 oder 2, der eine elektronische Steuereinrichtung und eine Benutzerschnittstelleneinrichtung aufweist, mittels der eine Benutzereingabe in Form von Daten erfassbar ist, und Informationen an den Benutzer ausgebbar sind, wobei die Steuereinrichtung dazu programmiert ist,
i) mittels der Benutzerschnittstelleneinrichtung eine Eingabe des Benutzers zu erfassen, mit der dieser das Aktivieren der Sensoreinrichtung anfordert,
ii) in Abhängigkeit von der Eingabe des Benutzers die Sensoreinrichtung automatisch zu aktivieren,
iii) mittels der Sensoreinrichtung mindestens einen Messwert zu erfassen, der die Konzentration der in den Reinigungsmittelrückständen enthaltenen VOCs charakterisiert.

7. Inkubator gemäß Anspruch 1 oder 2, der einen Temperatursensor aufweist, der insbesondere zum Erfassen einer Temperatur einer Wasserwanne des Inkubators eingerichtet ist oder der zur Messung einer Temperatur der Inkubatorkammer eingerichtet ist, und der eine elektronische Steuereinrichtung aufweist, die dazu programmiert ist,
i) in Abhängigkeit von der mittels des Temperatursensors erfassten Temperatur, insbesondere bei Überschreiten oder Unterschreiten eines vordefinierten Schwellwertes, die Sensoreinrichtung automatisch zu aktivieren,
ii) mittels der Sensoreinrichtung mindestens einen Messwert zu erfassen, der die Konzentration der in den Reinigungsmittelrückständen enthaltenen VOCs charakterisiert.

8. Inkubator gemäß Anspruch 1 oder 2, der eine Heizeinrichtung zur Durchführung einer automatischen Sterilisation der Inkubatorkammer während mindestens einer Hochtemperaturphase aufweist, insbesondere bei Temperaturen zwischen 100 °C und 200°C (inklusive dieser Werte), und der eine elektronische Steuereinrichtung aufweist, die dazu programmiert ist,
i) die Sensoreinrichtung vor, während oder nach der Durchführung der automatischen Sterilisation der Inkubatorkammer automatisch zu aktivieren,
ii) mittels der Sensoreinrichtung mindestens einen Messwert zu erfassen, der die Konzentration der in den Reinigungsmittelrückständen enthaltenen VOCs charakterisiert.

9. Inkubator gemäß einem der vorangehenden Ansprüche, der eine Benutzerschnittstelleneinrichtung aufweist, mittels der eine Benutzereingabe in Forma von Daten erfassbar ist, und Informationen an den Benutzer ausgebbar sind, eine elektronische Steuereinrichtung aufweist, die dazu programmiert ist,
i) die Sensoreinrichtung automatisch zu aktivieren,
ii) mittels der Sensoreinrichtung mindestens einen Messwert zu erfassen, der die Konzentration der in den Reinigungsmittelrückständen enthaltenen VOCs charakterisiert,
iii) den mindestens einen Messwert mit einem vorbestimmten Schwellenwert zu vergleichen, der typisch ist für eine gemäß einer reinigungsmittelbasierten, in vorbestimmter Weise ausgeführten Reinigung der Inkubatorkammer,
iv)in Abhängigkeit vom Ergebnis dieses Vergleichs dem Benutzer mittels der Benutzerschnittstelleneinrichtung eine Information auszugeben, dass eine in vorbestimmter Weise ausgeführte Reinigung der Inkubatorkammer erfasst wurde oder nicht erfasst wurde.

10. Verfahren zur Detektion einer Anreicherung von in Reinigungsmittelrückständen enthaltenen flüchtigen organischen Verbindungen (VOCs) in einem Inkubator (1; 50; 200; 300; 400; 500; 600; 700; 800; 900; 1000), insbesondere einem Inkubator gemäß einem der vorangehenden Ansprüche, welcher der Inkubation lebender Zellkulturen dient und der aufweist:
• eine Inkubatorkammer (2) zur Aufnahme von Objekten, insbesondere Zellkulturbehältern, in einem verschließbaren Innenraum der Inkubatorkammer, der eine kontrollierbare Gasatmosphäre aufweist,
• eine Sensoreinrichtung (11; 21; 31; 41) zur Detektion einer Anreicherung von in den Reinigungsmittelrückständen enthaltenen flüchtigen organischen Verbindungen (VOCs), wobei die Sensoreinrichtung mindestens einen VOC-Sensor (11) zur Detektion der VOCs aufweist und der mindestens eine VOC-Sensor mindestens einen Messbereich aufweist, der in Strömungsverbindung mit dem Atmosphärengas des Innenraums stehend angeordnet ist,
wobei das Verfahren die mittels einer elektronischen Steuereinrichtung des Inkubators auszuführenden Schritte aufweist:
A) Aktivieren der Sensoreinrichtung,
B) Erfassen mindestens eines Messwertes, der die Konzentration der in den Reinigungsmittelrückständen enthaltenen VOCs charakterisiert.

11. Verfahren gemäß Anspruch 10, das die Schritte aufweist:
A1) Erfassen eines Öffnens und/oder Schließens der Inkubatortüre mittels eines Türsensors des Inkubators in Form eines Türstatusparameters,
A2) Durchführen des Schrittes A) in Abhängigkeit von dem Türstatusparameter, insbesondere Durchführen des Schrittes A) nach dem Schließen der Inkubatortüre.

12. Verfahren gemäß Anspruch 11, wobei die Aktivierung in Schritt A2) erfolgt, wenn eine zeitabhängige Auswertung des Türparameters ergibt, dass die Inkubatortüre für mindestens die Dauer einer vorbestimmten, insbesondere in einer Datenspeichereinrichtung des Inkubators gespeicherten, Zeitspanne geöffnet war, und dass die Aktivierung in Schritt A2) insbesondere nicht erfolgt, wenn die zeitabhängige Auswertung des Türparameters ergibt, dass die Inkubatortüre für weniger als die Dauer der vorbestimmten Zeitspanne geöffnet war.

13. Verfahren gemäß Anspruch 10, aufweisend die Schritte:
A1) Ausgabe einer Anleitung zum Reinigen der Inkubatorkammer, beinhaltend mindestens eine Reinigungsanweisung als, insbesondere visuelle Information an einem Bildschirm oder per Sprachausgabe, für den Benutzer an einer Benutzerschnittstelleneinrichtung des Inkubators,
A2) Erfassen einer Eingabe des Benutzers an d Benutzerschnittstelleneinrichtung des Inkubators, wobei der Benutzer mittels der Eingabe die Durchführung der Reinigung gemäß der Reinigungsanweisung bestätigt,
A3) automatisches Aktivieren der Sensoreinrichtung gemäß Schritt A) in Abhängigkeit von der Eingabe des Benutzers.

14. Verfahren gemäß Anspruch 10, aufweisend die Schritte:
A1) Erfassen einer Eingabe des Benutzers mittels der Benutzerschnittstelleneinrichtung, wobei der Benutzer mit dieser Eingabe das Aktivieren der Sensoreinrichtung anfordert,
A2) in Abhängigkeit von der Eingabe des Benutzers den Schritt A) durchzuführen.

15. Verwendung einer Sensoreinrichtung (11; 21; 31; 41) zur Detektion einer Anreicherung von in Reinigungsmittelrückständen enthaltenen flüchtigen organischen Verbindungen (VOCs), die in der Inkubatorkammer (2) eines Inkubators (1; 50; 200; 300; 400; 500; 600; 700; 800; 900; 1000) vorhanden sind, welcher der Inkubation lebender Zellkulturen dient, wobei die Sensoreinrichtung mindestens einen VOC-Sensor (11) zur Detektion der VOCs aufweist.
